# EUROPEAN PATENT APPLICATION

(11) **EP 3 456 355 A1**
(43) Date of publication of application: **20.03.2019**
(21) Application number: 18202398.6
(22) Date of filing: 19.08.2016
(51) Int. Cl.: A61K 45/06, A61K 31/5386, A61K 31/433, A61K 31/4439, A61K 31/444, A61K 31/497, A61K 31/501, A61K 31/506, A61K 31/5377, A61P 19/02, A61P 35/00, A61P 35/02, A61P 37/06

(54) **COMPOSITIONS COMPRISING A PI3K INHIBITOR AND AN HDAC INHIBITOR**

(30) Priority: 09.08.2015 GB 201514756
(62) Divisional of application: 16756762.7
(71) Applicant: Karus Therapeutics Ltd, Harwell Campus Oxfordshire OX11 0SG (GB)
(72) Inventor: SHUTTLEWORTH, Stephen Joseph, Harwell Campus, Oxfordshire OX11 0SG (GB); WHALE, Andrew David, Harwell Campus, Oxfordshire OX11 0SG (GB)
(74) Representative: Gill Jennings & Every LLP

(57) **Abstract**

The invention relates to a pharmaceutical composition comprising at least one PI3K inhibitor of Formula I or a pharmaceutically acceptable salt thereof and at least one HDAC inhibitor such as a compound of Formula II or a pharmaceutically acceptable salt thereof; orat least one PI3K inhibitor such as a compound of Formula I or a pharmaceutically acceptable salt thereof and at least one HDAC inhibitor of Formula II or a pharmaceutically acceptable salt thereof.

## Description

### Field of the Invention

The present invention relates to novel combinations comprising a compound which acts as an inhibitor of the class IA phosphoinositide 3-kinase enzymes, PI3K-p110δ and PI3K-p110β, and a compound which acts as an inhibitor of histone deacetylase (HDAC). Such combinations are useful in therapy, for example in the therapy of cancer, immune and inflammatory diseases.

### Background of the Invention

The phosphoinositide 3-kinases (PI3Ks) constitute a family of lipid kinases involved in the regulation of a network of signal transduction pathways that control a range of cellular processes. PI3Ks are classified into three distinct subfamilies, named class I, II, and III based upon their substrate specificities. Class IA PI3Ks possess a p110α, p110β, or p110δ catalytic subunit complexed with one of three regulatory subunits, p85α, p85β or p55δ. Class IA PI3Ks are activated by receptor tyrosine kinases, antigen receptors, G-protein coupled receptors (GPCRs), and cytokine receptors. The class IA PI3Ks primarily generate phosphatidylinositol-3,4,5-triphosphate (PI(3,4,5)P₃), a second messenger that activates the downstream target AKT. The consequences of biological activation of AKT include tumour cell progression, proliferation, survival and growth, and there is significant evidence suggesting that the PI3K/AKT pathway is dysregulated in many human cancers. Additionally, PI3K activity has been implicated in endocrinology, cardiovascular disease, immune disorders and inflammation. It has been established that PI3K-p110δ plays a critical role in the recruitment and activation of immune and inflammatory cells. PI3K-p110δ is also upregulated in a number of human tumours and plays a key role in tumour cell proliferation and survival.

Compounds that are able to modulate p110β and p110δ activity have important therapeutic potential in cancer and immune and inflammatory disorders.

HDACs are zinc metalloenzymes that catalyse the hydrolysis of acetylated lysine residues. In histones, this returns lysines to their protonated state and is a global mechanism of eukaryotic transcriptional control, resulting in tight packaging of DNA in the nucleosome. Additionally, reversible lysine acetylation is an important regulatory process for non-histone proteins. Thus, compounds which are able to modulate HDAC have important therapeutic potential.

Combinations of HDAC inhibitors and PI3K inhibitors have been disclosed, for example in WO2015054355.

### Summary of the Invention

The present invention relates in part to combinations of certain PI3K compounds, such as those disclosed herein and certain HDAC compounds, such as those disclosed herein. These combinations may be synergistic and therefore offer may offer improvements with respect to the individual components. For example, they may allow a lower dose to be administered. The present invention is based at least in part on data presented herein.

Certain PI3K inhibitors disclosed herein are also disclosed in PCT/GB2015/050396 (which is unpublished as of 19 August 2015, and the contents of which are incorporated herein by reference). They may have increased activity and/or bioavailability over the compounds described in WO2011/021038, which is also incorporated herein by reference.

Certain HDAC inhibitors disclosed herein are also disclosed in WO2014/181137, which is incorporated herein by reference.

Therefore, the present invention is directed in part to
a) a pharmaceutical composition comprising a PI3K inhibitor compound of Formula I: 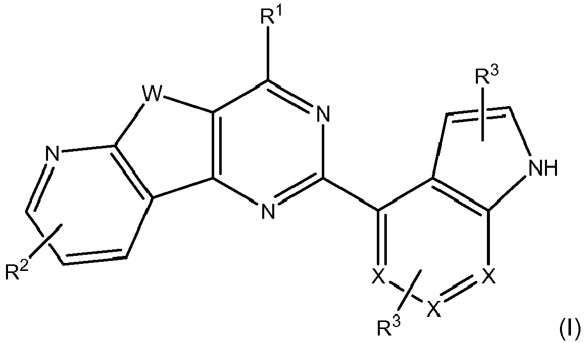 or a pharmaceutically acceptable salt thereof, wherein:
   W is O, N-H, N-(C₁-C₁₀ alkyl) or S;
   each X is selected independently for each occurrence from CH, CR³, or N;
   R¹ is a 5 to 7-membered saturated or unsaturated, optionally substituted heterocycle containing at least 1 heteroatom selected from N or O;
   R² is L-Y;
   each L is selected from the group consisting of a direct bond, C₁-C₁₀ alkylene, C₂-C₁₀ alkenylene and C₂-C₁₀ alkynylene;
   Y is an optionally substituted fused, bridged or spirocyclic non-aromatic heterocycle containing up to 4 heteroatoms (for example, one, two, three or four heteroatoms) each independently selected from N or O, and comprising 5 to 12 carbon or heteroatoms in total; and
   each R³ is independently H, C₁-C₁₀ alkyl, halogen, fluoro C₁-C₁₀ alkyl, O-C₁-C₁₀ alkyl, -NH-C₁-C₁₀ alkyl, S-C₁-C₁₀ alkyl, O-fluoro C₁-C₁₀ alkyl, NH-acyl, NH-C(O)-NH-C₁-C₁₀ alkyl, C(O)-NH-C₁-C₁₀ alkyl, aryl or heteroaryl;
   **in combination with**
   a HDAC inhibitor such as compound of formula II 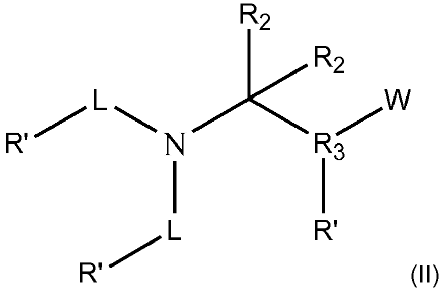
   or a pharmaceutically acceptable salt thereof, wherein:
   each R^{/} is independently selected from H and QR₁;
   each Q is independently selected from a bond, CO, CO₂, NH, S, SO, SO₂ or O;
   each R₁ is independently selected from H, C₁-C₁₀ alkyl, C₂-C₁₀ alkenyl, C₂-C₁₀ alkynyl, aryl, heteroaryl, C₁-C₁₀ cycloalkyl, halogen, C₁-C₁₀ alkylaryl, C₁-C₁₀ alkyl heteroaryl or C₁-C₁₀ heterocycloalkyl;
   each L is independently selected from a 5 to 10-membered nitrogen-containing heteroaryl;
   W is a zinc-binding group;
   each R² is independently hydrogen or C₁ to C₆ alkyl; and
   R³ is an aryl or heteroaryl;
   each aryl or heteroaryl may be substituted by up to three substituents selected from C₁-C₆ alkyl, hydroxy, C₁-C₃ hydroxyalkyl, C₁-C₃ alkoxy, C₁-C₃ haloalkoxy, amino, C₁-C₃ mono alkylamino, C₁-C₃ bis alkylamino, C₁-C₃ acylamino, C₁-C₃ aminoalkyl, mono (C₁-C₃ alkyl) amino C₁-C₃ alkyl, bis(C₁-C₃ alkyl) amino C₁-C₃ alkyl, C₁-C₃-acylamino, C₁-C₃ alkyl sulfonylamino, halo, nitro, cyano, trifluoromethyl, carboxy, C₁-C₃ alkoxycarbonyl, aminocarbonyl, mono C₁-C₃ alkyl aminocarbonyl, bis C₁-C₃ alkyl aminocarbonyl, -SO₃H, C₁-C₃ alkylsulfonyl, aminosulfonyl, mono C₁-C₃ alkyl aminosulfonyl and bis C₁-C₃-alkyl aminosulfonyl; and
   each alkyl, alkenyl or alkynyl may be substituted with halogen, NH₂, NO₂ or hydroxyl; **or**
b) a PI3K inhibitor such as a compound of Formula I or pharmaceutically salt thereof **in combination with** a HDAC inhibitor of Formula II or a pharmaceutically acceptable salt thereof.

Kits and methods comprising the compositions described above are also provided.

### Description of the Preferred Embodiments

### Definitions

As used herein, "alkyl" means a C₁-C₁₀ alkyl group, which can be linear or branched. Preferably, it is a C₁-C₆ alkyl moiety. More preferably, it is a C₁-C₄ alkyl moiety. Examples include methyl, ethyl, n-propyl and t-butyl. It may be divalent, e.g. propylene.

As used herein, "alkenyl" means a C₂-C₁₀ alkenyl group. Preferably, it is a C₂-C₆ alkenyl group. More preferably, it is a C₂-C₄ alkenyl group. The alkenyl radicals may be mono- or di-saturated, more preferably monosaturated. Examples include vinyl, allyl, 1-propenyl, isopropenyl and 1-butenyl. It may be divalent, e.g. propenylene.

As used herein, "alkynyl" is a C₂-C₁₀ alkynyl group which can be linear or branched. Preferably, it is a C₂-C₄ alkynyl group or moiety. It may be divalent.

Each of the C₁-C₁₀ alkyl, C₂-C₁₀ alkenyl and C₂-C₁₀ alkynyl groups may be optionally substituted with each other, i.e. C₁-C₁₀ alkyl optionally substituted with C₂-C₁₀ alkenyl. They may also be optionally substituted with aryl, cycloalkyl (preferably C₃-C₁₀), aryl or heteroaryl. They may also be substituted with halogen (e.g. F, Cl), NH₂, NO₂ or hydroxyl. Preferably, they may be substituted with up to 10 halogen atoms or more preferably up to 5 halogens. For example, they may be substituted by 1, 2, 3, 4 or 5 halogen atoms. Preferably, the halogen is fluorine. For example, they may be substituted with CF₃, CHF₂, CH₂CF₃, CH₂CHF₂, CF₂CF₃ or OCF₃, OCHF₂, OCH₂CF₃, OCH₂CHF₂ or OCF₂CF₃.

As used herein, the term "fluoro C₁-C₁₀ alkyl" means a C₁-C₁₀ alkyl substituted with one or more fluorine atoms. Preferably, one, two, three, four or five fluorine atoms. Examples of "fluoro C₁-C₁₀ alkyl" are CF₃, CHF₂, CH₂F, CH₂CF₃, CH₂CHF₂ or CF₂CF₃.

As used herein, "aryl" means a monocyclic, bicyclic, or tricyclic monovalent or divalent (as appropriate) aromatic radical, such as phenyl, biphenyl, naphthyl, anthracenyl, which can be optionally substituted with up to five substituents preferably selected from the group of C₁-C₆ alkyl, hydroxy, C₁-C₃ hydroxyalkyl, C₁-C₃ alkoxy, C₁-C₃ haloalkoxy, amino, C₁-C₃ mono alkylamino, C₁-C₃ bis alkylamino, C₁-C₃ acylamino, C₁-C₃ aminoalkyl, mono (C₁-C₃ alkyl) amino C₁-C₃ alkyl, bis(C₁-C₃ alkyl) amino C₁-C₃ alkyl, C₁-C₃-acylamino, C₁-C₃ alkyl sulfonylamino, halo, nitro, cyano, trifluoromethyl, carboxy, C₁-C₃ alkoxycarbonyl, aminocarbonyl, mono C₁-C₃ alkyl aminocarbonyl, bis C₁-C₃ alkyl aminocarbonyl, -SO₃H, C₁-C₃ alkylsulfonyl, aminosulfonyl, mono C₁-C₃ alkyl aminosulfonyl and bis C₁-C₃-alkyl aminosulfonyl.

As used herein, "heteroaryl" means a monocyclic, bicyclic or tricyclic monovalent or divalent (as appropriate) aromatic radical containing up to four heteroatoms selected from oxygen, nitrogen and sulfur, such as thiazolyl, isothiazolyl, tetrazolyl, imidazolyl, oxazolyl, isoxazolyl, thienyl, pyrazolyl, pyridinyl, pyrazinyl, pyrimidinyl, indolyl, quinolyl, isoquinolyl, triazolyl, thiadiazolyl, oxadiazolyl, said radical being optionally substituted with up to three substituents preferably selected from the group of C₁-C₆ alkyl, hydroxy, C₁-C₃ hydroxyalkyl, C₁-C₃ alkoxy, C₁-C₃ haloalkoxy, amino, C₁-C₃ mono alkylamino, C₁-C₃ bis alkylamino, C₁-C₃ acylamino, C₁-C₃ aminoalkyl, mono (C₁-C₃ alkyl) amino C₁-C₃ alkyl, bis (C₁-C₃ alkyl) amino C₁-C₃ alkyl, C₁-C₃-acylamino, C₁-C₃ alkyl sulfonylamino, halo, nitro, cyano, trifluoromethyl, carboxy, C₁-C₃ alkoxycarbonyl, aminocarbonyl, mono C₁-C₃ alkyl aminocarbonyl, bis C₁-C₃ alkyl aminocarbonyl, -SO₃H, C₁-C₃ alkylsulfonyl, aminosulfonyl, mono C₁-C₃ alkyl aminosulfonyl and bis C₁-C₃-alkyl aminosulfonyl.
In the compounds of the invention, certain heteroaryl groups (i.e. L and R₃) are attached to R'. However, they may still be substituted by up to three additional substituents, selected from the groups defined above. Preferably, R' is the only substituent.

As used herein, the term "heterocycle" or "heterocycloalkyl" is a mono- or di-valent carbocyclic radical containing up to 4 heteroatoms selected from oxygen, nitrogen and sulfur. Preferably, it contains one or two heteroatoms. Preferably, at least one of the heteroatoms is nitrogen. It may be monocyclic or bicyclic. It is preferably saturated. Examples of heterocycles are piperidine, piperazine, thiomorpholine, morpholine, azetidine or oxetane. More preferably, the heterocycle is morpholine.

The heterocyclic ring may be mono- or di-unsaturated. The radical may be optionally substituted with up to three substituents independently selected from C₁-C₆ alkyl, hydroxy, C₁-C₃ hydroxyalkyl, C₁-C₃ alkoxy, C₁-C₃ haloalkoxy, amino, C₁-C₃ mono alkylamino, C₁-C₃ bis alkylamino, C₁-C₃ acylamino, C₁-C₃ aminoalkyl, mono (C₁-C₃ alkyl) amino C₁-C₃ alkyl , bis (C₁-C₃ alkyl) amino C₁-C₃ alkyl, C₁-C₃-acylamino, C₁-C₃ alkyl sulfonylamino, halo (e.g. F), nitro, cyano, carboxy, C₁-C₃-haloalkyl (e.g. CF₃), C₁-C₃ alkoxycarbonyl, aminocarbonyl, mono C₁-C₃ alkyl aminocarbonyl, bis C₁-C₃ alkyl aminocarbonyl, -SO₃H, C₁-C₃ alkylsulfonyl, aminosulfonyl, mono C₁-C₃ alkyl aminosulfonyl and bis C₁-C₃-alkyl aminosulfonyl.

As used herein, the above groups can be followed by the suffix -ene. This means that the group is divalent, i.e. a linker group.

As used herein, "thiol-protecting group" is typically:
(a) a protecting group that forms a thioether to protect a thiol group, for example a benzyl group which is optionally substituted by C₁-C₆ alkoxy (for example methoxy), C₁-C₆ acyloxy (for example acetoxy), hydroxy and nitro, picolyl, picolyl-N-oxide, anthrylmethyl, diphenylmethyl, phenyl, t-butyl, adamantyl, C₁-C₆ acyloxymethyl (for example pivaloyloxymethyl, tertiary butoxycarbonyloxymethyl);
(b) a protecting group that forms a monothio, dithio or aminothioacetal to protect a thiol group, for example C₁-C₆ alkoxymethyl (for example methoxymethyl, isobutoxymethyl), tetrahydropyranyl, benzylthiomethyl, phenylthiomethyl, thiazolidine, acetamidemethyl, benzamidomethyl;
(c) a protecting group that forms a thioester to protect a thiol group, such as tertiary-butyloxycarbonyl (BOC), acetyl and its derivatives, benzoyl and its derivatives; or
(d) a protecting group that forms a carbamic acid thioester to protect a thiol group, such as carbamoyl, phenylcarbamoyl, C₁-C₆ alkylcarbamoyl (for example methylcarbamoyl and ethylcarbamoyl).

In summary, each of the groups defined above, i.e., alkyl, alkenyl, alkynyl, aryl, heteroaryl, heterocycle, heterocycloalkyl, may be optionally substituted with up to three substituents preferably selected from the group of C₁-C₆ alkyl, hydroxy, C₁-C₃ hydroxyalkyl, C₁-C₃ alkoxy, C₁-C₃ haloalkoxy, amino, C₁-C₃ mono alkylamino, C₁-C₃ bis alkylamino, C₁-C₃ acylamino, C₁-C₃ aminoalkyl, mono (C₁-C₃ alkyl) amino C₁-C₃ alkyl, bis (C₁-C₃ alkyl) amino C₁-C₃ alkyl, C₁-C₃-acylamino, C₁-C₃ alkyl sulfonylamino, acyl, halo (e.g. fluoro), nitro, cyano, trifluoromethyl, carboxy, C₁-C₃ alkoxycarbonyl, aminocarbonyl, mono C₁-C₃ alkyl aminocarbonyl, bis C₁-C₃ alkyl aminocarbonyl, -SO₃H, C₁-C₃ alkylsulfonyl, aminosulfonyl, mono C₁-C₃ alkyl aminosulfonyl and bis C₁-C₃-alkyl aminosulfonyl.

It should be noted that -NH-C₁-C₁₀ alkyl, NH-acyl, NH-C(O)-NH-C₁-C₁₀ alkyl and C(O)-NH-C₁-C₁₀ alkyl can also be written as -N-C₁-C₁₀ alkyl, N-acyl, N-C(O)-N-C₁-C₁₀ alkyl and C(O)-N-C₁-C₁₀ alkyl.

As used herein, the above groups can be followed by the suffix -ene. This means that the group is divalent, i.e. a linker group.

As used herein, the term "fused" is intended to take its usual meaning within the art of organic chemistry. Fused systems, for example fused bicyclic systems, are those in which two rings share two and only two atoms.

As used herein, the term "bridged" is intended to take its usual meaning within the art of organic chemistry. Bridged compounds are compounds which contain interlocking rings. According to the invention, the atoms of the bridged non-aromatic group which form the bridgehead is either a tertiary carbon atom (when the remaining atom is hydrogen) or a quaternary carbon atom (when the remaining atom is not hydrogen). The bridge can be considered to be a chain of atoms (for example, alkyl) or a single atom (for example, O, S, N, C) connecting two bridgeheads.

As used herein, the term "spirocyclic" is intended to take its usual meaning within the art of organic chemistry. For example, a spirocyclic compound is a bicycle whose rings are attached though just one atom (known as a spiroatom). The rings may be different in size, or they may be the same size. Preferably, according to the invention, the two rings which are joined via the same atom are non-aromatic heterocycles, preferably heterocycloalkyls. For example, the spirocyclic non-aromatic group of Formula I may be a bicycle wherein both rings are heterocycloalkyl and are attached through the same atom, preferably a carbon atom.

Compounds with which the invention is concerned which may exist in one or more stereoisomeric form, because of the presence of asymmetric atoms or rotational restrictions, can exist as a number of stereoisomers with R or S stereochemistry at each chiral centre or as atropisomeres with R or S stereochemistry at each chiral axis. The invention includes all such enantiomers and diastereoisomers and mixtures thereof.

### Preferred groups of the invention - PI3K and HDAC inhibitors

In some embodiments, the PI3K inhibitor is a compound of Formula I or a pharmaceutically acceptable salt thereof, or Pictilisib, Dactolisib, Alpelisib, Voxtalisib, Gedatolisib, Copanlisib, Wortmannin, Apitolisib, Idelalisib, Buparlisib, Duvelisib, Pilaralisib, LY294002, GSK-2636771, AZD6482, PF-4989216, GS-9820, AMG319, SAR260301, MLN1117, PX-866, CH5132799, AZD8186, RP6530, GNE-317, PI-103, NU7441, HS-173, VS-5584, CZC24832, TG100-115, ZSTK474, AS-252424, AS-604850, NVP-BGT226, XL765, GDC-0032, A66, CAY10505, PF04691502, PIK-75, PIK-93, AS-605240, BGT226, CUDC-907, IC-87114, CH5132799, PKI-420, TGX-221 or PIK-90. Preferably, the PI3K inhibitor is a compound of Formula I or a pharmaceutically acceptable salt thereof. It is preferred that PI3K inhibitors of the present invention are PI3K-p110δ inhibitors (i.e. they are delta selective). Alternatively, they may be P13K-p110β and PI3K-p110δ selective (i.e. they are beta and delta selective).

In some embodiments, the HDAC inhibitor is a compound of Formula II or a pharmaceutically acceptable salt thereof, or Vorinostat, Entinostat, Panobinostat, Mocetinostat, Belinostat, Ricolinostat, Romidepsin, Givinostat, Dacinostat, Quisinostat, Pracinostat, Resminostat, Droxinostat, Abexinostat, RGFP966, AR-42, PCI34051, Trichostatin A, SB939, CI994, CUDC-907, Tubacin, Chidamide, RG2833, M344, MC1568, Tubastatin A, Scriptaid, Valproic Acid, Sodium Phenylbutyrate, Tasquinimod, Kevetrin, HPOB, 4SC-202, TMP269, CAY10603, BRD73954, BG45, LMK-235, Nexturastat A, CG200745, CHR2845 or CHR3996. Preferably, the HDAC inhibitor is a compound of Formula II or a pharmaceutically acceptable salt thereof. It is preferred that the HDAC inhibitors of the present invention are HDAC6 selective. For example, they are selective for HDAC6 over HDAC1.

### Preferred groups of the invention - compounds of formula I

Preferably, a compound of formula I is as defined in claim 1, but may additionally be a compound where at least one R³ is NH₂.

Preferably, R¹ is represented by any of the following structures:

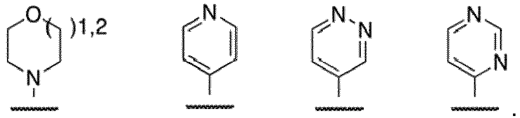

Most preferably, R¹ is morpholine.

In a preferred embodiment of a compound of formula I, W is oxygen or sulfur, preferably oxygen.

Preferably X is CH.

Preferably R³ is H, C₁-C₁₀ alkyl, halogen or fluoro C₁-C₁₀ alkyl. More preferably R³ is H.

Preferably, the 6,5-ring system in Formula I is an indole. In other words, R³ is hydrogen and X is CH.

R² may be attached to any suitable atom on the aryl group, as depicted in general formula I. However, it is preferred that R² is attached to the meta-position of the pyridine ring. For example, if the nitrogen atom of the pyridine is labelled as atom number 1, then R² is attached in the 3-position.

R² is LY. Preferably, L is C₁-C₁₀ alkylene, preferably methylene.

Preferably, Y is a an optionally substituted bridged or spirocyclic heterocycloalkyl group containing up to 4 heteroatoms selected from N or O, and comprising 5 to 12 atoms in total.

Preferably, Y contains one or two heteroatoms, preferably two heteroatoms. More preferably, at least one of the heteroatoms is nitrogen and Y is bonded to L through the nitrogen atom, as depicted in the preferable Y groups below: 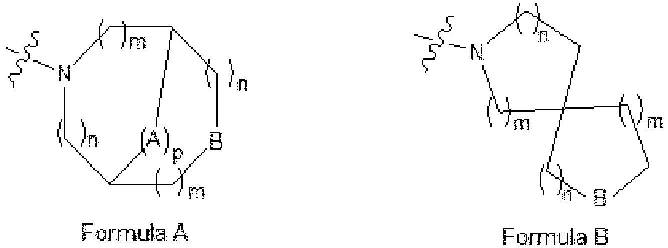 or wherein:
A is selected from the group consisting of O, S, NR⁴, optionally substituted C₁-C₃ alkylene, C₂-C₃ alkenylene and C₂-C₃ alkynylene;
B is selected from the group consisting of NR⁴, O and CH₂;
wherein R⁴ is selected from the group consisting of H, optionally substituted C₁-C₁₀ alkyl, C₂-C₁₀ alkenyl, C₂-C₁₀ alkynyl and C₁-C₃ halofluoroalkyl;
p is selected from 0, 1 or 2;
each m is independently selected from 0, 1 or 2; and
each n is independently selected from 1, 2 or 3.

Preferably, A is O or C₁-C₃ alkylene, most preferably methylene.

Preferably, B is O or CH₂, most preferably O.

When R⁴ is present, it is preferably H, C₁-C₃ alkyl or C₁-C₃ halofluoroalkyl. More preferably, R⁴ is H.

Preferably, each m and n is selected so as to form 5-, 6- or 7-membered nitrogen containing heterocycloalkyl groups. Preferably, p is 1. In particular, when A is O, S or NR⁴, p is 1.

Y is preferably bicyclic, more preferably bridged bicyclic or spirocyclic bicyclic.

Even more preferably, Y is selected from one of the following groups:

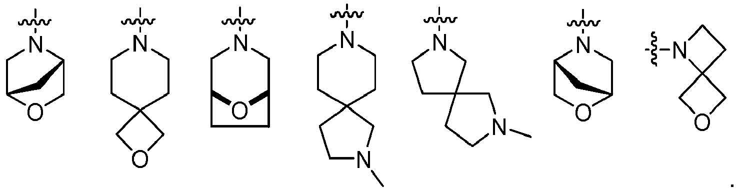

In certain embodiments, provided herein are compounds represented by: 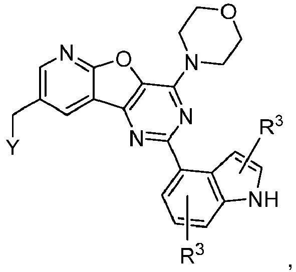 where Y and R³ are defined above.

In another embodiment, provided herein are compounds represented by: 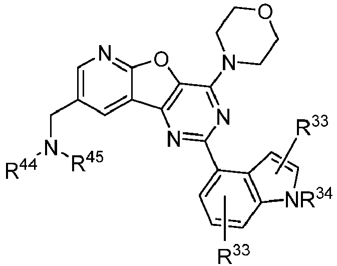 and pharmaceutically acceptable salts thereof, wherein:
R₃₃ is independently selected for each occurrence from the group consisting of H, halogen, NH-C₁₋₃alkyl, NH₂, C₁₋₆alkyl and -O-C₁₋₆alkyl (wherein C₁₋₆alkyl for each occurrence is optionally substituted by one, two or three substituents selected from halogen and hydroxyl);
R³⁴ is selected from H or C₁₋₃alkyl;
R⁴⁴ and R⁴⁵, when taken together with the nitrogen to which they are attached form a 7 -10 membered bicyclic spirocycle or bridged heterocycle each having an additional heteroatom selected from O, S, or NR⁵⁵, wherein R⁵⁵ is H or C₁₋₃alkyl.

For example, R⁴⁴ and R⁴⁵, when taken together with the nitrogen to which they are attached may form a 7 -8 membered bicyclic bridged heterocycle represented by: 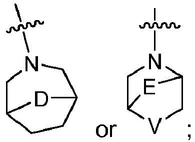 wherein D is O, S or NR⁵⁵,; E is O or (CH₂)ᵣ, wherein r is 1 or 2, and V is O or NR⁵⁵, wherein R⁵⁵ is H or C₁₋₃alkyl.

In another exemplary embodiment, R⁴⁴ and R⁴⁵, when taken together with the nitrogen to which they are attached form a 7 -10 membered spirocycle having one additional heteroatom selected from O or NR⁵⁵, wherein R⁵⁵ is H or C₁₋₃alkyl. Alternatively, R⁴⁴ and R⁴⁵, taken together with the nitrogen to which they are attached may be a Y substituent as described above.

Examples of structures embodying formula I are:

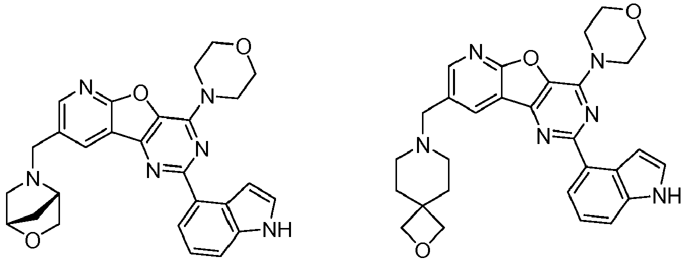

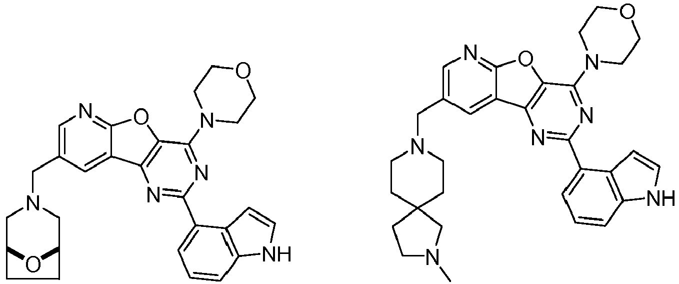

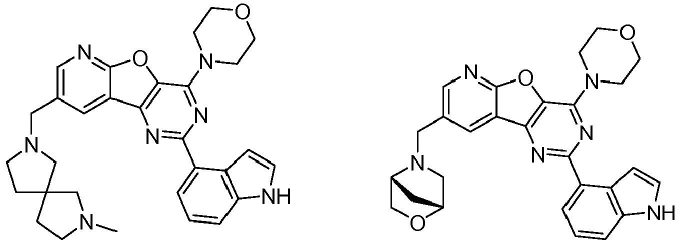

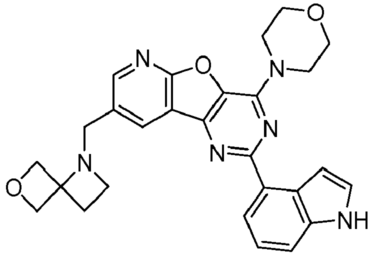

### Preferred groups of the invention - Compounds of Formula II

Preferably, at least one R² is H. Preferably, both R² groups are H.

The group W is a zinc-chelating residue, i.e. a metallophile capable of binding with zinc in the active site of HDAC. Suitable metallophiles are known to those skilled in the art.

In a preferred embodiment, W is selected from: 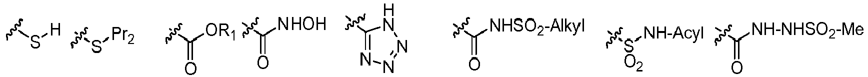 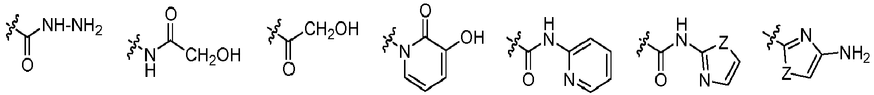 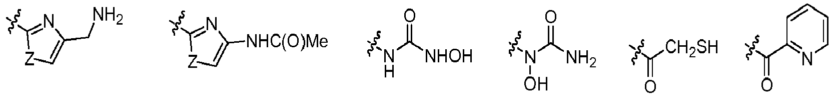 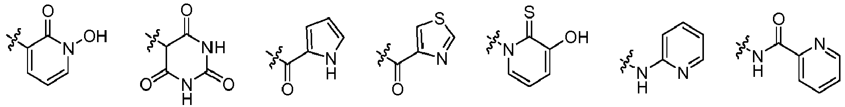 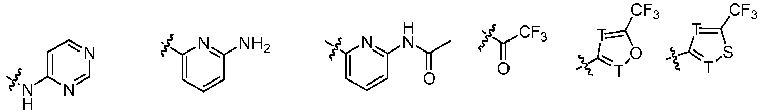 wherein R₁ is as defined in claim 1, Pr² is H or a thiol protecting group, Z is selected from O, S or NH and T is N or CH.

When W is COOR₁, preferably R₁ is not halogen. More preferably, when W is COOR₁, R₁ is H or C₁-C₁₀ alkyl.

Preferably, W is -COOH, -CONHOH, CONHSO₂CH₃, -CONHNHSO₂CH₃, -CONHNH₂, -CONH(2-pyridyl), -NHCONHOH, tetrazole, hydroxypyridin-2-thione or hydroxypyridin-2-one. Preferably W is not COOR₁. More preferably, W is COOMe, -CONHOH, CONHSO₂CH₃, -CONHNHSO₂CH₃, -CONHNH₂, -CONH(2-pyridyl) -NHCONHOH, tetrazole, hydroxypyridin-2-thione or hydroxypyridin-2-one. Even more preferably, W is -CONHOH, tetrazole, hydroxypyridin-2-thione or hydroxypyridin-2-one. Most preferably, W is -CONHOH.

In a preferred embodiment, in at least one, preferably both L groups, the atom that is directly bonded to X is a carbon, and at least one nitrogen atom is directly bonded to said carbon.

In an embodiment, at least one L group is a 5-membered heteroaryl. Preferably, at least one L group is a 6-membered heteroaryl. Even more preferably, both L groups are a 6-membered heteroaryl.

Preferably, at least one L group is pyridinyl, pyrimidinyl, pyridazinyl, oxadiazolyl, pyrazolyl, thiadiazolyl, pyrazinyl, benzofused thiazolyl, benzofused oxazolyl or benzofused imidazolyl. More preferably, at least one L group is pyridyl or pyrazinyl. Most preferably, one L is pyrazinyl and one L is pyridyl. Preferably, when L is pyridyl, it is substituted with a heteroaryl group. The heteroaryl group is preferably an optionally substituted (preferably substituted) pyridine.

Preferably, at least one L group is pyridinyl, oxadiazolyl, pyrazolyl, thiadiazolyl, pyrazinyl, benzofused thiazolyl, benzofused oxazolyl or benzofused imidazolyl.

Preferably, at least one L group is a 5 or 6-membered heteroaryl, which is optionally fused to a benzene.

Preferably, Q is a bond or O.

Preferably, R³ is aryl. More preferably, R³ is phenylene or phenylene substituted with a halogen.

Preferably, at least one, preferably both, R² is H.

In a preferred embodiment, at least one R' is H, halogen, CF₃, C₁-C₆ alkyl, aryl optionally substituted with halogen or heteroaryl optionally substituted with halogen. Preferably, the alkyl is substituted with at least one halogen, which is preferably fluorine.

In a preferred embodiment, the R' attached to R³ is hydrogen or halogen. Preferably, R³ is hydrogen or fluorine. More preferably, the R' attached to R³ is hydrogen. In a preferred embodiment, at least one R', and preferably at least one of the R' that is attached to L, is H, C₁-C₁₀ alkyl or O-(C₁-C₁₀ alkyl). Preferably, at least one R^{/} is substituted or unsubstituted aryl or O-(substituted or unsubstituted aryl). Preferably, at least one R^{/} is aryl or O-aryl, each of which may be substituted with a halogen, amino or C₁-C₁₀ alkyl. The aryl may be substituted in any position. The aryl may be mono-, bis-, or tri-substituted.
In a preferred embodiment, at least one R', and preferably at least one of the R' that is attached to L, is H, C₁-C₁₀ alkyl or O-(C₁-C₁₀ alkyl), halogen, C₁-C₁₀ heterocycloalkyl, aryl (preferably optionally substituted phenyl), trifluoromethyl or heteroaryl, preferably heteroaryl. Preferably, when R' is heteroaryl, it is optionally substituted pyridyl, preferably a substituted pyridyl.

In one embodiment, at least one R' that is attached to L is OCH₃ or CH₃. Preferably, at least one of the R' that is attached to L is heterocycloalkyl. Preferably, the heterocycloalkyl is morpholino.

In a preferred embodiment, when Q is a direct bond, R₁ is H, C₁-C₁₀ alkyl or O-(C₁-C₁₀ alkyl), halogen (preferably F), C₁-C₁₀ heterocycloalkyl (preferably morpholino), aryl (preferably optionally substituted phenyl), trifluoromethyl or heteroaryl, preferably heteroaryl. Preferably, when R₁ is heteroaryl, it is optionally substituted pyridyl, preferably a substituted pyridyl.

In a preferred embodiment, R₁ is C₁-C₁₀ alkyl, C₂-C₁₀ alkenyl or C₂-C₁₀ alkynyl, preferably those groups are substituted with halogen, NH₂, NO₂ or hydroxyl. More preferably, when R^{/} or R₁ is C₁-C₁₀ alkyl, it may be substituted with halogen which is preferably fluorine. The C₁-C₁₀ alkyl group may be substituted by up to 10 halogen atoms or preferably, by up to 5 halogen atoms, i.e., 1, 2, 3, 4 or 5 halogen atoms. For example, R^{/} or R₁ may be CF₃, CHF₂, CH₂CF₃, CH₂CHF₂ or CF₂CF₃ or OCF₃, OCHF₂, OCH₂CF₃, OCH₂CHF₂ or OCF₂CF₃.

R^{/} may be substituted onto any of the ring atoms of the L group or onto any of the ring atoms of the R² group.

Preferably, the L and R³ groups have no other substitutions other than R'. Preferably, Q is a direct bond.

Preferably, in addition to a N atom, L contains at least one other heteroatom in the heteroaryl ring which is selected from N, O or S.

In a preferred embodiment, L is:

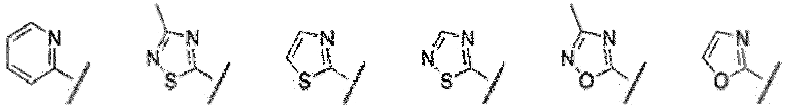

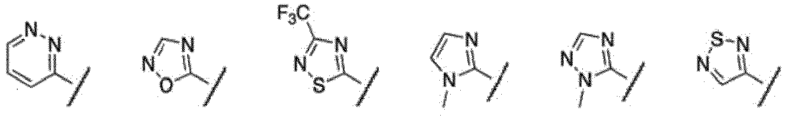

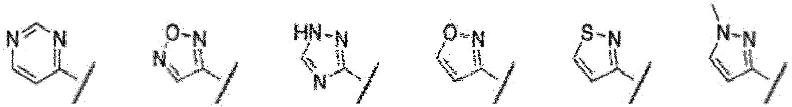

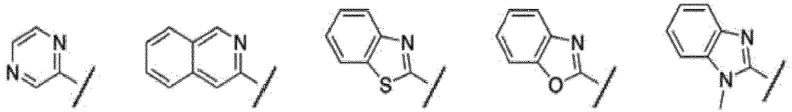

In a preferred embodiment, L is a hydrogen bond-acceptor, and preferably not also a hydrogen bond donor. Preferably, L does not have a hydrogen atom attached to an electronegative atom, such as N or O.

The definition of hydrogen bond acceptors/donors is known to those skilled in the art. For example, a hydrogen bond donor will have a hydrogen attached to an electronegative atom, such as N or O. For example, a hydrogen bond acceptor will have a N or O, which has a free lone pair.

Preferably the atom of L that is directly bonded to the N atom of the formula of claim 1 is carbon, and at least one nitrogen atom is directly bonded to said carbon (preferably via a double bond). More preferably, said nitrogen atom is a hydrogen bond acceptor.

### General description - compositions (combinations)

A pharmaceutical composition of the invention comprises a compound as defined above, and a pharmaceutically acceptable carrier or diluent. A pharmaceutical composition of the invention typically contains up to 85 wt% of a compound of the invention. More typically, it contains up to 50 wt% of a compound of the invention. Preferred pharmaceutical compositions are sterile and pyrogen-free. Further, the pharmaceutical compositions provided by the invention typically contain a compound of the invention which is a substantially pure optical isomer. Preferably, the pharmaceutical composition comprises a pharmaceutically acceptable salt form of a compound of the invention. For example, contemplated herein is a pharmaceutically acceptable composition comprising a disclosed compound and a pharmaceutically acceptable excipient.

As used herein, a pharmaceutically acceptable salt is a salt with a pharmaceutically acceptable acid or base. Pharmaceutically acceptable acids include both inorganic acids such as hydrochloric, sulfuric, phosphoric, diphosphoric, hydrobromic or nitric acid and organic acids such as citric, fumaric, maleic, malic, ascorbic, succinic, tartaric, benzoic, acetic, methanesulfonic, ethanesulfonic, salicylic, stearic, benzenesulfonic or p-toluenesulfonic acid. Pharmaceutically acceptable bases include alkali metal (e.g. sodium or potassium) and alkali earth metal (e.g. calcium or magnesium) hydroxides and organic bases such as alkyl amines, aryl amines or heterocyclic amines.

For the avoidance of doubt, the present invention also embraces prodrugs which react *in vivo* to give a compound of the present invention.

The compounds of the invention may be prepared by synthetic routes that will be apparent to those skilled in the art, e.g. based on the Examples.

The compounds of the invention and compositions comprising them may be administered in a variety of dosage forms. In one embodiment, a pharmaceutical composition comprising a compound of the invention may be formulated in a format suitable for oral, rectal, parenteral, intranasal or transdermal administration or administration by inhalation or by suppository. Typical routes of administration are parenteral, intranasal or transdermal administration or administration by inhalation.

The compounds of the invention can be administered orally, for example as tablets, troches, lozenges, aqueous or oily suspensions, dispersible powders or granules. Preferred pharmaceutical compositions of the invention are compositions suitable for oral administration, for example tablets and capsules. In some embodiments, disclosed compounds may have significantly higher oral bioavailability as compared to compounds having a non-spirocycle or non-bridged heterocyclic moiety, e.g., at R² above..

The compounds of the invention may also be administered parenterally, whether subcutaneously, intravenously, intramuscularly, intrasternally, transdermally or by infusion techniques. The compounds may also be administered as suppositories.

The compounds of the invention may also be administered by inhalation. An advantage of inhaled medications is their direct delivery to the area of rich blood supply in comparison to many medications taken by oral route. Thus, the absorption is very rapid as the alveoli have an enormous surface area and rich blood supply and first pass metabolism is bypassed. A further advantage may be to treat diseases of the pulmonary system, such that delivering drugs by inhalation delivers them to the proximity of the cells which are required to be treated.

The present invention also provides an inhalation device containing such a pharmaceutical composition. Typically said device is a metered dose inhaler (MDI), which contains a pharmaceutically acceptable chemical propellant to push the medication out of the inhaler.

The compounds of the invention may also be administered by intranasal administration. The nasal cavity's highly permeable tissue is very receptive to medication and absorbs it quickly and efficiently, more so than drugs in tablet form. Nasal drug delivery is less painful and invasive than injections, generating less anxiety among patients. By this method absorption is very rapid and first pass metabolism is usually bypassed, thus reducing inter-patient variability. Further, the present invention also provides an intranasal device containing such a pharmaceutical composition.

The compounds of the invention may also be administered by transdermal administration. The present invention therefore also provides a transdermal patch containing a compound of the invention.

The compounds of the invention may also be administered by sublingual administration. The present invention therefore also provides a sub-lingual tablet comprising a compound of the invention.

A compound of the invention may also be formulated with an agent which reduces degradation of the substance by processes other than the normal metabolism of the patient, such as anti-bacterial agents, or inhibitors of protease enzymes which might be the present in the patient or in commensural or parasite organisms living on or within the patient, and which are capable of degrading the compound.

Liquid dispersions for oral administration may be syrups, emulsions and suspensions.

Suspensions and emulsions may contain as carrier, for example a natural gum, agar, sodium alginate, pectin, methylcellulose, carboxymethylcellulose, or polyvinyl alcohol. The suspension or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for injection or infusion may contain as carrier, for example, sterile water or preferably they may be in the form of sterile, aqueous, isotonic saline solutions.

Where a kit and/or a method of the invention provides for the administration of more than one drug, they can be administered simultaneous, sequentially or separately. It is not necessary that they are packed together (but this is one embodiment of the invention). It is also not necessary that they are administered at the same time or that they are in the same dosage form. As used herein, "separate" administration means that the drugs are administered as part of the same overall dosage regimen (which could comprise a number of days), but preferably on the same day. As used herein "simultaneously" means that the drugs are to be taken together or formulated as a single composition. As used herein, "sequentially" means that the drugs are administered at about the same time, and preferably within about 1 hour of each other.

In some embodiments, a disclosed PI3K inhibitor may be administered at certain dosages (e.g., lower dosages than monotherapy) but may be therapeutically effective when combined with a HDAC inhibitor (e.g., HDAC6 specific inhibitor) For example, the combination of the HDAC inhibitor and the phosphatidylinositide 3-kinase (PI3K) inhibitor may achieve a synergistic effect in the treatment of the subject in need thereof, wherein the combination is administered at dosages that would not be effective when one or both of the compounds are administered alone, but which amounts are effective in combination.

### General disclosure - methods of use

The compositions or compounds of the present invention can be used in both the treatment and prevention of cancer and can be used in the combination therapy of the invention or in further combination. When used in a further combination therapy, the compounds of the present invention are typically used together with small chemical compounds such as platinum complexes, antimetabolites, DNA topoisomerase inhibitors, radiation, antibody-based therapies (for example herceptin and rituximab), anti-cancer vaccination, gene therapy, cellular therapies, hormone therapies or cytokine therapy.

In one embodiment of the invention a composition of the invention is used in further combination with another chemotherapeutic or antineoplastic agent in the treatment of a cancer. Examples of such other chemotherapeutic or antineoplastic agents include platinum complexes including cisplatin and carboplatin, mitoxantrone, vinca alkaloids for example vincristine and vinblastine, anthracycline antibiotics for example daunorubicin and doxorubicin, alkylating agents for example chlorambucil and melphalan, taxanes for example paclitaxel, antifolates for example methotrexate and tomudex, epipodophyllotoxins for example etoposide, camptothecins for example irinotecan and its active metabolite SN38 and DNA methylation inhibitors for example the DNA methylation inhibitors disclosed in WO02/085400.

According to the invention, therefore, products are provided which contain a composition of the invention and another chemotherapeutic or antineoplastic agent as a combined preparation for simultaneous, separate or sequential use in alleviating a cancer. Also provided according to the invention is the use of compound of the invention in the manufacture of a medicament for use in the alleviation of cancer by coadministration with another chemotherapeutic or antineoplastic agent. The compound of the invention and the said other agent may be administrated in any order. In both these cases the compound of the invention and the other agent may be administered together or, if separately, in any order as determined by a physician.

The compound combinations disclosed herein may also be used to treat abnormal cell proliferation due to insults to body tissue during surgery in a human patient. These insults may arise as a result of a variety of surgical procedures such as joint surgery, bowel surgery, and cheloid scarring. Diseases that produce fibrotic tissue that may be treated using the combinations of the present invention include emphysema. Repetitive motion disorders that may be treated using the present invention include carpal tunnel syndrome. An example of a cell proliferative disorder that may be treated using the invention is a bone tumour.

Proliferative responses associated with organ transplantation that may be treated using combinations of the invention include proliferative responses contributing to potential organ rejections or associated complications. Specifically, these proliferative responses may occur during transplantation of the heart, lung, liver, kidney, and other body organs or organ systems.

Abnormal angiogenesis that may be treated using this invention include those abnormal angiogenesis accompanying rheumatoid arthritis, ischemic-reperfusion related brain edema and injury, cortical ischemia, ovarian hyperplasia and hypervascularity, polycystic ovary syndrome, endometriosis, psoriasis, diabetic retinopathy, and other ocular angiogenic diseases such as retinopathy of prematurity (retrolental fibroplastic), macular degeneration, corneal graft rejection, neuroscular glaucoma and Osler-Weber-Rendu syndrome.

Examples of diseases associated with uncontrolled angiogenesis that may be treated according to the present invention include, but are not limited to, retinal/choroidal neovascularisation and corneal neovascularisation. Examples of diseases which include some component of retinal/choroidal neovascularisation include, but are not limited to, Best's diseases, myopia, optic pits, Stargart's diseases, Paget's disease, vein occlusion, artery occlusion, sickle cell anaemia, sarcoid, syphilis, pseudoxanthoma elasticum carotid apo structive diseases, chronic uveitis/vitritis, mycobacterial infections, Lyme's disease, systemic lupus erythematosus, retinopathy of prematurity, Eale's disease, diabetic retinopathy, macular degeneration, Bechet's diseases, infections causing a retinitis or chroiditis, presumed ocular histoplasmosis, pars planitis, chronic retinal detachment, hyperviscosity syndromes, toxoplasmosis, trauma and post-laser complications, diseases associated with rubesis (neovascularisation of the angle) and diseases caused by the abnormal proliferation of fibrovascular or fibrous tissue including all forms of proliferative vitreoretinopathy. Examples of corneal neovascularisation include, but are not limited to, epidemic keratoconjunctivitis, Vitamin A deficiency, contact lens overwear, atopic keratitis, superior limbic keratitis, pterygium keratitis sicca, Sjogrens, acne rosacea, phylectenulosis, diabetic retinopathy, retinopathy of prematurity, corneal graft rejection, Mooren ulcer, Terrien's marginal degeneration, marginal keratolysis, polyarteritis, Wegener sarcoidosis, Scleritis, periphigoid radial keratotomy, neovascular glaucoma and retrolental fibroplasia, syphilis, mycobacteria infections, lipid degeneration, chemical burns, bacterial ulcers, fungal ulcers, Herpes simplex infections, Herpes zoster infections, protozoan infections and Kaposi sarcoma.

Chronic inflammatory diseases associated with uncontrolled angiogenesis may also be treated using combinations of the present invention. Chronic inflammation depends on continuous formation of capillary sprouts to maintain an influx of inflammatory cells. The influx and presence of the inflammatory cells produce granulomas and thus maintains the chronic inflammatory state. Inhibition of angiogenesis using a combination of the invention alone or in conjunction with other anti-inflammatory agents may prevent the formation of the granulosmas and thus alleviate the disease. Examples of chronic inflammatory diseases include, but are not limited to, inflammatory bowel diseases such as Crohn's disease and ulcerative colitis, psoriasis, sarcoidosis, and rheumatoid arthritis.

Inflammatory bowel diseases such as Crohn's disease and ulcerative colitis are characterised by chronic inflammation and angiogenesis at various sites in the gastrointestinal tract. For example, Crohn's disease occurs as a chronic transmural inflammatory disease that most commonly affects the distal ileum and colon but may also occur in any part of the gastrointestinal tract from the mouth to the anus and perianal area. Patients with Crohn's disease generally have chronic diarrhoea associated with abdominal pain, fever, anorexia, weight loss and abdominal swelling. Ulcerative colitis is also a chronic, nonspecific, inflammatory and ulcerative disease arising in the colonic mucosa and is characterised by the presence of bloody diarrhoea. These inflammatory bowel diseases are generally caused by chronic granulomatous inflammation throughout the gastrointestinal tract, involving new capillary sprouts surrounded by a cylinder of inflammatory cells. Inhibition of angiogenesis by these inhibitors should inhibit the formation of the sprouts and prevent the formation of granulomas. Inflammatory bowel diseases also exhibit extra intestinal manifestations, such as skin lesions. Such lesions are characterized by inflammation and angiogenesis and can occur at many sites other than the gastrointestinal tract. Inhibition of angiogenesis by combinations according to the present invention can reduce the influx of inflammatory cells and prevent lesion formation.

Sarcoidosis, another chronic inflammatory disease, is characterized as a multisystem granulomatous disorder. The granulomas of this disease can form anywhere in the body. Thus, the symptoms depend on the site of the granulomas and whether the disease is active. The granulomas are created by the angiogenic capillary sprouts providing a constant supply of inflammatory cells. By using combinations according to the present invention to inhibit angiogenesis, such granulomas formation can be inhibited. Psoriasis, also a chronic and recurrent inflammatory disease, is characterised by papules and plaques of various sizes. Treatment using these inhibitors alone or in conjunction with other anti-inflammatory agents should prevent the formation of new blood vessels necessary to maintain the characteristic lesions and provide the patient relief from the symptoms.

Rheumatoid arthritis (RA) is also a chronic inflammatory disease characterised by non-specific inflammation of the peripheral joints. It is believed that the blood vessels in the synovial lining of the joints undergo angiogenesis. In addition to forming new vascular networks, the endothelial cells release factors and reactive oxygen species that lead to pannus growth and cartilage destruction. The factors involved in angiogenesis may actively contribute to, and help maintain, the chronically inflamed state of rheumatoid arthritis. Treatment using combinations according to the present invention alone or in conjunction with other anti-RA agents may prevent the formation of new blood vessels necessary to maintain the chronic inflammation.

Preferably, the condition is cancer, notably leukaemias including chronic myelogenous leukaemia and acute myeloid leukaemia, lymphomas, solid tumours, and PTEN-negative and/or PTEN-defective tumours including PTEN-negative haematological, breast, lung, endometrial, skin, brain and prostate cancers (where PTEN refers to "phosphatase and tensin homolog deleted on chromosome 10"). More preferably, the condition to be treated in a patient in need theref by administering an effective amount of a disclosedcompound is a disorder selected from rheumatoid arthritis, asthma, chronic obstructive pulmonary disease (COPD), multiple sclerosis, psoriasis and other inflammatory skin disorders, systemic lupus erythematosus, inflammatory bowel disease, and organ transplant rejection. For example, provided herein is a method of treating a patient suffering a disorder selected from the group consisting leukaemias (including e.g., chronic myelogenous leukaemia and acute myeloid leukaemia), lymphoma, a solid tumour cancer such as breast, lung, or prostate cancer, PTEN-negative tumours including PTEN-negative haematological, breast, lung, endometrial, skin, brain and prostate cancers (where PTEN refers to "phosphatase and tensin homolog deleted on chromosome 10") comprising administering an effective amount of a disclosed compound.

HDAC is believed to contribute to the pathology and/or symptomology of several different diseases such that reduction of the activity of HDAC in a subject through inhibition of HDAC may be used to therapeutically address these disease states. Examples of various diseases that may be treated using the HDAC inhibitors of the present invention in combination with the PI3K inhibitors of the present invention are described herein.

One set of indications that combinations of the present invention may be used to treat is those involving undesirable or uncontrolled cell proliferation. Such indications include benign tumours, various types of cancers such as primary tumours and tumour metastasis, restenosis (e.g. coronary, carotid, and cerebral lesions), abnormal stimulation of endothelial cells (atherosclerosis), insults to body tissue due to surgery, abnormal wound healing, abnormal angiogenesis, diseases that produce fibrosis of tissue, repetitive motion disorders, disorders of tissues that are not highly vascularized, and proliferative responses associated with organ transplants. More specific indications for the combinations of the invention include, but are not limited to prostate cancer, lung cancer, acute leukaemia, multiple myeloma, bladder carcinoma, renal carcinoma, breast carcinoma, colorectal carcinoma, neuroblastoma and melanoma.

In one embodiment, a method is provided for treating diseases associated with undesired and uncontrolled cell proliferation. The method comprises administering to a subject suffering from uncontrolled cell proliferation a therapeutically effective amount of a HDAC inhibitor in combination with a PI3K inhibitor, according to the present invention, such that said uncontrolled cell proliferation is reduced. The particular dosage of the inhibitor to be used will depend on the severity of the disease state, the route of administration, and related factors that can be determined by the attending physician. Generally, acceptable and effective daily doses are amounts sufficient to effectively slow or eliminate uncontrolled cell proliferation.

Combinations according to the present invention may also be used in conjunction with other agents to inhibit undesirable and uncontrolled cell proliferation. Examples of other anti-cell proliferation agents that may be used in conjunction with the combinations of the present invention include, but are not limited to, retinoid acid and derivatives thereof, 2-methoxyestradiol, Angiostatin™ protein, Endostatin™ protein, suramin, squalamine, tissue inhibitor of metalloproteinase-I, tissue inhibitor of metalloproteinase-2, plasminogen activator inhibitor-1, plasminogen activator inhibitor-2, cartilage-derived inhibitor, paclitaxel, platelet factor 4, protamine sulfate (clupeine), sulfated chitin derivatives (prepared from queen crab shells), sulfated polysaccharide peptidoglycan complex (sp-pg), staurosporine, modulators of matrix metabolism, including for example, proline analogs ((1-azetidine-2-carboxylic acid (LACA), cishydroxyproline, d,I-3,4-dehydroproline, thiaproline), beta-aminopropionitrile fumarate, 4-propyl-5-(4-pyridinyl)-2(3H)-oxazolone; methotrexate, mitoxantrone, heparin, interferons, 2 macroglobulin-serum, chimp-3, chymostatin, betacyclodextrin tetradecasulfate, eponemycin; fumagillin, gold sodium thiomalate, d-penicillamine (CDPT), beta-1-anticollagenase-serum, alpha-2-antiplasmin, bisantrene, lobenzarit disodium, n-(2-carboxyphenyl-4-chloroanthronilic acid disodium or "CCA", thalidomide; angiostatic steroid, carboxyaminoimidazole; metalloproteinase inhibitors such as BB94. Other anti-angiogenesis agents that may be used include antibodies, preferably monoclonal antibodies against these angiogenic growth factors: bFGF, aFGF, FGF-5, VEGF isoforms, VEGF-C, HGF/SF and Ang-1/Ang-2. Ferrara N. and Alitalo, K. "Clinical application of angiogenic growth factors and their inhibitors" (1999) Nature Medicine 5:1359-1364.

Generally, cells in benign tumours retain their differentiated features and do not divide in a completely uncontrolled manner. A benign tumour is usually localized and nonmetastatic. Specific types of benign tumours that can be treated using combinations of the present invention include hemangiomas, hepatocellular adenoma, cavernous haemangioma, focal nodular hyperplasia, acoustic neuromas, neurofibroma, bile duct adenoma, bile duct cystanoma, fibroma, lipomas, leiomyomas, mesotheliomas, teratomas, myxomas, nodular regenerative hyperplasia, trachomas and pyogenic granulomas.

In the case of malignant tumors, cells become undifferentiated, do not respond to the body's growth control signals, and multiply in an uncontrolled manner. Malignant tumors are invasive and capable of spreading to distant sites (metastasizing). Malignant tumors are generally divided into two categories: primary and secondary. Primary tumors arise directly from the tissue in which they are found. Secondary tumours, or metastases, are tumours that originated elsewhere in the body but have now spread to distant organs. Common routes for metastasis are direct growth into adjacent structures, spread through the vascular or lymphatic systems, and tracking along tissue planes and body spaces (peritoneal fluid, cerebrospinal fluid, etc.).

Specific types of cancers or malignant tumours, either primary or secondary, that can be treated using disclosed combinations of HDAC inhibitors and PI3K inhibitors of the present invention include, but are not limited to, leukaemia, breast cancer, skin cancer, bone cancer, prostate cancer, liver cancer, lung cancer, brain cancer, cancer of the larynx, gallbladder, pancreas, rectum, parathyroid, thyroid, adrenal, neural tissue, head and neck, colon, stomach, bronchi, kidneys, basal cell carcinoma, squamous cell carcinoma of both ulcerating and papillary type, metastatic skin carcinoma, osteo sarcoma, Ewing's sarcoma, veticulum cell sarcoma, myeloma, giant cell tumour, small-cell lung tumour, gallstones, islet cell tumour, primary brain tumour, acute and chronic lymphocytic and granulocytic tumours, hairy-cell tumour, adenoma, hyperplasia, medullary carcinoma, pheochromocytoma, mucosal neuromas, intestinal ganglloneuromas, hyperplastic corneal nerve tumour, marfanoid habitus tumour, Wilms' tumour, seminoma, ovarian tumour, leiomyomater tumour, cervical dysplasia and in situ carcinoma, neuroblastoma, retinoblastoma, soft tissue sarcoma, malignant carcinoid, topical skin lesion, mycosis fungoide, rhabdomyosarcoma, Kaposi's sarcoma, osteogenic and other sarcoma, malignant hypercalcemia, renal cell tumour, polycythermia vera, adenocarcinoma, glioblastoma multiforme, leukemias, lymphomas, malignant melanomas, epidermoid carcinomas, and other carcinomas and sarcomas.

The combinations of the present invention may also be used to treat abnormal cell proliferation due to insults to body tissue during surgery. These insults may arise as a result of a variety of surgical procedures such as joint surgery, bowel surgery, and cheloid scarring. Diseases that produce fibrotic tissue that may be treated using the combinations of the present invention include emphysema. Repetitive motion disorders that may be treated using the present invention include carpal tunnel syndrome. An example of a cell proliferative disorder that may be treated using the invention is a bone tumour.

Proliferative responses associated with organ transplantation that may be treated using combinations of the invention include proliferative responses contributing to potential organ rejections or associated complications. Specifically, these proliferative responses may occur during transplantation of the heart, lung, liver, kidney, and other body organs or organ systems.

Sarcoidosis, another chronic inflammatory disease, is characterized as a multisystem granulomatous disorder. The granulomas of this disease can form anywhere in the body. Thus, the symptoms depend on the site of the granulomas and whether the disease is active. The granulomas are created by the angiogenic capillary sprouts providing a constant supply of inflammatory cells. By using combinations according to the present invention to inhibit angiogenesis, such granulomas formation can be inhibited. Psoriasis, also a chronic and recurrent inflammatory disease, is characterized by papules and plaques of various sizes. Treatment using these inhibitors alone or in conjunction with other anti-inflammatory agents should prevent the formation of new blood vessels necessary to maintain the characteristic lesions and provide the patient relief from the symptoms.

Rheumatoid arthritis (RA) is also a chronic inflammatory disease characterized by non-specific inflammation of the peripheral joints. It is believed that the blood vessels in the synovial lining of the joints undergo angiogenesis. In addition to forming new vascular networks, the endothelial cells release factors and reactive oxygen species that lead to pannus growth and cartilage destruction. The factors involved in angiogenesis may actively contribute to, and help combinations according to the present invention alone or in conjunction with other anti-RA agents may prevent the formation of new blood vessels necessary to maintain the chronic inflammation.

The compounds of the present invention can further be used in the treatment of cardiac/vasculature diseases such as hypertrophy, hypertension, myocardial infarction, reperfusion, ischaemic heart disease, angina, arrhythmias, hypercholesterolemia, atherosclerosis and stroke. The compounds can further be used to treat neurodegenerative disorders/CNS disorders such as acute and chronic neurological diseases, including stroke, Huntington's disease, Amyotrophic Lateral Sclerosis and Alzheimer's disease.

The compounds of the present invention can also be used as antimicrobial agents, for example antibacterial agents. The invention therefore also provides a compound for use in the treatment of a bacterial infection. The compounds of the present invention can be used as anti-infectious compounds against viral, bacterial, fungal and parasitic infections. Examples of infections include protozoal parasitic infections (including plasmodium, cryptosporidium parvum, toxoplasma gondii, sarcocystis neurona and Eimeria sp.)

The compounds of the present invention are particularly suitable for the treatment of undesirable or uncontrolled cell proliferation, preferably for the treatment of benign tumours/hyperplasias and malignant tumours, more preferably for the treatment of malignant tumours and most preferably for the treatment of chronic lymphocytic leukaemia (CLL), breast cancer, prostate cancer, ovarian cancer, mesothelioma, T-cell lymphoma.

In a preferred embodiment of the invention, the compounds of the invention are used to alleviate cancer, cardiac hypertrophy, chronic heart failure, an inflammatory condition, a cardiovascular disease, a haemoglobinopathy, a thalassemia, a sickle cell disease, a CNS disorder, an autoimmune disease, organ transplant rejection, diabetes, osteoporosis, MDS, benign prostatic hyperplasia, oral leukoplakia, a genentically related metabolic disorder, an infection, Rubens-Taybi, fragile X syndrome, or alpha-1 antitrypsin deficiency, or to accelerate wound healing, to protect hair follicles or as an immunosuppressant.

Typically, said inflammatory condition is a skin inflammatory condition (for example psoriasis, acne and eczema), asthma, chronic obstructive pulmonary disease (COPD), rheumatoid arthritis (RA), inflammatory bowel disease (IBD), Crohn's disease or colitis.

Typically, said cancer is chronic lymphocytic leukaemia, breast cancer, prostate cancer, ovarian cancer, mesothelioma or T-cell lymphoma.

Typically, said cardiovascular disease is hypertension, myocardial infarction (MI), ischemic heart disease (IHD) (reperfusion), angina pectoris, arrhythmia, hypercholesterolemia, hyperlipidaemia, atherosclerosis, stroke, myocarditis, congestive heart failure, primary and secondary i.e. dilated (congestive) cardiomyopathy, hypertrophic cardiomyopathy, restrictive cardiomyopathy, peripheral vascular disease, tachycardia, high blood pressure or thrombosis.

Typically, said genentically related metabolic disorder is cystic fibrosis (CF), peroxisome biogenesis disorder or adrenoleukodystrophy.

Typically, the compounds of the invention are used as an immunosuppressant following organ transplant.

Typically, said infection is a viral, bacterial, fungal or parasitic infection, in particular an infection by S aureus, P acne, candida or aspergillus.

Typically, said CNS disorder is Huntingdon's disease, Alzheimer's disease, multiple sclerosis or amyotrophic lateral sclerosis.

In this embodiment, the compounds of the invention may be used to alleviate cancer, cardiac hypertrophy, chronic heart failure, an inflammatory condition, a cardiovascular disease, a haemoglobinopathy, a thalassemia, a sickle cell disease, a CNS disorder, an autoimmune disease, diabetes or osteoporosis, or are used as an immunosuppressant.

The compounds of the invention may also be used to alleviate chronic lymphocytic leukaemia (CLL), breast cancer, prostate cancer, ovarian cancer, mesothelioma, T-cell lymphoma, cardiac hypertrophy, chronic heart failure or a skin inflammatory condition, in particular psoriasis, acne or eczema.

The compounds of the present invention can be used in the treatment of animals, preferably in the treatment of mammals and more preferably in the treatment of humans.

The compounds of the invention may, where appropriate, be used prophylactically to reduce the incidence of such conditions.

In use, a therapeutically effective amount of a compound of the invention is administered to a patient. A typical dose is from about 0.001 to 50 mg per kg of body weight, according to the activity of the specific compound, the age, weight and conditions of the subject to be treated, the type and severity of the disease and the frequency and route of administration.

The invention will now be illustrated by the following Examples.

### EXAMPLES - Compounds of Formula I

### Synthesis of Intermediate X (a precursor to the compounds of Formula I)

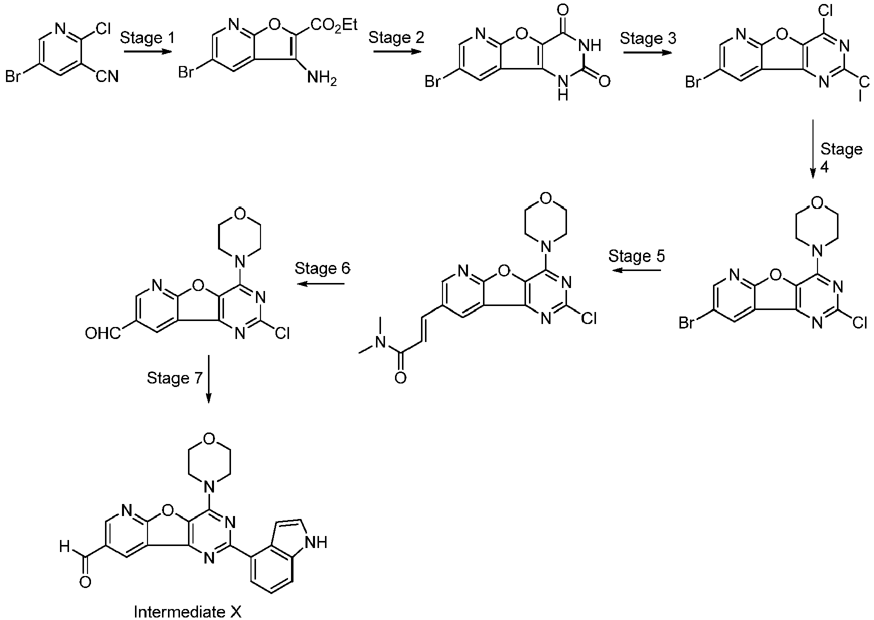

*Reagents and conditions:* 1) K₂CO₃, ethyl glycolate, DMF, 115°C; 2) (i) chlorosulfonyl isocyanate, CH₂Cl₂, 0-10°C then rt (ii) water, 75°C (iii) NaOH max temp 40°C; 3) POCl₃, N,N-dimethylaniline, 107°C; 4) morpholine, MeOH, rt; 5) N,N,-dimethylacrylamide, PdCl₂(PPh₃)₂, NaOAc, DMF, 110°C; 6) NalO₄, OsO₄, THF, water, rt; 7) indole-4-boronic acid pinacol ester, PdCl₂(PPh₃)₂, sodium carbonate, dioxane, water, 102°C.

### i. Ethyl-3-amino-5-bromofuro[2,3-b]pyridine-2-carboxylate

To a 10L flask under N₂(g) was added 5-bromo-2-chloropyridine-3-carbonitrile (435g, 2.0mol, 1eq), DMF (2790mL) and potassium carbonate (553g, 4.0mol, 2eq). This was followed by the addition of ethyl glycolate (208.2mL, 2.2mol, 1.1eq). The reaction mixture was heated to 115°C overnight. Upon completion, the reaction mixture was cooled to rt and water (13.1L) was added, this led to the formation of a precipitate. The mixture was stirred for 20mins, then filtered. The resulting brown solid was dried at 50°C, slurried in Et₂O:heptane (9:1, 2.8L) and filtered to give 405.6g. Further purification via soxhlet extraction using TBME (4.5L) yielded the product as a yellow solid (186g, 34%). This procedure was repeated twice.
¹H NMR (400MHz, CDCl₃) δ_{H}: 8.53 (d, *J*=2.0Hz, 1H), 8.07 (d, *J*=2.0Hz, 1H), 5.00 (br. s., 2H), 4.44 (q, *J*=7.0Hz, 2H), 1.44 (t, *J*=7.0Hz, 3H).
MS (ES⁺) 309 (100%, [M+Na]⁺), 307 (100%, [M+Na]⁺).

### ii. 12-Bromo-8-oxa-3,5,10-triazatricyclo[7.4.0.0^{2,7}]trideca-1(9),2(7),10,12-tetraene-4,6-dione

To ethyl-3-amino-5-bromofuro[2,3-b]pyridine-2-carboxylate (239.0g, 0.84mol, 1eq) dissolved in CH₂Cl₂ (5.5L) was added chlorosulfonyl isocyanate (87.6mL, 1.0mol, 1.2eq) dropwise at 0-10°C. The resulting reaction was stirred for 30min, stripped to dryness and the resulting solid ground to a fine powder. Water (5.5L) was added to the solid and the suspension was heated at 75°C for 1h. After cooling to rt, solid NaOH (335g, 8.4mol, 10eq) was added allowing the reaction to exotherm (maximum temperature 40°C). The reaction was cooled to 0-10°C and the pH adjusted to 5-6 using 5M HCI (∼1L). The reaction was stirred for 30mins, then filtered. The solid was washed with water (2.3L) and pulled dry. Further drying in a vacuum oven at 40°C yielded the product as a brown solid (193g, 76%). This procedure was repeated twice.
¹H NMR (400MHz, DMSO-*d*₆) δ_{H}: 12.01 (br. s., 1H), 11.58 (br. s, 1H), 8.72 (d, *J*=2.0Hz, 1H), 8.59 (d, *J*=2.0Hz, 1H).
MS (ES⁻) 282 (100%, [M+H]⁺).

### iii. 12-Bromo-4,6-dichloro-8-oxa-3,5,10-triazatricyclo[7.4.0.0^{2,7}]trideca-1(9),2(7),3,5,10,12-hexaene

To 12-bromo-8-oxa-3,5,10-triazatricyclo[7.4.0.0*^{2,7}*]trideca-1(9),2(7),10,12-tetraene-4,6-dione (387g, 1.27mol, 1eq) was added POCl₃ (6070mL) and *N,N-*dimethylaniline (348mL, 2.8mol, 2.2eq). The mixture was heated at 107°C for 10h. Once cooled to rt, solvent was removed *in vacuo* azeotroping with toluene (3 x 3.9L). The resulting residue was partitioned between CH₂Cl₂ (12.76L) and water (3.9L) and the phases separated. The organic phase was washed with water (2 x 3.9L). The combined aqueous was back-extracted with CH₂Cl₂ (7.7L) and the combined organics dried over MgSO₄, filtered and stripped to yield the product as brown solid (429g, -quant.).
¹H NMR (400MHz, CDCl₃) δ_{H}: 8.78 (d, *J*=2.5Hz, 1H), 8.72 (d, *J*=2.5Hz, 1H).

### iv. 12-bromo-4-chloro-6-(morpholin-4-yl)-8-oxa-3,5,10-triazatricyclo[7.4.0.0^{2,7}]trideca-1(9),2(7),3,5,10,12-hexaene

To 12-bromo-4,6-dichloro-8-oxa-3,5,10-triazatricyclo[7.4.0.0^{2,7}]trideca-1(9),2(7),3,5,10,12-hexaene (419.3g, 1.32mol, 1eq) in MeOH (8588mL) was added Morpholine (259mL, 2.90mol, 2.2eq) at rt. After stirring for 2h, water (0.8L) was added. It was then cooled to 0-5°C and stirred for an additional 30mins. The resulting solid was filtered, washed with water (5.2L) and pulled dry. Further purification by silica gel column chromatography with CH₂Cl₂/EtOAc (1:0-9:1) yielded the desired product (419g, 84%).
¹H NMR (400MHz, CDCl₃) δ_{H}: 8.66 (d, *J*=2.0Hz, 1H), 8.62 (d, *J*=2.0Hz, 1H), 4.07-4.21 (m, 4H), 3.85-3.91 (m, 4H).
MS (ES⁺) 393 (100%, [M+Na]⁺), 391 (80%, [M+Na]⁺).

### v. (2E)-3-[4-Chloro-6-(morpholin-4-yl)-8-oxa-3,5,10-triazatricyclo[7.4.0.0^{2,7}]trideca-1(9),2(7),3,5,10,12-hexaen-12-yl]-N,N-dimethylprop-2-enamide

To 12-bromo-4-chloro-6-(morpholin-4-yl)-8-oxa-3,5,10-triazatricyclo[7.4.0.0*^{2,7}*]trideca-1(9),2(7),3,5,10,12-hexaene (60g, 0.15mol, 1eq) was added *N,N*-dimethylacrylamide (16.7mL, 0.15mol, 1eq), PdCl₂(PPh₃)₂ (3.4g, 4.5mmol, 0.03eq) and NaOAc (40g, 0.45mol, 3eq) in DMF (1.2L). The reaction was heated at 110°C for 7h. This process was repeated 3 times and batches combined. Once cooled down to rt, solvent was removed *in vacuo* and the resulting residue was partitioned between CH₂Cl₂ (6.5L) and water (5.5L). The phases were separated and the aqueous phase was extracted with CH₂Cl₂ (2 x 4L). The combined organics were washed with brine (2 x 4L), dried over MgSO₄, filtered and stripped. The resulting solid was slurried in EtOAc/heptane (1:1, 0.8L) for 30mins, filtered, washed and washed with EtOAc/heptane (1:1, 2 x 450mL). Further drying in a vacuum oven at 40°C yielded the desired product as an orange solid (203.0g, 86%).
¹H NMR (400MHz, CDCl₃) δ_{H}: 8.70 (s, 2H), 7.82 (d, *J*=15.6Hz, 1H), 7.07 (d, *J*=15.6Hz, 1H), 4.11-4.19 (m, 4H), 3.85-3.93 (m, 4H), 3.22 (s, 3H), 3.11 (s, 3H). MS (ES⁺) 388 (100%, [M+H]⁺).

### vi. 4-Chloro-6-(morpholin-4-yl)-8-oxa-3,5,10-triazatricyclo[7.4.0.0^{2,7}]trideca-1(9),2(7),3,5,10,12-hexaene-12-carbaldehyde

(2E)-3-[4-chloro-6-(morpholin-4-yl)-8-oxa-3,5,10-triazatricyclo[7.4.0.0*^{2,7}*]trideca-1(9),2(7),3,5,10,12-hexaen-12-yl]-N,N-dimethylprop-2-enamide (124.0g, 0.39mol, 1eq) was dissolved in THF (12.4L) at 65°C. Once cooled to 35°C, water (4.1L), NalO₄ (205.4g, 1.17mol, 3eq) and OsO₄ (2.5wt% in ^{t}BuOH, 80.3mL, 2%) were added. The reaction was stirred at rt for 60h. The reaction was cooled to 0-5°C, stirred for 30mins then filtered. The solid was washed with water (545mL) and pulled dry. The crude product was combined with two further batches (2 x 118.3g scale) and slurried in water (6.3L) for 30mins at rt. The solids were filtered, washed with water (1.6L) and pulled dry. Further drying in a vacuum oven yielded the desired product as a pink solid (260g, 88%)
¹H NMR (400MHz, CDCl₃:MeOD, 9:1) δ_{H}: 10.13 (s, 1H), 9.04 (d, *J*=2.0Hz, 1H), 8.91 (d, *J*=2.0Hz, 1H), 3.99-4.13 (m, 4H), 3.73-3.84 (m, 4H).
MS (ES⁺) 351 (100%, [M+MeOH+H]⁺).

### vii. 4-(1H-Indol-4-yl)-6-(morpholin-4-yl)-8-oxa-3,5,10-triazatricyclo[7.4.0.0^{2,7}]trideca-1(9),2,4,6,10,12-hexaene-12-carbaldehyde

To 4-chloro-6-(morpholin-4-yl)-8-oxa-3,5,10-triazatricyclo[7.4.0.0*^{2,7}*]trideca-1(9),2(7),3,5,10,12-hexaene-12-carbaldehyde (164.4g, 0.52mol, 1eq) was added indole-4-boronic acid pinacol ester (376.0g, 1.55mol, 3eq), PdCl₂(PPh₃)₂ (72.0g, 0.10mol, 2eq) and sodium carbonate (110.2g, 1.04mol, 2eq) in dioxane (16.4L) / water (5.8L). Reaction mixture was refluxed for 1h. It was then cooled to 60-70°C. Water (9.8L), brine (4.9L) and EtOAc (9.5L) were added. The phases were separated and the aqueous phase extracted with EtOAc (3 x 9.5L) at 60-65°C. The combined organics were dried over MgSO₄, filtered and stripped. The resulting solid was slurried in CH₂Cl₂ (4.75L) for 30mins, filtered, washed with CH₂Cl₂ (3 x 238mL) and pulled dry. Further drying in a vacuum oven at 40°C yielded **Intermediate X** as a yellow solid (135.7g, 66%).
¹H NMR (300MHz, CDCl₃) δ_{H}: 11.27 (br. s, 1H), 10.26 (s, 1H), 9.16 (d, *J*=2.3Hz, 1H), 9.11 (d, *J*=2.3Hz, 1H), 8.18 (d, *J*=7.5Hz, 1H), 7.58-7.67 (m, 2H), 7.49 (t, *J*=2.8Hz, 1H), 7.23 (t, *J*=7.7Hz, 1H), 4.08-4.16 (m, 4H), 3.83-3.90 (m, 4H).
MS (ES⁺) 432.0 (100%, [M+MeOH+H]⁺).

### Synthesis of Examples of compounds of formula (i) as used the present invention

### Example A:

### 4-(1H-Indol-4-yl)-6-(morpholin-4-yl)-12-[(1S,4S)-2-oxa-5-azabicyclo[2.2.1]heptan-5-ylmethyl]-8-oxa-3,5,10-triazatricyclo[7.4.0.0^{2,7}]trideca-1(13),2(7),3,5,9,11-hexaene

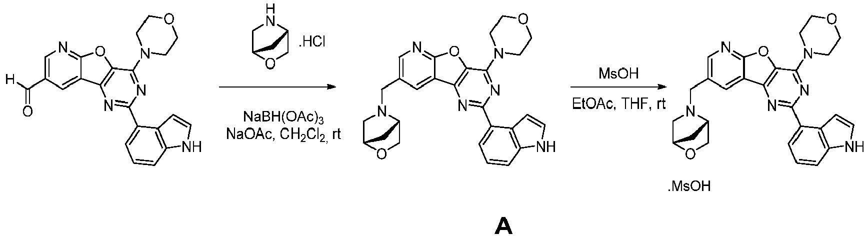

To a suspension of intermediate **X** (7.00g, 17.53mmol, 1eq), (1S,4S)-2-oxa-5-azabicyclo[2.2.1]heptane hydrochloride (7.13g, 52.58mmol, 3eq) and NaOAc (4.31g, 52.58mmol, 3eq) in anhydrous CH₂Cl₂ (150mL) was added NaBH(OAc)₃ (7.43g, 35.06mmol, 2eq). The reaction mixture was stirred at rt overnight. Then, it was partitioned with 1N NaOH (100mL) and extracted with CH₂Cl₂ (3 x 200mL). The combined organic extracts were washed with brine (50mL) then dried over MgSO₄ and the solvent was removed *in vacuo.* Purification by silica gel column chromatography with EtOAc/MeOH (1:0-7:1) yielded the product **A** as a white solid (6.02g, 71%).
¹H NMR (300MHz, CDCl₃) δ_{H}: 8.65 (d, *J*=2.1 Hz, 1H), 8.58 (d, *J*=2.1 Hz, 1H), 8.37 (br. s., 1H), 8.24 (dd, *J*=7.5, 0.9 Hz, 1H), 7.62 (td, *J*=2.6, 0.8 Hz, 1H), 7.53 (d, *J*=8.1 Hz, 1H), 7.37-7.41 (m, 1H), 7.31-7.37 (m, 1H), 4.47 (s, 1H), 4.22-4.30 (m, 4H), 4.18 (d, *J*=8.1 Hz, 1H), 3.98 (d, *J*=2.3 Hz, 2H), 3.91-3.97 (m, 4H), 3.70 (dd, *J*=7.9, 1.7 Hz, 1H), 3.53 (s, 1H), 2.94 (dd, *J*=10.0, 1.5 Hz, 1H), 2.64 (d, *J*=10.2 Hz, 1H), 1.97 (dd, *J*=9.8, 1.9 Hz, 1H), 1.80 (dt, *J*=9.8, 1.1 Hz, 1H).
MS (ES⁺) 483.1 (100%, [M+H]⁺).

### 4-(1H-lndol-4-yl)-6-(morpholin-4-yl)-12-[(1S,4S)-2-oxa-5-azabicyclo[2.2.1]heptan-5-ylmethyl]-8-oxa-3,5,10-triazatricyclo[7.4.0.0^{2,7}]trideca-1(13),2(7),3,5,9,11-hexaene; methanesulfonic acid

**A** (5.98g, 12.38mmol, 1eq) was dissolved in hot EtOAc (1L) and THF (200mL). Once cooled down to rt, a solution of MsOH (884µL, 13.6mmol, 1.1eq) in EtOAc (5mL) was added slowly. An instant yellow precipitate formed. The suspension was shaken vigorously for 10s then left to stand at rt overnight. As solid settled, excess supernatant was decanted off (200mL), then EtOAc was added (200mL). The suspension was shaken again and left to stand for 1h. This operation was repeated twice, then the solvent was removed *in vacuo.* The salt form of **A** was obtained as a yellow solid (6.50g, 91%).
¹H NMR (300MHz, DMSO-d₆) δ_{H}: 11.33 (br. s., 1H), 9.69-10.24 (m, 1H), 9.05 (d, *J*=2.1 Hz, 1H), 8.79-8.93 (m, 1H), 8.19 (d, *J*=7.5 Hz, 1H), 7.54-7.62 (m, 2H), 7.50 (t, *J*=2.7 Hz, 1H), 7.24 (t, *J*=7.7 Hz, 1H), 4.64-4.89 (m, 2H), 4.47-4.61 (m, 2H), 4.14 (m, 4H), 3.94-4.00 (m, 2H), 3.83-3.91 (m, 4H), 3.72-3.83 (m, 1H), 3.29-3.46 (m, 2H), 2.33 (s, 4H), 2.02-2.15 (m, 1H).
MS (ES⁺) 483.1 (100%, [M-MsOH+H]⁺).

### Example B:

### 4-(1H-Indol-4-yl)-6-(morpholin-4-yl)-12-{2-oxa-7-azaspiro[3.5]nonan-7-ylmethyl}-8-oxa-3,5,10-triazatricyclo[7.4.0.0^{2,7}]trideca-1(13),2(7),3,5,9,11-hexaene

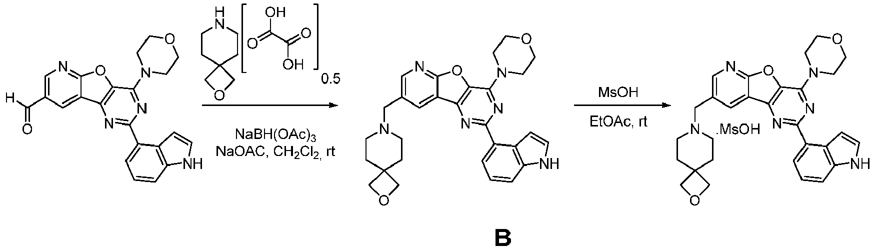

To a suspension of intermediate **X** (3.108g, 7.78mmol 1eq), 2-oxa-7-azaspiro[3.5]nonane hemioxalate (4.02g, 23.3mmol, 3eq) and NaOAc (1.91g, 23.3mmol, 3eq) in anhydrous CH₂Cl₂ (280mL) was added NaBH(OAc)₃ (3.30g, 15.6mmol, 2eq). The reaction mixture was stirred at rt overnight. Then, it was partitioned with 1N NaOH (150mL) and extracted with CH₂Cl₂ (2 x 100mL). The combined organic extracts were washed with 50% brine (100mL) then dried over MgSO₄ and the solvent was removed *in vacuo.* Purification by silica gel column chromatography with EtOAc/MeOH (1:0-8:1) yielded the product **B** as an off-white solid (3.154g, 79%).
¹H NMR (300MHz, CDCl₃) δ_{H}: 8.59 (d, *J*=2.1 Hz, 1H), 8.53 (d, *J*=1.9 Hz, 1H), 8.41 (br. s., 1H), 8.24 (dd, *J*=7.4, 0.8 Hz, 1H), 7.61 (t, *J*=2.3 Hz, 1H), 7.53 (d, *J*=8.1 Hz, 1H), 7.37-7.41 (m, 1H), 7.34 (t, *J*=7.9 Hz, 1H), 4.43 (s, 4H), 4.22-4.30 (m, 4H), 3.86-4.00 (m, 4H), 3.68 (s, 2H), 2.23-2.59 (m, 4H), 1.83-2.00 (m, 4H).
MS (ES⁺) 511.1 (100%, [M+H]⁺).

### 4-(1H-Indol-4-yl)-6-(morpholin-4-yl)-12-{2-oxa-7-azaspiro[3.5]nonan-7-ylmethyl}-8-oxa-3,5,10-triazatricyclo[7.4.0.0^{2,7}]trideca-1(13),2(7),3,5,9,11-hexaene;

### methanesulfonic acid

To a solution of **B** (2.987g, 5.854mmol, 1eq) in EtOAc (1.2L, heat to 70°C for 5 min to dissolve) at rt was added a solution of MsOH (590 µL, 6.14mmol, 1.05eq) in EtOAc (16mL). A yellow precipitate formed instantly. The suspension was shaken vigorously for 20s then left to stand at rt overnight. The excess supernatant was decanted off (600mL), then EtOAc was added (500mL). The suspension was shaken again and left to stand for 1h before another 500mL of excess supernatant was decanted off. The solvent was removed *in vacuo* to give the salt form of **F** as a yellow solid (3.230g, 91%).
¹H NMR (300MHz, DMSO-d₆) δ_{H}: 11.33 (br. s., 1H), 9.45 (br. s., 1H), 8.90 (d, *J*=1.9 Hz, 1H), 8.72 (d, *J*=1.9 Hz, 1H), 8.19 (d, *J*=7.3 Hz, 1H), 7.41-7.69 (m, 3H), 7.23 (t, *J*=7.8 Hz, 1H), 4.58 (d, *J*=3.8 Hz, 2H), 4.39 (s, 2H), 4.29 (s, 2H), 4.03-4.22 (m, 4H), 3.81-3.97 (m, 4H), 3.40 (d, *J*=12.1 Hz, 2H), 2.88-3.13 (m, 2H), 2.33 (s, 3H), 2.26 (d, *J*=13.9 Hz, 2H), 1.69-1.91 (m, 2H).
MS (ES⁺) 511.1 (100%, [M-MsOH+H]⁺).

### Example C:

### 4-(1H-Indol-4-yl)-6-(morpholin-4-yl)-12-{8-oxa-3-azabicyclo[3.2.1]octan-3-ylmethyl}-8-oxa-3,5,10-triazatricyclo[7.4.0.0^{2,7}]trideca-1(13),2(7),3,5,9,11-hexaene

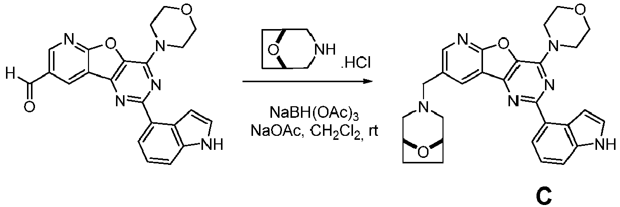

To a suspension of intermediate **X** (100mg, 0.25mmol, 1eq), 8-oxa-3-azabicyclo[3.2.1]octane hydrochloride (112mg, 0.75mmol, 3eq) and NaOAc (62mg, 0.75mmol, 3eq) in anhydrous CH₂Cl₂ (10mL) was added NaBH(OAc)₃ (106mg, 0.50mmol, 2eq). The reaction mixture was stirred at rt overnight. Then, it was partitioned with 1N NaOH (10mL), extracted with CH₂Cl₂ (3 x 10mL). The combined organic extracts were washed with brine (10mL) then dried over MgSO₄ and the solvent was removed *in vacuo.* Purification by silica gel column chromatography with EtOAc/MeOH (1:0-49:1) yielded the product **C** as an off white solid (116mg, 93%).
¹H NMR (300MHz, CDCl₃) δ_{H}: 8.56 (d, *J*=3.6 Hz, 2H), 8.35 (br. s., 1H), 8.24 (d, *J*=7.5 Hz, 1H), 7.58-7.66 (m, 1H), 7.51-7.57 (m, 1H), 7.31-7.44 (m, 2H), 4.30-4.38 (m, 2H), 4.23-4.30 (m, 4H), 3.89-4.01 (m, 4H), 3.68 (s, 2H), 2.61 (d, *J*=10.7 Hz, 2H), 2.40-2.52 (m, 2H), 1.96-2.09 (m, 2H), 1.83-1.95 (m, 2H).
MS (ES⁺) 497.1 (100%, [M+H]⁺).

### Example D:

### 4-(1H-Indol-4-yl)-12-({2-methyl-2,8-diazaspiro[4.5]decan-8-yl}methyl)-6-(morpholin-4-yl)-8-oxa-3,5,10-triazatricyclo[7.4.0.0^{2,7}]trideca-1(13),2(7),3,5,9,11-hexaene

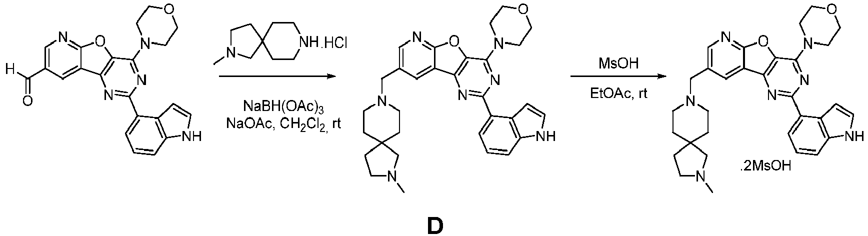

To a suspension of intermediate **X** (1.02g, 2.55mmol, 1eq), 2-methyl-2,8-diazaspiro[4.5]decane hydrochloride (1.46g, 7.66mmol, 3eq) and NaOAc (628mg, 7.66mmol, 3eq) in anhydrous CH₂Cl₂ (100mL) was added NaBH(OAc)₃ (1.08g, 5.1mmol, 2eq). The reaction mixture was stirred at rt overnight. Then, it was partitioned with 1N NaOH (30mL) and extracted with CH₂Cl₂ (3 x 50mL). The combined organic extracts were washed with brine (10mL) then dried over MgSO₄ and the solvent was removed *in vacuo.* Purification by silica gel column chromatography with CH₂Cl₂/MeOH (0:1-4:1) yielded the product **D** as a white solid (890mg, 65%).
¹H NMR (300MHz, CDCl₃) δ_{H}: 8.60 (d, *J*=2.1 Hz, 1H), 8.54 (d, *J*=2.1 Hz, 1H), 8.39 (br. s., 1H), 8.24 (dd, *J*=7.4, 0.8 Hz, 1H), 7.62 (t, *J*=2.3 Hz, 1H), 7.53 (d, *J*=8.1 Hz, 1H), 7.38 (t, *J*=2.8 Hz, 1H), 7.30-7.37 (m, 1H), 4.21-4.31 (m, 4H), 3.89-3.99 (m, 4H), 3.69 (s, 2H), 2.59 (t, *J*=6.8 Hz, 2H), 2.38-2.50 (m, 5H), 2.35 (s, 3H), 1.54-1.73 (m, 7H).
MS (ES⁺) 538.2 (100%, [M+H]⁺).

### 4-(1H-Indol-4-yl)-12-({2-methyl-2,8-diazaspiro[4.5]decan-8-yl}methyl)-6-(morpholin-4-yl)-8-oxa-3,5,10-triazatricyclo[7.4.0.0^{2,7}]trideca-1(13),2(7),3,5,9,11-hexaene; bis(methanesulfonic acid)

Compound **D** (821mg, 1.52mmol, 1eq) was dissolved in hot EtOAc (400mL). Once cooled down to rt, a solution of MsOH (218µL, 3.36mmol, 2.2eq) in EtOAc (5mL) was added slowly. An instant yellow precipitate formed. The suspension was shaken vigorously for 10s then left to stand at rt overnight. As solid settled, excess supernatant was decanted off (200mL), then EtOAc was added (200mL). The suspension was shaken again and left to stand for 1h. This operation was repeated twice, then the solvent was removed *in vacuo.* The salt form of **D** was obtained as a yellow solid (1.037g, 93%).
¹H NMR (300MHz, DMSO-d₆) δ_{H}: 11.32 (br. s., 1H), 9.46-10.03 (m, 2H), 8.93 (d, *J*=2.1 Hz, 1H), 8.76 (d, *J*=1.7 Hz, 1H), 8.19 (dd, *J*=7.4, 0.7 Hz, 1H), 7.53-7.60 (m, 2H), 7.50 (t, *J*=2.6 Hz, 1H), 7.24 (t, *J*=7.8 Hz, 1H), 4.63 (br. s., 2H), 4.10-4.20 (m, 4H), 3.82-3.91 (m, 5H), 3.54-3.77 (m, 2H), 3.36-3.51 (m, 2H), 3.05-3.25 (m, 3H), 2.89-3.03 (m, 1H), 2.80-2.89 (m, 3H), 2.36 (s, 6H), 2.02-2.17 (m, 1H), 1.65-1.95 (m, 4H).
MS (ES⁺) 538.2 (100%, [M-2MsOH+H]⁺).

### Example E:

### 4-(1H-Indol-4-yl)-12-({7-methyl-2,7-diazaspiro[4.4]nonan-2-yl}methyl)-6-(morpholin-4-yl)-8-oxa-3,5,10-triazatricyclo[7.4.0.0^{2,7}]trideca-1(13),2(7),3,5,9,11-hexaene

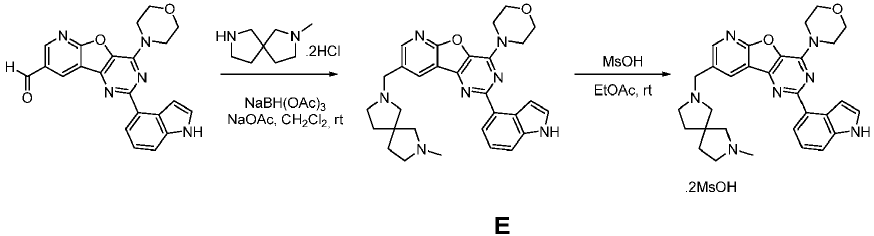

To a suspension of intermediate **X** (250mg, 0.63mmol, 1eq), 2-methyl-2,7-diazaspiro[4,4]nonane dihydrochloride (400mg, 1.87mmol, 3eq) and NaOAc (305mg, 3.70mmol, 6eq) in anhydrous CH₂Cl₂ (20mL) was added NaBH(OAc)₃ (265mg, 1.25mmol, 2eq). The reaction mixture was stirred at rt overnight. Then, it was partitioned with 1N NaOH (10mL), extracted with CH₂Cl₂ (3 x 10mL) and EtOAc (10mL). The combined organic extracts were washed with brine (10mL) then dried over MgSO₄ and the solvent was removed *in vacuo.* Purification by silica gel column chromatography with CH₂Cl₂/MeOH (0:1-4:1) yielded the product **E** as a white solid (169mg, 52%).
¹H NMR (300MHz, CDCl₃) δ_{H}: 8.58 (d, *J*=2.1 Hz, 1H), 8.53 (d, *J*=2.1 Hz, 1H), 8.48 (br. s., 1H), 8.23 (dd, *J*=7.4, 0.8 Hz, 1H), 7.63 (t, *J*=2.2 Hz, 1H), 7.53 (d, *J*=7.9 Hz, 1H), 7.39 (t, *J*=2.7 Hz, 1H), 7.29-7.36 (m, 1H), 4.21-4.30 (m, 4H), 3.89-3.99 (m, 4H), 3.72-3.85 (m, 2H), 2.49-2.83 (m, 8H), 2.45 (s, 3H), 1.81-2.06 (m, 4H).
MS (ES⁺) 524.1 (100%, [M+H]⁺).

### 4-(1H-Indol-4-yl)-12-({7-methyl-2,7-diazaspiro[4.4]nonan-2-yl}methyl)-6-(morpholin-4-yl)-8-oxa-3,5,10-triazatricyclo[7.4.0.0^{2,7}]trideca- 1(13),2(7),3,5,9,11-hexaene; bis(methanesulfonic acid)

Compound **E** (129mg, 0.25mmol, 1eq) was dissolved in hot EtOAc (50mL). Once cooled down to rt, a solution of MsOH (35µL, 0.54mmol, 2.2eq) in EtOAc (2mL) was added slowly. An instant yellow precipitate formed. The suspension was shaken vigorously for 10s then left to stand at rt overnight. As solid settled, excess supernatant was decanted off (20mL), then EtOAc was added (20mL). The suspension was shaken again and left to stand for 1h. This operation was repeated twice, then the solvent was removed *in vacuo.* The salt form of **E** was obtained as a yellow solid (173mg, 98%).
¹H NMR (300MHz, DMSO-d₆) δ_{H}: 11.33 (br. s., 1H), 10.39 (br. s., 1H), 9.72-10.12 (m, 1H), 8.73-9.09 (m, 2H), 8.19 (d, *J*=7.5 Hz, 1H), 7.41-7.63 (m, 3H), 7.24 (t, *J*=7.8 Hz, 1H), 4.53-4.87 (m, 2H), 4.10-4.22 (m, 4H), 3.79-3.93 (m, 4H), 3.32-3.77 (m, 6H), 2.99-3.29 (m, 2H), 2.78-2.89 (m, 3H), 2.36 (s, 6H), 1.87-2.22 (m, 3H).
MS (ES⁺) 524.5 (100%, [M-2MsOH+H]⁺).

### Example F:

### 4-(1H-Indol-4-yl)-6-(morpholin-4-yl)-12-[(1R,4R)-2-oxa-5-azabicyclo[2.2.1]heptan-5-ylmethyl]-8-oxa-3,5,10-triazatricyclo[7.4.0.0^{2,7}]trideca-1(13),2(7),3,5,9,11-hexaene

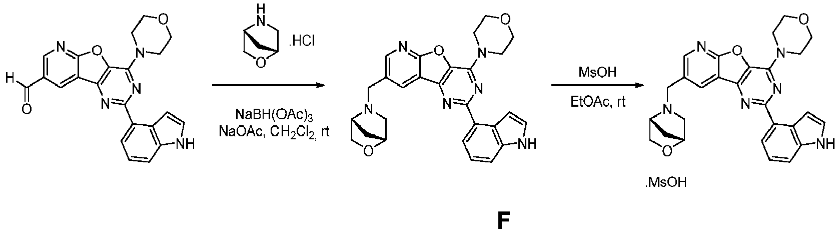

To a suspension of intermediate **X** (200mg, 0.50mmol, 1eq), (1R,4R)-2-oxa-5-azabicyclo[2.2.1]heptane hydrochloride (204mg, 1.50mmol, 3eq) and NaOAc (123mg, 1.5mmol, 3eq) in anhydrous CH₂Cl₂ (10mL) was added NaBH(OAc)₃ (160mg, 0.76mmol, 2eq). The reaction mixture was stirred at rt overnight. Then, it was partitioned with 1N NaOH (20mL) and extracted with CH₂Cl₂ (3 x 20mL). The combined organic extracts were passed through a phase separator and the solvent was removed *in vacuo.* Purification by silica gel column chromatography with EtOAc/MeOH (1:0-9:1) yielded the product **F** as a white solid (141.1mg, 59%).
¹H NMR (400MHz, CDCl₃) δ_{H}: 8.64 (d, *J*=2.1 Hz, 1H), 8.57 (d, *J*=2.1 Hz, 1H), 8.35 (br. s., 1H), 8.23 (dd, *J*=7.5, 0.9 Hz, 1H), 7.62 (m, 1H), 7.53 (d, *J*=8.1 Hz, 1H), 7.36-7.39 (m, 1H), 7.31-7.36 (m, 1H), 4.46 (s, 1H), 4.25 (m, 4H), 4.18 (d, *J*=8.1 Hz, 1H), 3.97 (d, *J*=2.3 Hz, 2H), 3.93-3.97 (m, 4H), 3.68 (dd, *J*=7.9, 1.7 Hz, 1H), 3.53 (s, 1H), 2.93 (dd, *J*=10.0, 1.5 Hz, 1H), 2.62 (d, *J*=10.2 Hz, 1H), 1.95 (dd, *J*=9.8, 1.9 Hz, 1H), 1.79 (dt, *J*=9.8, 1.1 Hz, 1H).
MS (ES⁺) 483.1 (100%, [M+H]⁺).

### 4-(1H-Indol-4-yl)-6-(morpholin-4-yl)-12-[(1R,4R)-2-oxa-5-azabicyclo[2.2.1]heptan-5-ylmethyl]-8-oxa-3,5,10-triazatricyclo[7.4.0.0^{2,7}]trideca-1(13),2(7),3,5,9,11-hexaene; methanesulfonic acid

Compound F (141mg, 0.29mmol, 1eq) was dissolved in hot EtOAc (100mL) then treated with 0.87 ml of a 0.308M MsOH solution in EtOAc under vigorously swirling. The mixture was set aside overnight. The excess supernatant was decanted (using a small Pasteur pipette) and more EtOAc (50 ml) was added. The suspension was once again shaken vigorously then left to stand at rt overnight. The excess supernatant was once more decanted and the solvent was removed *in vacuo.* The resulting solid was dried in a vacuum oven at 40°C. The salt form of **F** was obtained as a yellow solid (160mg, 95%).
¹H NMR (400MHz, DMSO-d₆) δ_{H}: 11.33 (br. s., 1H), 9.65-10.16 (m, 1H), 9.05 (d, J=2.0 Hz, 1H), 8.83-8.90 (m, 1H), 8.20 (d, *J*=7.3 Hz, 1H), 7.58-7.61 (m, 1H), 7.56 (d, *J*=7.8 Hz, 1H), 7.51 (t, *J*=2.8 Hz, 1H), 7.23 (t, *J*=7.7 Hz, 1H), 4.82 (dd, J=13.1, 4.5 Hz, 1H), 4.65-4.76 (m, 1H), 4.50-4.59 (m, 2H), 4.11-4.19 (m, 4H), 3.99 (d, *J*=9.6 Hz, 1H), 3.88 (t, *J*=4.5 Hz, 4H), 3.78 (dd, *J*=9.5, 1.4 Hz, 1H), 3.31-3.38 (m, 2H), 2.52-2.57 (m, 1H), 2.30 (s, 3H), 2.02-2.18 (m, 1H).
MS (ES⁺) 483.2 (100%, [M-MsOH+H]⁺).

### Example G

### 4-(1H-indol-4-yl)-6-(morpholin-4-yl)-12-{6-oxa-1-azaspiro[3.3]heptan-1-ylmethyl}-8-oxa-3,5,10-triazatricyclo[7.4.0.0^{2,7}]1(13),2(7),3,5,9,11-hexaene

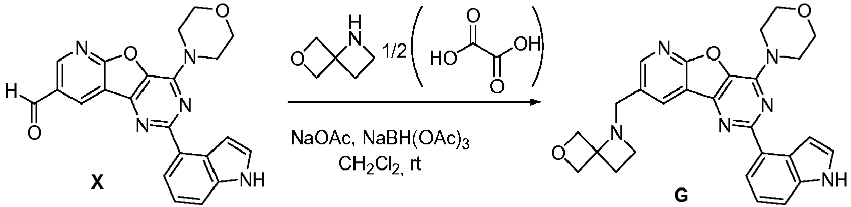

Intermediate **X** (125mg, 0.31mmol), 6-oxa-1-azaspiro[3.3]heptane hemioxalate (134mg, 0.93mmol, 3eq) and NaOAc (76mg, 0.93mmol, 3eq) were suspended in CH₂Cl₂ (16 mL) at rt. The mixture was stirred for 15mins then NaBH(OAc)₃ (131mg, 0.62mmol, 2eq) was added. The resulting suspension was stirred at rt overnight. The reaction mixture was then partitioned with 0.5 N NaOH (8 mL) and extracted with CH₂Cl₂ (2 x 10mL). The combined organics were washed with 50% brine (5mL) then dried over MgSO₄ and the solvent was removed *in vacuo.* The residue was dissolved in DMSO (2 mL) and purified by basic preparative LCMS to yield **G** as a white solid (48mg, 32%).
¹H NMR (DMSO-d₆) δ_{H}: 11.30 (br s, 1H), 8.62 (s, 2H), 8.18 (d, J=7.6 Hz, 1H), 7.51-7.58 (m, 2H), 7.46-7.51 (m, 1H), 7.22 (t, J=7.7 Hz, 1H), 4.89 (d, J=7.6 Hz, 2H), 4.55 (d, J=7.3 Hz, 2H), 4.08-4.17 (m, 4H), 4.03 (s, 2H), 3.81-3.91 (m, 4H), 3.03 (t, J=6.7 Hz, 2H), 2.32 (t, J=6.7 Hz, 2H).
MS (ES⁺) 483.3 (100%, [M+H]⁺).

### Examples - compounds of Formulae II

### General methods

### i. General Procedure for Synthesis of Secondary Amines

**Method A (Using BINAP):** 4,6-Dimethylpyridin-2-amine (200mg, 1.63mmol), 2-bromo-5-fluoropyridine (317mg, 1.8mmol), potassium *tert*-butoxide (236mg, 2.45mmol) and (±)-BINAP (40mg, 0.06mmol) were stirred in toluene (4mL) and degassed using Ar(g) for 30 min. Pd₂(dba)₃ (45mg, 0.049mmol) was then added and the reaction mixture stirred for 12h at 90°C under Ar(g). The reaction was monitored by TLC. Following complete consumption of starting material, the reaction mixture was diluted with CH₂Cl₂ (20mL) and silica was added. The solvent was removed *in vacuo* and the resulting dry loaded material was purified by silica gel column chromatography with hexane/EtOAc (4:1-1:1), to provide N-(5-fluoropyridin-2-yl)-4,6-dimethylpyridin-2-amine.

**Method B (Using SPhos):** 2-Bromopyridine (200mg, 1.26mmol), 5-methylpyridin-2-amine (150mg, 1.38mmol), potassium *tert*-butoxide (182mg, 1.89mmol) and 2-dicyclohexylphosphino-2',6'-dimethoxybiphenyl (SPhos) (20mg, 0.05mmol) were stirred in toluene (4mL) and the reaction mixture was degassed using Ar(g) for 30 min. Pd₂(dba)₃ (34mg, 0.037mmol) was then added, and the reaction mixture was stirred for 12h at 90°C under Ar(g). The reaction was monitored by TLC. Following complete consumption of the starting material, the reaction mixture was diluted with CH₂Cl₂ (20mL) and silica was added. The solvent was removed *in vacuo,* and the resulting dry loaded material was purified by silica gel column chromatography with hexane/EtOAc,(4:1-1:1), to provide N-(pyridin-2-yl)-5-methylpyridin-2-amine.

### a) 3-Methoxy-N-(5-methylpyridin-2-yl)pyridin-2-amine

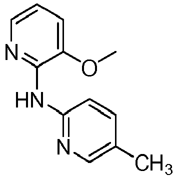

Synthesised according to the general procedure Method B (Using SPhos). ¹H NMR (400 MHz, Chloroform-*d*), δ_{H} ppm: 8.44 (d, *J*=8.6 Hz, 1H), 8.02-8.13 (m, 1H), 7.73-7.93 (m, 2H), 7.48 (dd, *J*=8.6, 2.3 Hz, 1H), 6.99 (dd, *J*=7.8, 1.5 Hz, 1H), 6.83-6.71 (m, 1H), 3.89 (s, 3H), 2.27 (s, 3H).

### b) 5-Methoxy-N-(5-methylpyridin-2-yl)pyridin-2-amine

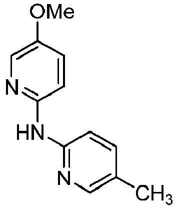

Synthesised according to the general procedure Method B (Using SPhos).
¹H NMR (400 MHz, Chloroform-*d*), δ_{H} ppm: 8.04 (d, *J*=2.5 Hz, 1H), 7.95 (d, *J*=3.0 Hz, 1H), 7.50 (d, *J*=9.0 Hz, 1H), 7.40 (dd, *J*=8.4, 2.6 Hz, 1H), 7.31 (d, *J*=8.4 Hz, 1H), 7.22 (dd, *J*=9.0, 3.1 Hz, 1H), 3.87 (m, 3H), 2.25 (s, 3H).

### c) 3-Methoxy-N-(5-morpholinopyridin-2-yl)pyridin-2-amine

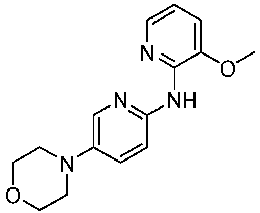

Synthesised according to the general procedure Method B (Using SPhos).
¹H NMR (400 MHz, Chloroform-*d*), δ_{H} ppm: 8.45 (d, *J*=9.1 Hz, 1H), 7.94 (d, *J*=3.0 Hz, 1H), 7.83 (dd, *J*=5.1, 1.5 Hz, 1H), 7.31 (dd, *J*=9.1, 3.1 Hz, 1H), 6.98 (dd, *J*=7.9, 1.5 Hz, 1H), 6.73 (dd, *J*=7.8, 5.1 Hz, 1H), 3.76-3.98 (m, 7H), 3.06-3.16 (m, 4H).

### d) 5-Methoxy-N-(5-morpholinopyridin-2-yl)pyridin-2-amine

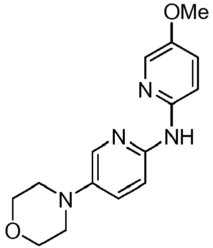

Synthesised according to the general procedure Method B (Using SPhos).
¹H NMR (400 MHz, Chloroform-*d*), δ_{H} ppm: 7.90 (dd, *J*=15.8, 3.0 Hz, 2H), 7.43 (d, *J*=9.0 Hz, 2H), 7.19-7.30 (m, 2H), 3.87 (t, *J*=4.8 Hz, 4H), 3.82 (s, 3H), 3.00-3.16 (m, 4H).

### e) N-(Pyridin-2-yl)thieno[3,2-c]pyridin-4-amine

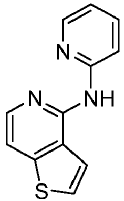

Synthesised according to the general procedure Method B (Using SPhos).
¹H NMR (400 MHz, Chloroform-*d*), δ_{H} ppm: 8.58 (d, *J*=8.4 Hz, 1H), 8.26 (dd, *J*=5.1, 2.0 Hz, 1H), 8.12 (d, *J*=5.7 Hz, 1H), 7.72 (ddd, *J*=8.8, 7.1, 1.9 Hz, 1H), 7.51 (d, *J*=5.9 Hz, 1H), 7.46 (d, *J*=5.4 Hz, 1H), 7.38 (d, *J*=5.7 Hz, 1H), 6.93 (ddd, *J*=7.1, 4.8, 1.0 Hz, 1H).

### f) 6-Methyl-N-(5-morpholinopyridin-2-yl)pyridin-2-amine

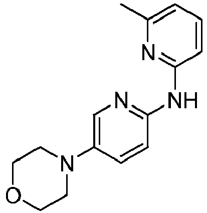

Synthesised according to the general procedure Method B (Using SPhos).
¹H NMR (400 MHz, Chloroform-*d*), δ_{H} ppm: 7.94 (d, *J*=3.0 Hz, 1H), 7.40-7.59 (m, 2H), 7.24 (d, *J*=8.1 Hz, 2H), 6.66 (d, *J*=7.3 Hz, 1H), 3.80-3.96 (m, 4H), 3.01-3.17 (m, 4H), 2.45 (s, 3H).

### g) N-(6-(Trifluoromethyl)pyridin-2-yl)thieno[3,2-c]pyridin-4-amine

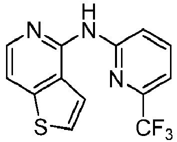

Synthesised according to the general procedure Method A (Using BINAP).
¹H NMR (400 MHz, Chloroform-*d*), δ_{H} ppm: 8.82 (d, *J*=8.5 Hz, 1H), 8.14 (d, *J*=5.7 Hz, 1H), 7.83 (dd, *J*=18.3, 10.3 Hz, 2H), 7.51 (s, 1H), 7.44 (d, *J*=5.7 Hz, 1H), 7.29 (d, *J*=7.4 Hz, 1H).

### h) N5-(2-Methoxyethyl)-N5-methyl-N2-(4-(trifluoromethyl)pyridin-2-yl)pyridine-2,5-diamine

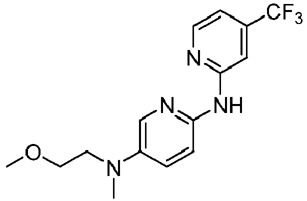

Synthesised according to the general procedure Method A (Using BINAP).
¹H NMR (400 MHz, Chloroform-*d*), δ_{H} ppm: 8.32 (d, *J*=5.2 Hz, 1H), 7.87 (d, *J*=3.1 Hz, 1H), 7.70-7.78 (m, 1H), 7.29-7.37 (m, 1H), 7.15 (dd, *J*=9.0, 3.1 Hz, 1H), 6.88-6.98 (m, 1H), 3.54-3.59 (m, 2H), 3.48 (t, *J*=5.5 Hz, 2H), 3.37 (s, 3H), 2.98 (s, 3H).

### i) N5-(2-Methoxyethyl)-N2-(3-methoxypyridin-2-yl)-N5-methylpyridine-2,5-diamine

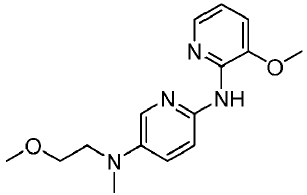

Synthesised according to the general procedure Method B (Using SPhos).
¹H NMR (400 MHz, Chloroform-*d*), δ_{H} ppm: 8.37 (d, *J*=9.1 Hz, 1H), 7.81 (q, *J*=1.7 Hz, 2H), 7.19 (dd, *J*=9.1, 3.1 Hz, 1H), 6.96 (dd, *J*=7.7, 1.5 Hz, 1H), 6.70 (dd, *J*=7.8, 5.1 Hz, 1H), 3.88 (s, 3H), 3.56 (t, *J*=5.8 Hz, 2H), 3.45 (t, *J*=5.8 Hz, 2H), 3.36 (s, 3H), 2.96 (s, 3H).

### j) N5-(2-methoxyethyl)-N2-(5-methoxypyridin-2-yl)-N5-methylpyridine-2,5-diamine

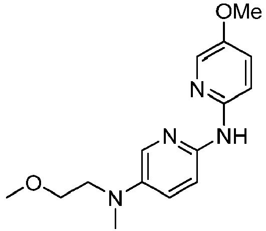

Synthesised according to the general procedure Method B (Using SPhos).
¹H NMR (400 MHz, Chloroform-*d*), δ_{H} ppm: 7.89 (d, *J*=3.0 Hz, 1H), 7.74 (d, *J*=3.1 Hz, 1H), 7.45 (d, *J*=9.1 Hz, 1H), 7.37 (d, *J*=9.0 Hz, 1H), 7.19 (ddd, *J*=12.0, 9.0, 3.1 Hz, 2H), 3.82 (s, 3H), 3.55 (t, *J*=5.8 Hz, 2H), 3.43 (t, *J*=5.8 Hz, 2H), 3.36 (s, 3H), 2.94 (s, 3H).

### iii. General Procedure for Alkylation and Hydroxamic Acid Formation

NaH (12mg, 0.5mmol, 2eq) was added portion-wise to secondary amine (50mg, 0.25mmol, 1eq) in DMF (2mL) at 0°C under Ar(g). Following addition, the reaction mixture was stirred for 20min, then methyl-4-(bromomethyl)benzoate (57mg, 0.25mmol, 1eq) was added. The reaction mixture was stirred at rt under Ar(g) for 2h, and the reaction was monitored by TLC. Following complete consumption of the starting material, the reaction mixture was poured onto brine (25mL), extracted with EtOAc (3 x 25mL). The organic phases were combined, dried over Na₂SO₄, filtered and subsequently concentrated *in vacuo.* The resulting crude product was purified by silica gel column chromatography with hexane/EtOAc (19:1-3:1), to provide the desired methyl ester as a gummy, yellowish solid.

To a stirred solution of the methyl ester (70mg, 0.20mmol) in MeOH/CH₂Cl₂ (3:1, 4mL) under an inert atmosphere was added 50% aq. hydroxylamine sol (2.5mL) at 0°C, and the resulting reaction mixture was stirred for 20min. Sodium hydroxide solution (54mg in 1mL water, 1.35mmol) was then added to the reaction mixture; this was following by stirring for 30min, and the mixture was then warmed to rt and stirred for 2h. The reaction was monitored by TLC. Following complete consumption of the starting material, the volatiles were concentrated *in vacuo.* The residue was acidified with acetic acid to pH∼6. The compound was extracted with CH₂Cl₂/MeOH (9:1) (3 x 20mL); the combined organic extracts were concentrated *in vacuo* to obtain the crude product, which was purified by silica gel column chromatography (1-10% MeOH/CH₂Cl₂) to afford the desired product as gummy, yellowish solid.

### Specific Examples

### Example A

### 4-{[Bis(pyridin-2-yl)amino]methyl}-N-hydroxybenzamide

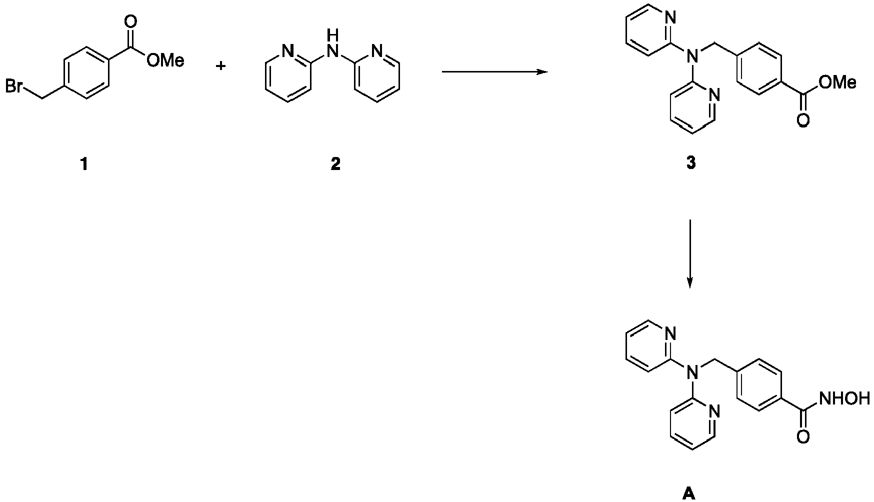

NaH (83mg, 2.18mmol) was added to 2,2'-dipyridylamine, **2** (373mg, 2.18mmol) in DMF (5mL) at rt. After 15 min, methyl-4-(bromomethyl)benzoate (**1**) (500mg, 2.18mmol) was added, and the reaction mixture was subsequently stirred at 90°C for 1h under Ar(g). Once cooled to rt, the reaction mixture was poured onto brine (50mL) and extracted twice with EtOAc (2 x 25mL). The organic phases were combined, dried over MgSO₄, filtered, and subsequently concentrated *in vacuo.* The resulting residue was purified by silica gel column chromatography with hexanes/EtOAc (4:1) to furnish **3** as a white solid (429mg, 62%).
LCMS (ES): found 319.9 [M+H]⁺.

A freshly prepared solution of NH₂OH in MeOH (0.4M, 20mL) was added to 4-{[bis(pyridin-2-yl)amino]methyl}benzoate (**3**) (100mg, 0.3mmol) at 0°C followed by KOH solubilized in MeOH (0.8M, 4mL). The reaction mixture was then stirred at rt for 18h, was subsequently concentrated *in vacuo* (*ca* 5mL) and poured onto water (50mL). The basic aqueous phase was extracted initially with EtOAc (25mL) and the phases were separated. The aqueous was then neutralized with 2N HCI and extracted again with EtOAc (25mL). The resulting organic phase was dried over MgSO₄, filtered and subsequently concentrated *in vacuo* to provide **Example A** as a white solid (51mg, 51%).
¹H NMR (400 MHz, Methanol-*d*₄), □_{H} ppm: 6.69-6.76 (m, 2H), 6.07-6.15 (m, 4H), 5.91 (d, *J*=8.6 Hz, 2H), 5.65 (d, *J*=8.1 Hz, 2H), 5.44 (dd, *J*=6.6, 5.1 Hz, 2H), 3.97 (s, 2H).
LCMS (ES): found 321.1 [M+H]⁺.

### Example B

### 4-{[Bis(3-methyl-1,2,4-thiadiazol-5-yl)amino]methyl}-2-fluoro-N-hydroxybenzamide

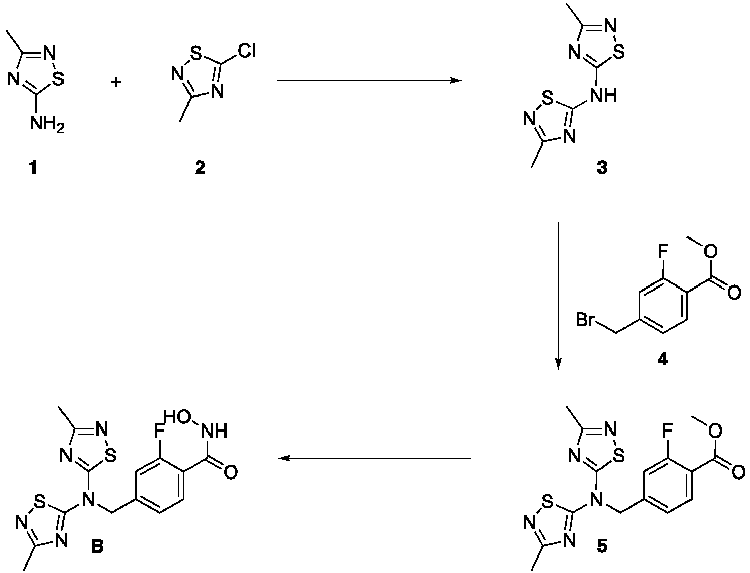

NaH (60% in oil) (50mg) was added to a solution of 3-methyl-1,2,4-thiadiazol-5-amine (1) (115mg, 1mmol) in NMP (2mL). After 10min, 5-chloro-3-methyl-1,2,4-thiadiazole (**2**) (140mg, 1.05mmol) was added and the resultant mixture stirred at 45°C under N₂(g). After 4h, the reaction mixture was diluted with EtOAc and extracted with saturated bicarbonate solution (x3). Analysis indicated that all desired product was in the aqueous phase. The combined aqueous phases were concentrated to dryness; the resultant residue was slurried with MeCN (2 x 100mL) and filtered. The filtrate was concentrated to afford **(3)** as an oil / NMP solution (700mg).
LCMS (ES): found 214.0 [M+H]⁺.

Potassium carbonate (360mg) and methyl 4-(bromomethyl)-2-fluorobenzoate **(4)** (160mg, 0.65mmol) were added to a solution of 3-methyl-N-(3-methyl-1,2,4-thiadiazol-5-yl)-1,2,4-thiadiazol-5-amine **(3)** (<1mmol) in MeCN (10mL) and the reaction mixture was heated, under N₂(g), with stirring, at 50°C. After 2h, the reaction mixture was cooled, diluted with EtOAc and extracted sequentially with water, saturated bicarbonate solution and saturated brine solution, and was then dried over Na₂SO₄, filtered and concentrated. Purification on silica with CH₂Cl₂/MeOH (1:0-97:3) yielded **(5)** as a solid (180mg, 73%).
LCMS (ES): found 380.0 [M+H]⁺.

50% Hydroxylamine aqueous solution (2mL) was added to a solution of methyl 4-{[bis(3-methyl-1,2,4-thiadiazol-5-yl)amino]methyl}-2-fluorobenzoate **(5)** (180mg, 0.47mmol) in MeOH (8mL). The solution was stirred at 45°C for 7 days, sealed in a vial. The resulting reaction mixture became heterogeneous; on cooling, a white solid was collected by filtration, washed with cold methanol and dried *in vacuo* to afford the title product, **Example B,** as solid (50mg, 28%).
¹H NMR (400 MHz, DMSO-*d*₆), δ_{H} ppm: 10.90 (br. s., 1H), 9.17 (br. s., 1H), 7.51 (t, *J*=7.6 Hz, 1H), 7.27 (d, *J*=10.8 Hz, 1H), 7.16 (dd, *J*=7.9, 1.3 Hz, 1H), 5.57 (s, 2H), 2.50 (s, 6H).
LCMS (ES): found 381.0 [M+H]⁺.

### Example C

### 2-Fluoro-N-hydroxy-4-{[(3-methyl-1,2,4-oxadiazol-5-yl)(3-methyl-1,2,4-thiadiazol-5-yl)amino]methyl}benzamide

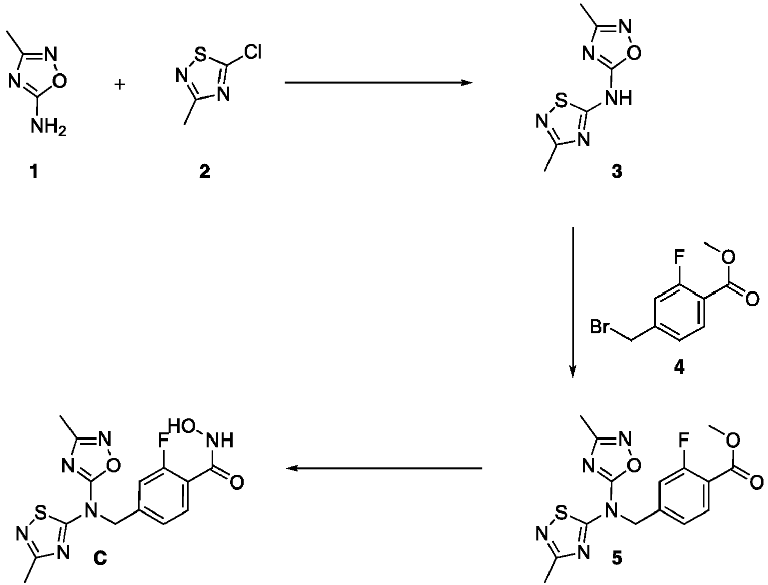

NaH (60% in oil) (50mg) was added to a solution of 3-methyl-1,2,4-oxadiazol-5-amine (1) (100mg, 1mmol) in NMP (2mL). After 10min, 5-chloro-3-methyl-1,2,4-thiadiazole (2) (150mg, 1.1mmol) was added, and the resultant mixture was stirred at 45°C under N₂(g). After 18h, analysis by LCMS was conducted.
LCMS (ES): found 198.0 [M+H]⁺.

NaH (60% in oil) (70mg) and methyl 4-(bromomethyl)-2-fluorobenzoate **(4)** (200mg, 0.81 mmol) were added to the above reaction mixture and heating was continued at 45°C under N₂(g). After 3h, a further quantity of **(4)** (90mg, 0.36mmol) was added. After an additional 2h, the reaction mixture was cooled, diluted with EtOAc, and extracted sequentially with water saturated bicarbonate solution (x2), and was then dried over Na₂SO₄, filtered and concentrated. Purification by silica gel chromatography with CH₂Cl₂/MeOH (1:0-97:3) yielded a residue **(5)** (350mg, 96% over 2 steps).
LCMS (ES): found 364.0 [M+H]⁺.

50% Hydroxylamine aqueous solution (1mL) was added to a crude solution of methyl 4-{[bis(3-methyl-1,2,4-thiadiazol-5-yl)amino]methyl}-2-fluorobenzoate **(5)** (350mg, 0.96mmol) in methanol (5mL). The resulting solution was stirred at 45-50°C for 5 days, sealed in a vial. The reaction mixture turned heterogeneous and, on cooling, a white solid was filtered off and the resulting filtrate was concentrated. The filtrate was purified by RP-HPLC on Xterra 10-70% MeCN/water + 0.1% formic acid, to furnish the title compound, **Example C** (30mg, 8%).
¹H NMR (400 MHz, Methanol-*d*₄), δ_{H} ppm: 7.69 (t, *J*=7.6 Hz, 1H), 7.12-7.22 (m, 2H), 5.48 (s, 2H), 2.44 (s, 3H), 2.32 (s, 3H).
LCMS (ES): found 365.0 [M+H]⁺.

### Example D

### N-Hydroxy-4-(((3-methyl-1,2,4-oxadiazol-5-yl)(pyridin-2-yl)amino)methyl)benzamide

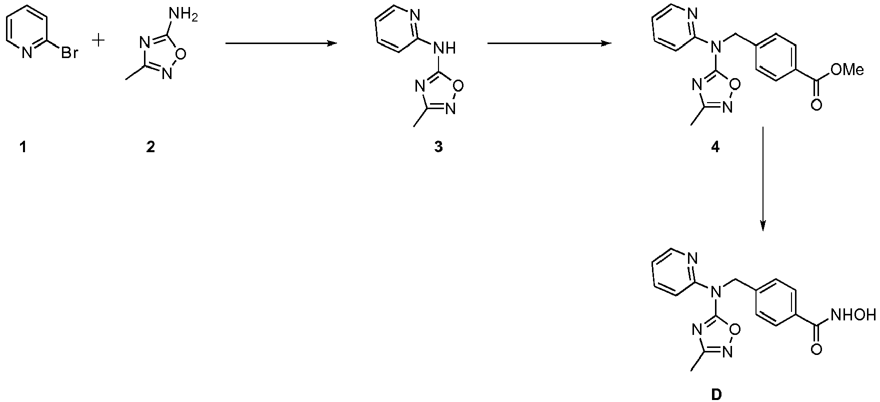

2-Bromopyridine **(1)** (1.0g, 6.32mmol), 3-methyl-1,2,4-oxadiazol-5-amine (**2)** (0.940g, 9.49mmol), Xantphos (0.366g, 0.63mmol), and Cs₂CO₃ (4.1g, 12.64mmol) were combined in dry 1,4-dioxane (15mL). The reaction mixture was degassed with N₂(g) and placed under vacuum for 10min. Pd₂(dba)₃ (0.28g, 0.31mmol) was then added to the reaction mixture, which was heated at 90°C for 30h. It was then poured into demineralized water (200mL) and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide 3-methyl-N-(pyridin-2-yl)-1, 2, 4-oxadiazol-5-amine **(3)** as a white solid (0.7g, 63%). LCMS (ES): Found 177.1 [M+H]⁺.

NaH (60%) (52.5mg, 1.31mmol) was added portion-wise to 3-methyl-N-(pyridin-2-yl)-1,2,4-oxadiazol-5-amine **(3)** (220mg,1.25mmol) in DMF (5mL) at 5°C under Ar(g). The reaction mixture was stirred for 20min, then methyl 4-(bromomethyl) benzoate (372mg, 1.62mmol) was added, and stirring was continued at 80°C under Ar(g) for 1h. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (1:1) to furnish methyl 4-(((3-methyl-1,2,4-oxadiazol-5-yl)(pyridin-2-yl)amino)methyl)benzoate **(4)** as a white solid (130mg, 40%).
LCMS (ES): Found 325.1 [M+H]⁺.

A fresh solution of NH₂OH in MeOH was prepared: [KOH (0.91 g, 16.3mmol) in MeOH (10mL) was added to NH₂OH.HCl (1.12g, 16.3mmol) in MeOH (10mL) at 0°C]. The reaction mixture was stirred for 20min at 0°C, then filtered to remove salts; it was then added to methyl 4-(((3-methyl-1,2,4-oxadiazol-5-yl)(pyridin-2-yl)amino)methyl)benzoate **(4)** (105.5mg, 0.3mmol) followed by KOH (181mg, 3.2mmol) solubilized in MeOH (5mL). The reaction mixture was stirred at rt for 21h, and then concentrated *in vacuo,* poured onto brine/H₂O (15mL/35mL), and extracted with CH₂Cl₂ (3 x 50mL). The organic phases were combined, dried over Na₂SO₄, filtered and subsequently concentrated in vacuo. The resulting residue was purified by flash chromatography with MeOH/CH₂Cl₂ (10:90) to provide N-hydroxy-8-((3-methyl-1,2,4-oxadiazol-5-yl)(pyridin-2-yl)amino)octanamide, **Example D,** as a light yellow solid (12.2mg, 40%).
¹H NMR (400 MHz, DMSO-*d*₆), δ_{H} ppm: 11.14 (br. s., 1H), 9.01 (br. s., 1H), 8.42 (dd, *J*=4.8, 1.1 Hz, 1H), 8.07 (d, *J*=8.4 Hz, 1H), 7.92 (ddd, *J*=8.5, 7.4, 2.0 Hz, 1H), 7.66 (d, *J*=8.3 Hz, 2H), 7.34 (d, *J*=8.3 Hz, 2H), 7.23 (ddd, *J*=7.3, 4.9, 0.8 Hz, 1H), 5.48 (s, 2H), 2.23 (s, 3H).
LCMS (ES): Found 326.1 [M+H]⁺.

### Example E

### N-Hydroxy-4-(((1-methyl-1H-pyrazol-3-yl)(pyridin-2-yl)amino)methyl)benzamide

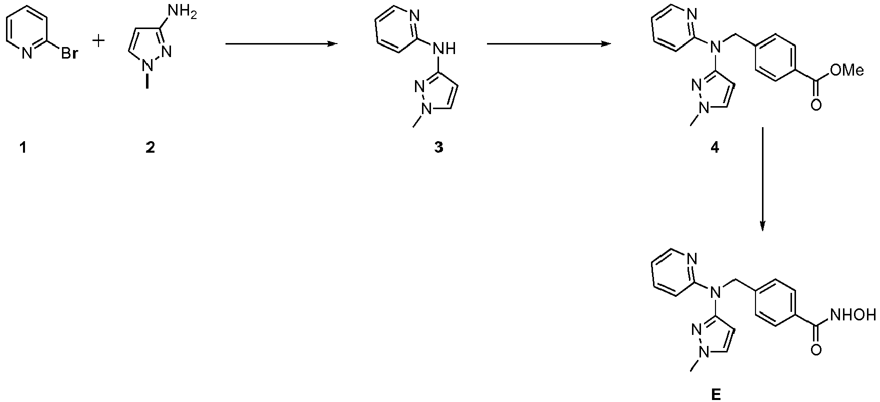

2-Bromopyridine **(1)** (1.0g, 6.3mmol), 1-methyl-1H-pyrazol-3-amine **(2)** (0.79g, 8.2mmol), Xantphos (0.37g, 0.63mmol), and CS₂CO₃ (4.1g, 12.6mmol) were combined in dry 1,4-dioxane (15mL). The reaction mixture was then degassed with N₂(g), and placed under vacuum for 10min. Pd₂(dba)₃ (0.29g, 0.31mmol) was added and the resulting reaction mixture was heated at 90°C for 30h. It was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide N-(1-methyl-1H-pyrazol-3-yl)pyridin-2-amine **(3)** as a yellow solid (0.75g, 68%).
LCMS (ES): Found 175.2 [M+H]⁺.

NaH (60%) (60.4mg, 1.5mmol) was added portion-wise to N-(1-methyl-1H-pyrazol-3-yl)pyridin-2-amine **(3)** (250mg,1.4mmol) in DMF (8mL) at 5°C under Ar(g). The reaction mixture was stirred for 20min, then methyl 4-(bromomethyl) benzoate (428mg, 1.8mmol) was added, and stirring was continued at 70°C under Ar(g) for 1h. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (3:7) to furnish methyl 4-(((1-methyl-1H-pyrazol-3-yl)(pyridin-2-yl)amino)methyl)benzoate **(4)** as a light yellow solid (440mg, 82%).
LCMS (ES): Found 323.1 [M+H]⁺.

A fresh solution of NH₂OH in MeOH was prepared: [KOH (3.83g, 68.3mmol) in MeOH (20mL) was added to NH₂OH.HCl (4.74g, 68.3mmol) in MeOH (20mL) at 0°C]. The reaction mixture was stirred for 20min at 0°C, then filtered to remove salts; it was then added to 4-(((1-methyl-1H-pyrazol-3-yl)(pyridin-2-yl)amino)methyl)benzoate **(4)** (440mg, 1.3mmol) followed by KOH (766mg, 13.0mmol) solubilized in MeOH (10mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to provide N-hydroxy-4-(((1-methyl-1H-pyrazol-3-yl)(pyridin-2-yl)amino)methyl)benzamide, **Example E,** as a light brown liquid (50mg, 11%).
¹H NMR (400 MHz, Methanol-*d*₄), δ_{H} ppm: 8.09 (ddd, *J*=5.0, 1.9, 0.8 Hz, 1H), 7.64 (d, *J*=8.3 Hz, 2H), 7.52 (d, *J*=2.3 Hz, 1H), 7.49 (ddd, *J*=8.7, 7.0, 1.9 Hz, 1H), 7.40 (d, *J*=8.4 Hz, 2H), 6.91 (d, *J*=8.6 Hz, 1H), 6.73 (ddd, *J*=7.1, 5.1, 0.7 Hz, 1H), 6.10 (d, *J*=2.4 Hz, 1H), 5.26 (s, 2H), 3.81 (s, 3H).
LCMS (ES): Found 324.4 [M+H]⁺.

### Example F

### N-Hydroxy-4-((pyridin-2-yl(1,3,4-thiadiazol-2-yl)amino)methyl)benzamide

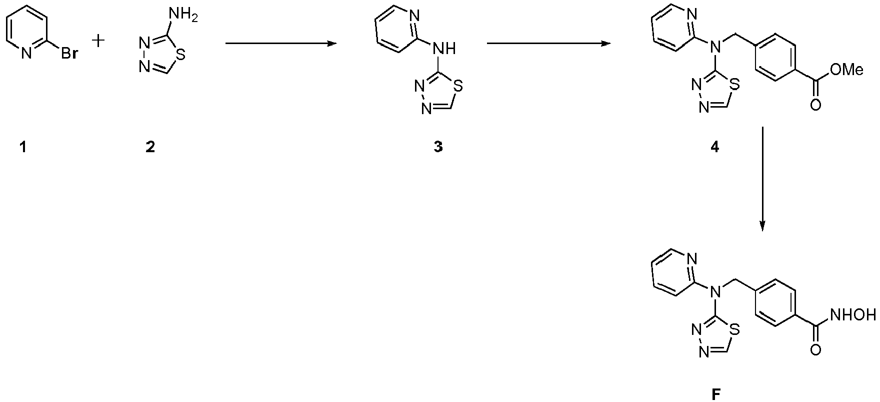

2-Bromopyridine **(1)** (1.0g, 6.3mmol), 1,3,4-thiadiazol-2-amine **(2)** (0.64g, 6.3mmol), Xantphos (0.37g, 0.63mmol), and Cs₂CO₃ (3.1g, 9.4mmol) were combined in dry 1,4-dioxane (15mL). The reaction mixture was degassed with N₂(g) and placed under vacuum for 10min. Pd₂(dba)₃ (0.29g, 0.31mmol) was then added and the resulting reaction mixture was then heated at 90°C for 30h. It was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide N-(pyridin-2-yl)-1, 3, 4-thiadiazol-2-amine **(3)** as a yellow solid (0.33g, 30%).
LCMS (ES): Found 179.0 [M+H]⁺.

NaH (60%) (53mg, 1.3mmol) was added portion-wise to N-(pyridin-2-yl)-1,3,4-thiadiazol-2-amine **(3)** (225mg,1.26mmol) in DMF (8mL) at 5°C under Ar(g). The reaction mixture was stirred for 20min, then methyl 4-(bromomethyl)benzoate (336mg, 1.6mmol) was added, and stirring was continued at 70°C under Ar(g) for 1h in the dark. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (3:7) to furnish methyl 4-((pyridin-2-yl(1,3,4-thiadiazol-2-yl)amino)methyl)benzoate **(4)** as a light yellow solid (118mg, 33%).
LCMS (ES): Found 327.3 [M+H]⁺.

A fresh solution of NH₂OH in MeOH was prepared: [KOH (1.01g, 18.1mmol) in MeOH (20mL) was added to NH₂OH.HCl (1.26g, 18.1mmol) in MeOH (20mL) at 0°C]. The reaction mixture was stirred for 20min at 0°C, then filtered to remove salts; it was then added to methyl 4-((pyridin-2-yl(1,3,4-thiadiazol-2-yl)amino)methyl)benzoate **(4)** (118mg, 0.36mmol) followed by KOH (203mg, 3.6mmol) solubilized in MeOH (10mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to provide N-hydroxy-4-((pyridin-2-yl(1,3,4-thiadiazol-2-yl)amino)methyl)benzamide, **Example F,** as a light brown liquid (15mg, 13%).
¹H NMR (400 MHz, Methanol-*d*₄), δ_{H} ppm: 8.96 (s, 1H), 8.44 (dd, *J*=5.0, 1.1 Hz, 1H), 7.72-7.78 (m, 1H), 7.69 (d, *J*=8.2 Hz, 2H), 7.33 (d, *J*=8.2 Hz, 2H), 7.06-7.11 (m, 2H), 5.79 (s, 2H).
LCMS (ES): Found 328.1 [M+H]⁺.

### Example G

### N-Hydroxy-4-((pyrazin-2-yl(pyridin-2-yl)amino)methyl)benzamide

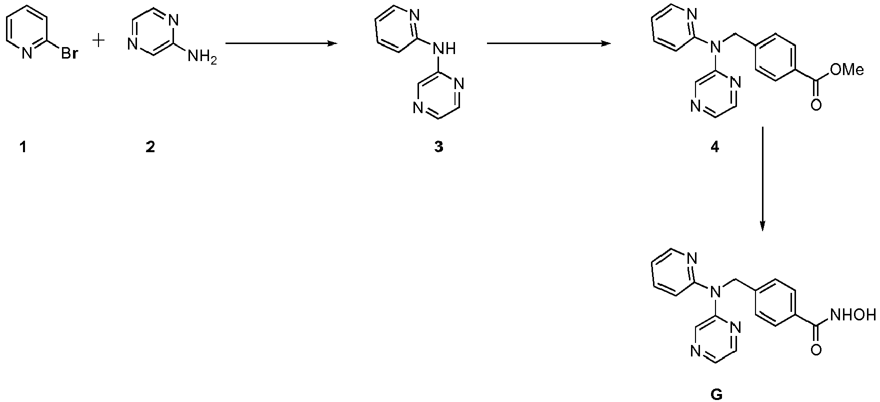

2-Bromopyridine **(1)** (1.0g, 6.3mmol), pyrazin-2-amine **(2)** (0.67g, 6.9mmol), BINAP (0.12g, 0.18mmol), t-BuOK (0.99g, 8.8mmol) were combined in dry toluene (15 mL). The reaction mixture was degassed with N₂(g) and placed under vacuum for 10min. Pd₂(dba)₃ (0.11g,0.12mmol) was added, and the mixture heated at 90°C for 3 h. It was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide N-(pyridin-2-yl)pyrazin-2-amine **(3)** as a yellow solid (0.9g, 83%). LCMS (ES): Found 173.1 [M+H]⁺.

NaH (60%) (61mg, 1.52mmol) was added portion-wise to N-(pyridin-2-yl)pyrazin-2-amine **(3)** (250mg,1.45mmol) in DMF (10mL) at 5°C under Ar(g). The reaction mixture was stirred for 20min, then methyl 4-(bromomethyl) benzoate (432mg, 1.88mmol) was added, and stirring was continued at 70°C under Ar(g) for 1h in the dark. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (3:7) to furnish methyl 4-((pyrazin-2-yl(pyridin-2-yl)amino)methyl)benzoate **(4)** as a light yellow solid (380mg, 81%).
LCMS (ES): Found 321.3 [M+H]⁺.

A fresh solution of NH₂OH in MeOH was prepared: [KOH (3.33g, 59.0mmol) in MeOH (20mL) was added to NH₂OH.HCl (4.1g, 59.0mmol) in MeOH (20mL) at 0°C]. The reaction mixture was stirred for 20min at 0°C, then filtered to remove salts; it was then added to methyl 4-((pyrazin-2-yl(pyridin-2-yl)amino)methyl)benzoate **(4)** (380mg, 1.1mmol) followed by KOH (666mg, 11.8mmol) solubilized in MeOH (10mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to provide N-hydroxy-4-((pyrazin-2-yl(pyridin-2-yl)amino)methyl)benzamide, **Example G,** as a light cream solid (20mg, 5%).
¹H NMR (400 MHz, DMSO-*d*₆), δ_{H} ppm: 11.10 (br. s., 1H), 8.99 (br. s., 1H), 8.65 (d, *J*=1.4 Hz, 1H), 8.32 (ddd, *J*=4.9, 1.9, 0.8 Hz, 1H), 8.27 (dd, *J*=2.7, 1.5 Hz, 1H), 8.10 (d, *J*=2.6 Hz, 1H), 7.74 (ddd, *J*=8.4, 7.3, 2.0 Hz, 1H), 7.64 (d, *J*=8.3 Hz, 2H), 7.36 (d, *J*=8.2 Hz, 2H), 7.33 (d, *J*=8.4 Hz, 1H), 7.06 (ddd, *J*=7.3, 4.9, 0.8 Hz, 1H), 5.45 (s, 2H).
LCMS (ES): Found 322.3 [M+H]⁺.

### Example H

### N-Hydroxy-4-(((5-methyl-1,3,4-thiadiazol-2-yl)(pyridin-2-yl)amino)methyl)benzamide

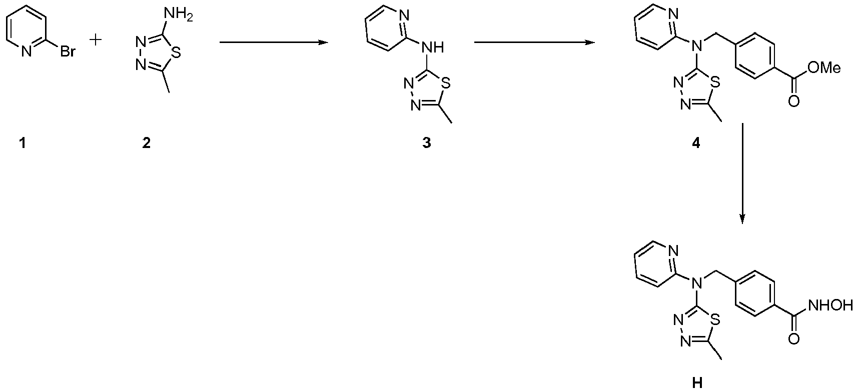

2-Bromopyridine **(1)** (1.0g, 6.3mmol), 5-methyl-1,3,4-thiadiazol-2-amine **(2)** (0.947g, 8.2mmol), Xantphos (0.366g, 0.63mmol), and Cs₂CO₃ (3.09g, 9.4mmol) were combined in dry 1,4-dioxane (15mL). The reaction mixture was degassed with N₂(g) and placed under vacuum for 10min. Pd₂(dba)₃ (0.289g, 0.31mmol) was then added and the resulting reaction mixture was heated at 90°C for 30h. It was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide 5-methyl-N-(pyridin-2-yl)-1, 3, 4-thiadiazol-2-amine **(3)** as a yellow solid (0.22g, 18%).
LCMS (ES): Found 193.2 [M+H]⁺.

NaH (60%) (109.3mg, 1.3mmol) was added portion-wise to 5-methyl-N-(pyridin-2-yl)-1,3,4-thiadiazol-2-amine **(3)** (500mg,2.6mmol) in DMF (8mL) at 5°C under Ar(g). The reaction mixture was stirred for 20min, then methyl 4-(bromomethyl)benzoate (775mg, 3.3mmol) was added, and stirring was continued at 70°C under Ar(g) for 1h in the dark. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (1:3) to furnish methyl 4-(((5-methyl-1,3,4-thiadiazol-2-yl)(pyridin-2-yl)amino)methyl)benzoate **(4)** as a light yellow solid (134mg, 39%).
LCMS (ES): Found 341.4 [M+H]⁺.

A fresh solution of NH₂OH in MeOH was prepared: [KOH (1.0g, 19.7mmol) in MeOH (20mL) was added to NH₂OH.HCl (1.36g, 19.7mmol) in MeOH (20mL) at 0°C]. The reaction mixture was stirred for 20min at 0°C, then filtered to remove salts; it was then added to methyl 4-(((5-methyl-1,3,4-thiadiazol-2-yl)(pyridin-2-yl)amino)methyl)benzoate **(4)** (134mg, 0.39mmol) followed by KOH (221mg, 3.9mmol) solubilized in MeOH (10mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to provide N-hydroxy-4-(((5-methyl-1,3,4-thiadiazol-2-yl)(pyridin-2-yl)amino)methyl)benzamide, **Example H,** as a light brown liquid (15mg, 11%). ¹H NMR (400 MHz, Methanol-*d*₄), δ_{H} ppm: 8.42 (dd, J=4.9, 1.1 Hz, 1H), 7.73 (ddd, J=8.6, 7.2, 1.8 Hz, 1H), 7.69 (d, J=8.3 Hz, 2H), 7.33 (d, J=8.2 Hz, 2H), 7.02-7.09 (m, 2H), 5.72 (s, 2H), 2.65 (s, 3H).
LCMS (ES): Found 342.1 [M+H]⁺.

### Example I

### 4-((Benzo[d]oxazol-2-yl(pyridin-2-yl)amino)methyl)-N-hydroxybenzamide

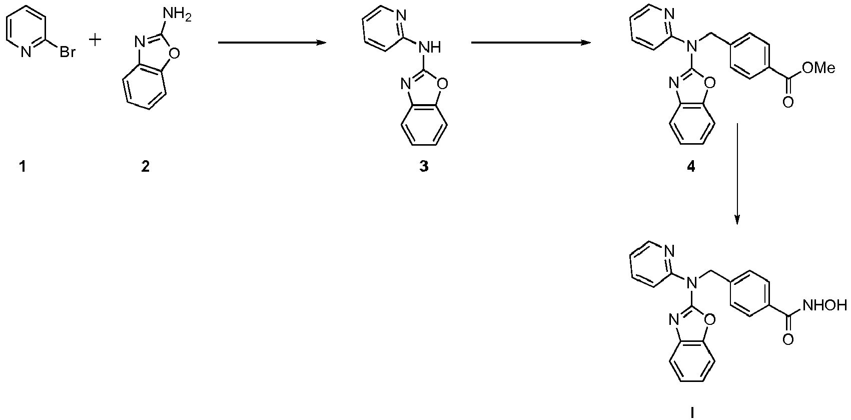

2-Bromopyridine **(1)** (1.0g, 6.3mmol), benzo[d]oxazol-2-amine **(2)** (0.871g, 6.4mmol), Xantphos (0.37g, 0.63mmol), and Cs₂CO₃ (3.09g, 9.4mmol) were combined in dry 1,4-dioxane (15mL). The reaction mixture was degassed with N₂(g) and placed under vacuum for 10min. Pd₂(dba)₃ (0.289g, 0.31 mmol) was then added and the resulting reaction mixture was heated at 90°C for 30h. It was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide N-(pyridin-2-yl)benzo[d]oxazol-2-amine **(3)** as a yellow solid (0.8g, 60%).
LCMS (ES): Found 212.1 [M+H]⁺.

NaH (60%) (53mg, 1.3mmol) was added portion-wise to N-(pyridin-2-yl)benzo[d]oxazol-2-amine **(3)** (265mg, 1.28mmol) in DMF (8mL) at 5°C under Ar(g). The reaction mixture was stirred for 20min, then methyl 4-(bromomethyl) benzoate (380mg, 1.66mmol) was added, and stirring was continued at 70°C under Ar(g) for 1h. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (3:7) to furnish methyl 4-((benzo[d]oxazol-2-yl(pyridin-2-yl)amino)methyl)benzoate **(4)** as a light yellow solid (220mg, 48%).
LCMS (ES): Found 360.1 [M+H]⁺.

A fresh solution of NH₂OH in MeOH was prepared: [KOH (1.75g, 31.0mmol) in MeOH (15mL) was added to NH₂OH.HCl (2.16g, 31.0mmol) in MeOH (15mL) at 0°C. The reaction mixture was stirred for 20min at 0°C, then filtered to remove salts; it was then added to methyl 4-((benzo[d]oxazol-2-yl(pyridin-2-yl)amino)methyl)benzoate **(4)** (220mg, 0.62mmol) followed by KOH (348mg, 6.2mmol) solubilized in MeOH (5mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to provide 4-((benzo[d]oxazol-2-yl(pyridin-2-yl)amino)methyl)-N-hydroxybenzamide, **Example I,** as a light orange solid (50mg, 23%).
¹H NMR (400 MHz, DMSO-*d*₆), δ_{H} ppm: 11.12 (br. s., 1H), 9.00 (br. s., 1H), 8.40 (dd, *J*=4.7, 1.8 Hz, 1H), 8.17 (d, *J*=8.4 Hz, 1H), 7.88-7.94 (m, 1H), 7.65 (d, *J*=8.2 Hz, 2H), 7.47-7.55 (m, 2H), 7.41 (d, *J*=8.2 Hz, 2H), 7.26 (t, *J*=7.8 Hz, 1H), 7.14-7.22 (m, 2H), 5.59 (s, 2H).
LCMS (ES): Found 361.1 [M+H]⁺.

### Example J

### N-Hydroxy-4-(((1-methyl-1H-benzo[d]imidazol-2-yl)(pyridin-2-yl)amino)methyl)benzamide

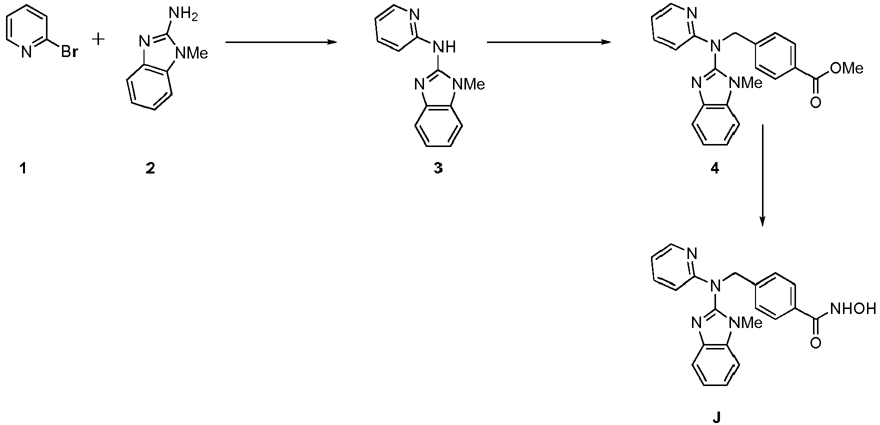

2-Bromopyridine **(1)** (1.0g, 6.3mmol), 1-methyl-1H-pyrazol-3-amine **(2)** (1.21g, 6.9mmol), Xantphos (0.37g, 0.63mmol), and Cs₂CO₃ (4.1g, 12.6mmol) were combined in dry 1,4-dioxane (15mL). The reaction mixture was degassed with N₂(g) and placed under vacuum for 10min. Pd₂(dba)₃ (0.29g, 0.31mmol) was then added and the resulting reaction mixture was heated at 90°C for 30h. It was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide 1-methyl-N-(pyridin-2-yl)-1H-benzo[d]imidazol-2-amine **(3)** as a yellow solid (0.35g, 25%).
LCMS (ES): Found 225.1 [M+H]⁺.

NaH (60%) (32.8mg, 0.82mmol) was added portion-wise to 1-methyl-N-(pyridin-2-yl)-1H-benzo[d]imidazol-2-amine **(3)** (175mg, 0.78mmol) in DMF (5mL) at 5°C under Ar(g). The reaction mixture was stirred for 20min, then methyl 4-(bromomethyl) benzoate (232mg, 1.01mmol) was added, and stirring was continued at 70°C under Ar(g) for 1h in the dark. The reaction mixture was then poured onto demineralized water (100mL), and extracted with EtOAc (3 x 50mL). The organic phases were combined, dried over Na₂SO₄, filtered and subsequently concentrated *in vacuo.* The residue was purified by flash chromatography with EtOAc/Hexane (3:7) to furnish methyl 4-(((1-methyl-1H-benzo[d]imidazol-2-yl)(pyridin-2-yl)amino)methyl)benzoate **(4)** as a light yellow solid (42mg, 16%).
LCMS (ES): Found 373.2 [M+H]⁺.

A fresh solution of NH₂OH in MeOH was prepared: [KOH (1.07g, 19.0mmol) in MeOH (10mL) was added to NH₂OH.HCl (530mg, 19.0mmol) in MeOH (10mL) at 0°C]. The reaction mixture was stirred for 20min at 0°C, then filtered to remove salts; it was then added to methyl 4-(((1-methyl-1H-benzo[d]imidazol-2-yl)(pyridin-2-yl)amino)methyl)benzoate **(4)** (142mg, 0.38mmol) followed by KOH (214mg, 3.8mmol) solubilized in MeOH (5mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (10:90) to provide N-hydroxy-4-(((1-methyl-1H-benzo[d]imidazol-2-yl)(pyridin-2-yl)amino)methyl)benzamide, **Example J,** as an off white solid (9mg, 7%).
¹H NMR (400 MHz, Methanol-*d*₄), δ_{H} ppm: 8.23 (dd, *J*=5.0, 1.1 Hz, 1H), 7.65 (d, *J*=8.3 Hz, 2H), 7.58-7.63 (m, 2H), 7.52 (d, *J*=8.2 Hz, 2H), 7.41 (dd, *J*=6.8, 1.9 Hz, 1H), 7.24-7.32 (m, 2H), 6.92 (dd, *J*=6.8, 5.1 Hz, 1H), 6.56 (d, *J*=8.4 Hz, 1H), 5.37 (s, 2H), 3.37-3.42 (m, 3H).
LCMS (ES): Found 374.3 [M+H]⁺.

### Example K

### N-Hydroxy-4-((pyridin-2-yl(1,2,4-thiadiazol-5-yl)amino)methyl)benzamide

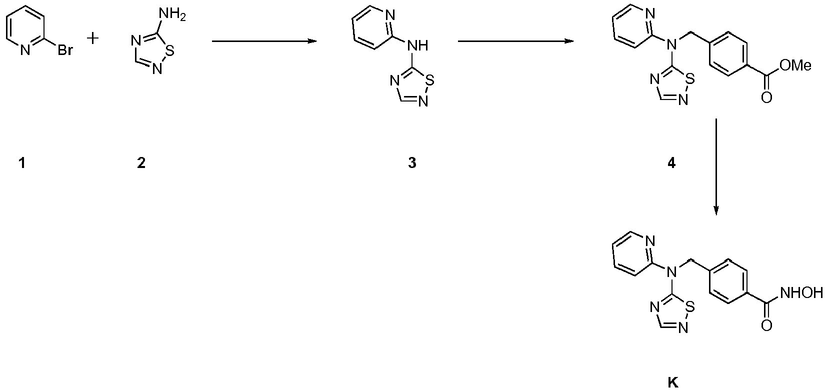

2-Bromopyridine **(1)** (1.0g, 6.3mmol), 1, 2, 4-thiadiazol-5-amine **(2)** (0.830g, 8.22mmol), Xantphos (0.366g, 0.63mmol), and Cs₂CO₃ (3.09g, 9.4mmol) were combined in dry 1,4-dioxane (15mL). The reaction mixture was degassed with N₂(g) and placed under vacuum for 10min. Pd₂(dba)₃ (0.29g, 0.31mmol) was then added and the resulting reaction mixture was heated at 90°C for 30h. It was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide N-(pyridin-2-yl)-1, 2, 4-thiadiazol-5-amine **(3)** as a yellow solid (0.188g, 16%).
LCMS (ES): Found 179.0 [M+H]⁺

NaH (60%) (49mg, 1.23mmol) was added portion-wise to N-(pyridin-2-yl)-1,2,4-thiadiazol-5-amine **(3)** (210mg, 1.19mmol) in DMF (8mL) at 5°C under Ar(g). The reaction mixture was stirred for 20min, then methyl 4-(bromomethyl)benzoate (351mg, 1.5mmol) was added, and stirring was continued at 70°C under Ar(g) for 1h in the dark. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (3:7) to furnish methyl 4-((pyridin-2-yl(1,2,4-thiadiazol-5-yl)amino)methyl)benzoate **(4)** as a light yellow solid (110mg, 28%).
LCMS (ES): Found 327.4 [M+H]⁺.

A fresh solution of NH₂OH in MeOH was prepared: [KOH (949mg, 16.9mmol) in MeOH (10mL) was added to NH₂OH.HCl (1.17g, 16.9mmol) in MeOH (10mL) at 0°C]. The reaction mixture was stirred for 20min at 0°C, then filtered to remove salts; it was then added to methyl 4-((pyridin-2-yl(1,2,4-thiadiazol-5-yl)amino)methyl)benzoate **(4)** (110mg, 0.33mmol) followed by KOH (185mg, 3.3mmol) solubilized in MeOH (5mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to provide N-hydroxy-4-((pyridin-2-yl(1,2,4-thiadiazol-5-yl)amino)methyl)benzamide,
**Example K,** as a light orange solid (11mg, 10%).
¹H NMR (400 MHz, Methanol-*d*₄), δ_{H} ppm: 8.54 (d, *J*=4.3 Hz, 1H), 8.22-8.31 (m, 1H), 7.81 (br. s., 1H), 7.65-7.76 (m, 2H), 7.08-7.38 (m, 4H), 5.82 (s, 2H).
LCMS (ES): Found 328.0 [M+H]⁺.

### Example L

### 4-(((5-Fluoropyridin-2-yl)(pyrazin-2-yl)amino)methyl)-N-hydroxybenzamide

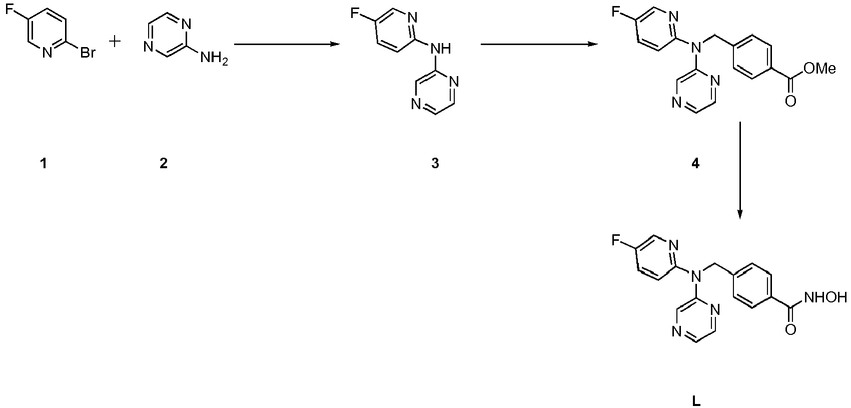

2-Bromo-5-fluoropyridine **(1)** (1.0g, 5.71mmol), pyrazin-2-amine **(2)** (543mg, 5.71mmol), Xantphos (0.330g, 0.57mmol), Cs₂CO₃ (2.79g, 8.56mmol) were combined in dry 1,4-dioxane (15mL). The reaction mixture was degassed with N₂(g), and placed under vacuum for 10min. Pd₂(dba)₃ (0.26g, 0.28mmol) was added and the reaction mixture was then heated at 90°C for 30h. It was then poured onto demineralized water (200 mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide N-(5-fluoropyridin-2-yl)pyrazin-2-amine **(3)** as a yellow solid (0.56g, 51%).
LCMS (ES): Found 191.1 [M+H]⁺.

NaH (60%) (39mg, 0.99mmol) was added portion-wise to N-(5-fluoropyridin-2-yl)pyrazin-2-amine **(3)** (180mg, 0.94mmol) in DMF (5mL) at 5°C under Ar(g). The reaction mixture was stirred for 20min, then methyl 4-(bromomethyl) benzoate (281mg, 1.23mmol) was added, and stirring was continued at 70°C under Ar(g) for 1h. The reaction mixture was then poured onto demineralized water (100mL), and extracted with EtOAc (3 x 50mL). The organic phases were combined, dried over Na₂SO₄, filtered and subsequently concentrated in vacuo. The resulting residue was purified by flash chromatography with EtOAc/Hexane (3:7) to furnish methyl 4-(((5-fluoropyridin-2-yl)(pyrazin-2-yl)amino)methyl)benzoate **(4)** as a light yellow solid (190mg, 59%).
LCMS (ES): Found 339.1 [M+H]⁺.

A fresh solution of NH₂OH in MeOH was prepared: [KOH (1.57g, 28.1mmol) in MeOH (15mL) was added to NH₂OH.HCl (1.95g, 28.1mmol) in MeOH (15mL) at 0°C]. The reaction mixture was stirred for 20min at 0°C, then filtered to remove salts; it was then added to methyl 4-(((5-fluoropyridin-2-yl)(pyrazin-2-yl)amino)methyl)benzoate **(4)** (190mg, 0.56mmol) followed by KOH (315mg, 5.6mmol) solubilized in MeOH (5mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to provide 4-(((5-fluoropyridin-2-yl)(pyrazin-2-yl)amino)methyl)-N-hydroxybenzamide,
**Example L,** as a creamish solid (40mg, 21%).
¹H NMR (400 MHz, DMSO-*d*₆), δ_{H} ppm: 11.08 (br. s, 1H), 8.84-9.09 (m, 1H), 8.54 (d, *J*=1.4 Hz, 1H), 8.34 (d, *J*=3.1 Hz, 1H), 8.24 (dd, *J*=2.7, 1.5 Hz, 1H), 8.09 (d, *J*=2.7 Hz, 1H), 7.72 (ddd, *J*=9.0, 8.2, 3.1 Hz, 1H), 7.64 (d, *J*=8.3 Hz, 2H), 7.46 (dd, *J*=9.1, 3.7 Hz, 1H), 7.37 (d, *J*=8.3 Hz, 2H), 5.42 (s, 2H)
LCMS (ES): Found 340.1 [M+H]⁺.

### Example M

### 4-(((5-Fluoropyridin-2-yl)(3-methyl-1,2,4-oxadiazol-5-yl)amino)methyl)-N-hydroxybenzamide

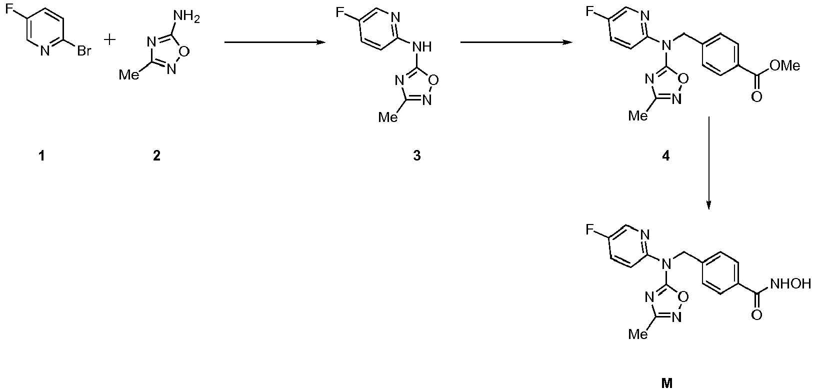

2-Bromo-5-fluoropyridine **(1)** (1.0g, 5.71mmol), 3-methyl-1, 2, 4-oxadiazol-5-amine **(2)** (566mg, 5.71mmol), Xantphos (0.330g, 0.57mmol), and Cs₂CO₃ (2.79g, 8.56mmol) were combined in dry 1,4-dioxane (15mL). The reaction mixture was degassed with N₂(g) and placed under vacuum for 10min. Pd₂(dba)₃ (0.261g, 0.28mmol) was then added and the resulting reaction mixture was heated at 90°C for 30h. It was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide N-(5-fluoropyridin-2-yl)-3-methyl-1, 2, 4-oxadiazol-5-amine **(3)** as a yellow solid (0.70g, 63%).
LCMS (ES): Found 195.0 [M+H]⁺.

NaH (60%) (56mg, 1.4mmol) was added portion-wise to N-(5-fluoropyridin-2-yl)-3-methyl-1,2,4-oxadiazol-5-amine **(3)** (260mg, 1.34mmol) in DMF (10mL) at 5°C under Ar(g). The reaction mixture was stirred for 20min, then methyl 4-(bromomethyl) benzoate (398mg, 1.7mmol) was added, and stirring was continued at 70°C under Ar(g) for 1h. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (3:7) to furnish methyl-4-(((5-fluoropyridin-2-yl)(3-methyl-1,2,4-oxadiazol-5-yl)amino)methyl)benzoate **(4)** as a light yellow solid (170mg, 37%).
LCMS (ES): Found 343.1 [M+H]⁺.

A fresh solution of NH₂OH in MeOH was prepared: [KOH (1.39g, 24.8mmol) in MeOH (15mL) was added to NH₂OH.HCl (1.72g, 24.8mmol) in MeOH (15mL) at 0°C]. The reaction mixture was stirred for 20min at 0°C, then filtered to remove salts; it was then added to methyl 4-(((5-fluoropyridin-2-yl)(3-methyl-1,2,4-oxadiazol-5-yl)amino)methyl)benzoate **(4)** (170mg, 0.49mmol) followed by KOH (278mg, 4.9mmol) solubilized in MeOH (5mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to provide 4-(((5-fluoropyridin-2-yl)(3-methyl-1,2,4-oxadiazol-5-yl)amino)methyl)-N-hydroxybenzamide, **Example M,** as a light orange solid (20mg, 12%).
¹H NMR (400 MHz, DMSO-*d*₆), δ_{H} ppm: 11.11 (br. s., 1H), 9.01 (br. s., 1H), 8.43 (d, *J*=3.0 Hz, 1H), 8.11 (dd, *J*=9.2, 3.8 Hz, 1H), 7.89 (td, *J*=8.6, 3.1 Hz, 1H), 7.67 (d, *J*=8.3 Hz, 2H), 7.34 (d, *J*=8.2 Hz, 2H), 5.43 (s, 2H), 2.22 (s, 4H).
LCMS (ES): Found 344.1 [M+H]⁺.

### Example N

### 4-(((5-Fluoropyridin-2-yl)(1-methyl-1H-benzo[d]imidazol-2-yl)amino)methyl)-N-hydroxybenzamide

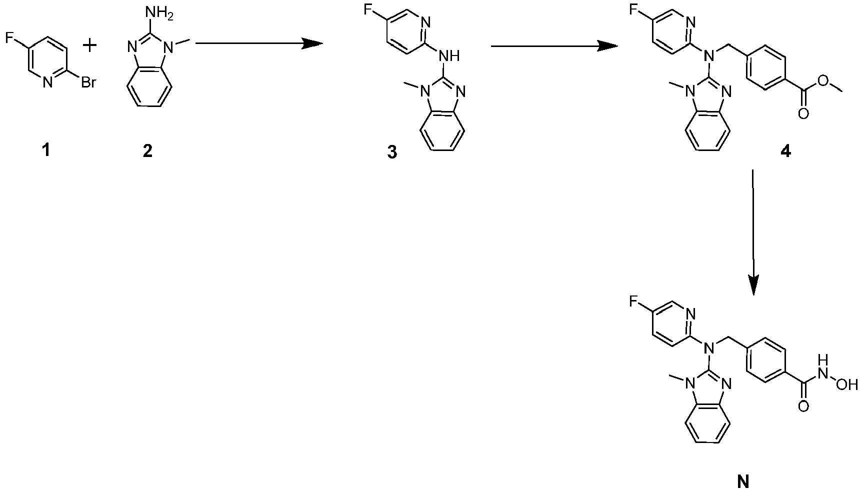

2-Bromo-5-fluoropyridine **(1)** (1.0g, 5.71mmol), 1-methyl-1H-benzo[d]imidazol-2-amine **(2)** (840mg, 5.71mmol), Xantphos (0.33g, 0.57mmol), and Cs₂CO₃ (2.79g, 8.56mmol) were combined in dry 1,4-dioxane (15mL). The reaction mixture was degassed with N₂(g) and placed under vacuum for 10min. Pd₂(dba)₃ (0.26g, 0.28mmol) was then added and the resulting reaction mixture was heated at 90°C for 30h. It was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide N-(5-fluoropyridin-2-yl)-1-methyl-1H-benzo[d]imidazol-2-amine **(3)** as a yellow solid (0.56g, 41%).
LCMS (ES): Found 243.1 [M+H]⁺.

NaH (60%) (27mg, 0.66mmol) was added portion-wise to N-(5-fluoropyridin-2-yl)-1-methyl-1H-benzo[d]imidazol-2-amine **(3)** (154mg, 0.63mmol) in DMF (5mL) at 5°C under Ar(g). The reaction mixture was stirred for 20min, then methyl 4-(bromomethyl) benzoate (189mg, 0.82mmol) was added, and stirring was continued at 70°C under Ar(g) for 1h. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (3:7) to furnish methyl 4-(((5-fluoropyridin-2-yl)(1-methyl-1H-benzo[d]imidazol-2-yl)amino)methyl)benzoate **(4)** as a light yellow solid (165mg, 66%).
LCMS (ES): Found 391.2 [M+H]⁺.

A fresh solution of NH₂OH in MeOH was prepared: [KOH (1.20g, 21.4mmol) in MeOH (15mL) was added to NH₂OH.HCl (1.48g, 21.4mmol) in MeOH (15mL) at 0°C]. The reaction mixture was stirred for 20min at 0°C, then filtered to remove salts; it was then added to methyl4-(((5-fluoropyridin-2-yl)(1-methyl-1H-benzo[d]imidazol-2-yl)amino)methyl)benzoate **(4)** (165mg, 0.40mmol) followed by KOH (240mg, 4.0mmol) solubilized in MeOH (5mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to provide 4-(((5-fluoropyridin-2-yl)(1-methyl-1H-benzo[d]imidazol-2-yl)amino)methyl)-N-hydroxybenzamide, **Example N,** as a light orange solid (20mg, 12%).
¹H NMR (400 MHz, DMSO-*d*₆), δ_{H} ppm: 8.19 (d, *J*=2.9 Hz, 1H), 7.66 (d, *J*=8.2 Hz, 1H), 7.55-7.63 (m, 3H), 7.42-7.54 (m, 3H), 7.15-7.27 (m, 2H), 6.74 (dd, *J*=9.2, 3.4 Hz, 1H), 5.22-5.31 (m, 2H), 3.42 (s, 3H).
LCMS (ES): Found 392.25 [M+H]⁺.

### Example O

### 4-(((5-Fluoropyridin-2-yl)(1-methyl-1H-pyrazol-3-yl)amino)methyl)-N-hydroxybenzamide

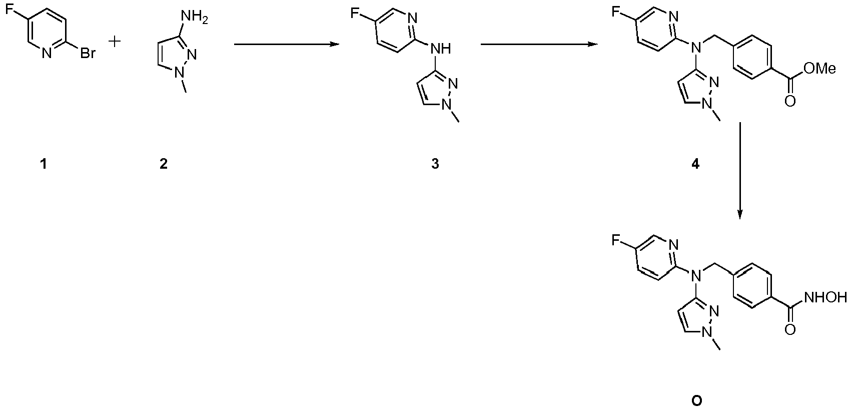

2-Bromo-5-fluoropyridine **(1)** (1.0g, 5.71mmol), 1-methyl-1H-pyrazol-3-amine **(2)** (554mg, 5.71mmol), Xantphos (0.330g, 0.57mmol), and Cs₂CO₃ (2.79g, 8.56mmol) were combined in dry 1,4-dioxane (15mL). The reaction mixture was degassed with N₂(g) and placed under vacuum for 10min. Pd₂(dba)₃ (0.261g, 0.28mmol) was then added and the resulting reaction mixture was heated at 90°C for 30h. It was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide 5-fluoro-N-(1-methyl-1H-pyrazol-3-yl)pyridin-2-amine **(3)** as a yellow solid (0.65g, 61%).
LCMS (ES): Found 193.0 [M+H]⁺.

NaH (60%) (50mg, 1.25mmol) was added portion-wise to 5-fluoro-N-(1-methyl-1H-pyrazol-3-yl)pyridin-2-amine **(3)** (230mg, 1.19mmol) in DMF (10mL) at 5°C under Ar(g). The reaction mixture was stirred for 20min, then methyl 4-(bromomethyl) benzoate (356mg, 1.55mmol) was added, and stirring was continued at 70°C under Ar(g) for 1h. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (3:7) to furnish methyl 4-(((5-fluoropyridin-2-yl)(1-methyl-1H-pyrazol-3-yl)amino)methyl)benzoate **(4)** as a light yellow solid (312mg, 76%).
LCMS (ES): Found 341.1 [M+H]⁺.

A fresh solution of NH₂OH in MeOH was prepared: [KOH (2.57g, 45.8mmol) in MeOH (15mL) was added to NH₂OH.HCl (3.18g, 45.8mmol) in MeOH (15mL) at 0°C]. The reaction mixture was stirred for 20min at 0°C, then filtered to remove salts; it was then added to methyl methyl 4-(((5-fluoropyridin-2-yl)(1-methyl-1H-pyrazol-3-yl)amino)methyl)benzoate **(4)** (312mg, 0.91mmol) followed by KOH (512mg, 9.1mmol) solubilized in MeOH (5mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to provide 4-(((5-fluoropyridin-2-yl)(1-methyl-1H-pyrazol-3-yl)amino)methyl)-N-hydroxybenzamide, **Example O,** as a cream solid (65mg, 20%).
¹H NMR (400 MHz, DMSO-*d*₆), δ_{H} ppm: 11.11 (br. s, 1H), 8.96 (br. s, 1H), 8.10 (d, *J*=3.1 Hz, 1H), 7.59-7.66 (m, 3H), 7.51 (ddd, *J*=9.3, 8.2, 3.1 Hz, 1H), 7.31 (d, *J*=8.1 Hz, 2H), 7.19 (dd, *J*=9.4, 3.7 Hz, 1H), 6.13 (d, *J*=2.3 Hz, 1H), 5.21 (s, 2H), 3.76 (s, 3H).
LCMS (ES): Found 342.1 [M+H]⁺.

### Example P

### 4-((Benzo[d]oxazol-2-yl(5-fluoropyridin-2-yl)amino)methyl)-N-hydroxybenzamide

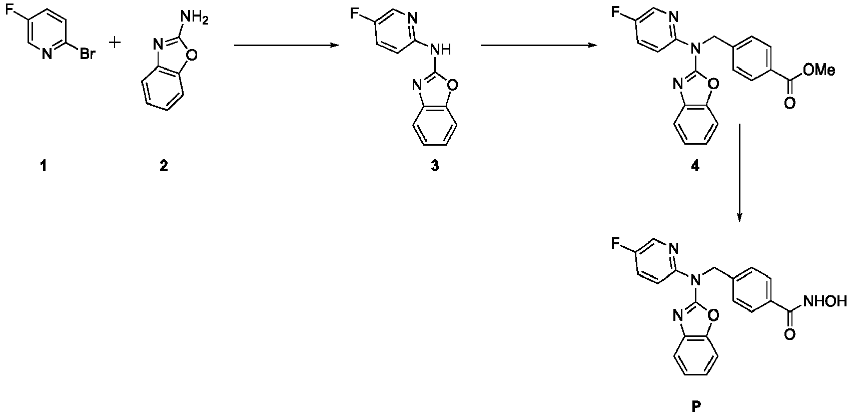

2-Bromo-5-fluoropyridine **(1)** (1.0g, 5.71mmol), benzo[d]oxazol-2-amine **(2)** (766mg, 5.71mmol), Xantphos (0.33g, 0.57mmol), and Cs₂CO₃ (2.79g, 8.56mmol) were combined in dry 1,4-dioxane (15mL). The reaction mixture was degassed with N₂(g) and placed under vacuum for 10min. Pd₂(dba)₃ (0.261g, 0.28mmol) was then added and the resulting reaction mixture was heated at 90°C for 30h. It was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide N-(5-fluoropyridin-2-yl)benzo[d]oxazol-2-amine **(3)** as a yellow solid (0.6g, 46%).
LCMS (ES): Found 230.1 [M+H]⁺.

NaH (60%) (36mg, 0.91 mmol) was added portion-wise to N-(5-fluoropyridin-2-yl)benzo[d]oxazol-2-amine **(3)** (200mg, 0.87mmol) in DMF (8mL) at 5°C under Ar(g). The reaction mixture was stirred for 20min, then methyl 4-(bromomethyl) benzoate (259mg, 1.13mmol) was added, and stirring was continued at 70°C under Ar(g) for 1h. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (3:7) to furnish methyl 4-((benzo[d]oxazol-2-yl(5-fluoropyridin-2-yl)amino)methyl)benzoate **(4)** as a light yellow solid (144mg, 43%).
LCMS (ES): Found 378.1 [M+H]⁺.

A fresh solution of NH₂OH in MeOH was prepared: [KOH (1.07g, 19.0mmol) in MeOH (15mL) was added to NH₂OH.HCl (1.33g, 19.0mmol) in MeOH (15mL) at 0°C]. The reaction mixture was stirred for 20min at 0°C, then filtered to remove salts; it was then added to methyl 4-((benzo[d]oxazol-2-yl(5-fluoropyridin-2-yl)amino)methyl)benzoate **(4)** (144mg, 0.38mmol) followed by KOH (214mg, 3.8mmol) solubilized in MeOH (5mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to provide 4-((benzo[d]oxazol-2-yl(5-fluoropyridin-2-yl)amino)methyl)-N-hydroxybenzamide, **Example P,** as an orange solid (30mg, 20%).
¹H NMR (400 MHz, DMSO-*d*₆), δ_{H} ppm: 11.13 (br. s, 1H), 9.01 (br. s., 1H), 8.41 (d, *J*=3.1 Hz, 1H), 8.25 (dd, *J*=9.2, 3.8 Hz, 1H), 7.89 (ddd, *J*=9.2, 8.1, 3.1 Hz, 1H), 7.66 (d, *J*=8.3 Hz, 2H), 7.47-7.54 (m, 2H), 7.41 (d, *J*=8.2 Hz, 2H), 7.26 (td, *J*=7.7, 1.1 Hz, 1H), 7.13-7.20 (m, 1H), 5.54 (s, 2H).
LCMS (ES): Found 379.1 [M+H]⁺.

### Example Q

### 4-(((4-(4-Fluorophenyl)pyridin-2-yl)(1-methyl-1H-pyrazol-3-yl)amino)methyl)-N-hydroxybenzamide

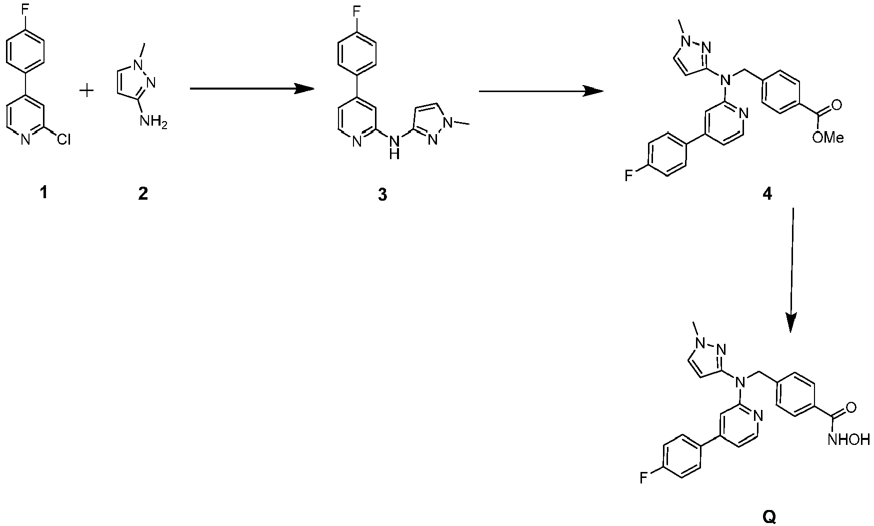

2-Chloro-4-(4-fluorophenyl)pyridine **(1)** (1.0g, 4.8mmol), 1-methyl-1H-pyrazol-3-amine **(2)** (470mg, 4.8mmol), Xantphos (0.28g, 0.48mmol), and Cs₂CO₃ (2.35g, 7.24mmol) were combined in dry 1,4-dioxane (15mL). The reaction mixture was degassed with N₂(g) and placed under vacuum for 10min. Pd₂(dba)₃ (0.22g, 0.24mmol) was then added and the resulting reaction mixture was heated at 90°C for 30h. It was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide 4-(4-fluorophenyl)-N-(1-methyl-1H-pyrazol-3-yl)pyridin-2-amine **(3)** as a yellow solid (1.0g, 71%).
LCMS (ES): Found 269.1 [M+H]⁺.

NaH (60%) (37mg, 0.93mmol) was added portion-wise to 4-(4-fluorophenyl)-N-(1-methyl-1H-pyrazol-3-yl)pyridin-2-amine **(3)** (250mg, 0.93mmol) in DMF (10mL) at 5°C under Ar(g). The reaction mixture was stirred for 20min, then methyl 4-(bromomethyl) benzoate (277mg, 1.2mmol) was added, and stirring was continued at 70°C under Ar(g) for 1h in the dark. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (3:7) to furnish methyl 4-(((4-(4-fluorophenyl)pyridin-2-yl)(1-methyl-1H-pyrazol-3-yl)amino)methyl)benzoate **(4)** as a light yellow solid (267mg, 68%).
LCMS (ES): Found 417.4 [M+H]⁺.

A fresh solution of NH₂OH in MeOH was prepared: [KOH (1.79g, 32.0mmol) in MeOH (15mL) was added to NH₂OH.HCl (2.23g, 32.0mmol) in MeOH (15mL) at 0°C]. The reaction mixture was stirred for 20min at 0°C, then filtered to remove salts; it was then added to methyl 4-(((4-(4-fluorophenyl)pyridin-2-yl)(1-methyl-1H-pyrazol-3-yl)amino)methyl)benzoate **(4)** (267mg, 0.64mmol) followed by KOH (359mg, 6.41mmol) solubilized in MeOH (10mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to 4-(((4-(4-fluorophenyl)pyridin-2-yl)(1-methyl-1H-pyrazol-3-yl)amino)methyl)-N-hydroxybenzamide, **Example Q,** as an off white solid (30mg, 11%).
¹H NMR (400 MHz, DMSO-*d*₆), δ_{H} ppm: 11.11 (br. s, 1H), 9.00 (br. s, 1H), 8.19 (d, *J*=5.3 Hz, 1H), 7.59-7.71 (m, 5H), 7.24-7.39 (m, 5H), 6.98-7.05 (m, 1H), 6.26 (d, *J*=2.2 Hz, 1H), 5.30 (s, 2H), 3.74-3.79 (m, 3H).
LCMS (ES): Found 418.2 [M+H]⁺.

### Example R

### 4-(((5-Fluoropyridin-2-yl)(3-methyl-1,2,4-thiadiazol-5-yl)amino)methyl)-N-hydroxybenzamide

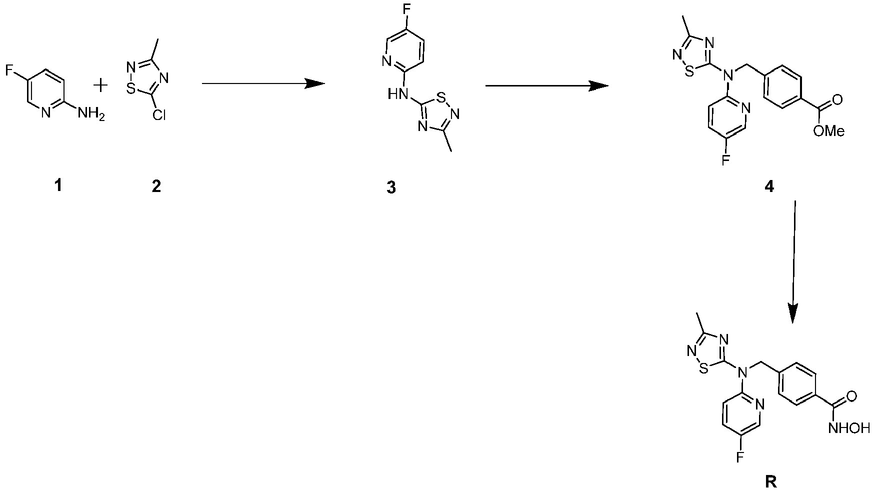

5-Fluoropyridin-2-amine **(1)** (1.0g, 8.9mmol), 5-chloro-3-methyl-1, 2, 4-thiadiazole **(2)** (1.19g, 8.9mmol), Xantphos (0.52g, 0.89mmol), and Cs₂CO₃ (4.35g, 13.3mmol) were combined in dry 1,4-dioxane (15mL). The reaction mixture was degassed with N₂(g) and placed under vacuum for 10min. Pd₂(dba)₃ (0.41g, 0.44mmol) was then added and the resulting reaction mixture was heated at 90°C for 30h. The reaction mixture was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (3:7) to provide N-(5-fluoropyridin-2-yl)-3-methyl-1, 2, 4-thiadiazol-5-amine **(3)** as a yellow solid (1.2g, 67%).
LCMS (ES): Found 211.1 [M+H]⁺.

NaH (60%) (59mg, 1.49mmol) was added portion-wise to N-(5-fluoropyridin-2-yl)-3-methyl-1,2,4-thiadiazol-5-amine **(3)** (300mg,1.42mmol) in DMF (7mL) at 5°C under Ar(g). The reaction mixture was stirred for 20min, then methyl 4-(bromomethyl) benzoate (425mg, 1.85mmol) was added, and stirring was continued at 70°C under Ar(g) for 1h in the dark. The reaction mixture was then poured onto water (100mL), and extracted with EtOAc (3 x 50mL). The organic phases were combined, dried over Na₂SO₄, filtered and subsequently concentrated *in vacuo.* The residue was purified by flash chromatography with EtOAc/Hexane (3:7) to furnish methyl-4-(((5-fluoropyridin-2-yl)(3-methyl-1,2,4-thiadiazol-5-yl)amino)methyl)benzoate **(4)** as a yellow solid (480mg, 90%).
LCMS (ES): Found 359.3 [M+H]⁺.

A fresh solution of NH₂OH in MeOH was prepared: [KOH (4.63g, 67.0mmol) in MeOH (20mL) was added to NH₂OH.HCl (3.76g, 67.0mmol) in MeOH (20mL) at 0°C]. The reaction mixture was stirred for 20min at 0°C, then filtered to remove salts; it was then added to methyl 4-(((5-fluoropyridin-2-yl)(3-methyl-1,2,4-thiadiazol-5-yl)amino)methyl)benzoate **(4)** (480mg, 1.3mmol) followed by KOH (750mg, 1.3mmol) solubilized in MeOH (10mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to provide 4-(((5-fluoropyridin-2-yl)(3-methyl-1,2,4-thiadiazol-5-yl)amino)methyl)-N-hydroxybenzamide, **Example R,** as an orange solid (90mg, 19%).
¹H NMR (400 MHz, DMSO-*d*₆), δ_{H} ppm: 11.16 (br. s., 1H), 9.03 (br. s., 1H), 8.60 (d, *J*=2.9 Hz, 1H), 7.86 (td, *J*=8.7, 2.8 Hz, 1H), 7.64-7.76 (m, 2H), 7.19-7.34 (m, 3H), 5.77 (s, 2H), 2.39 (s, 3H).
LCMS (ES): Found 359.8 [M+H]⁺.

### Example S

### 4-(((4-(4-Fluorophenyl)pyridin-2-yl)(3-methyl-1,2,4-thiadiazol-5-yl)amino)methyl)-N-hydroxybenzamide

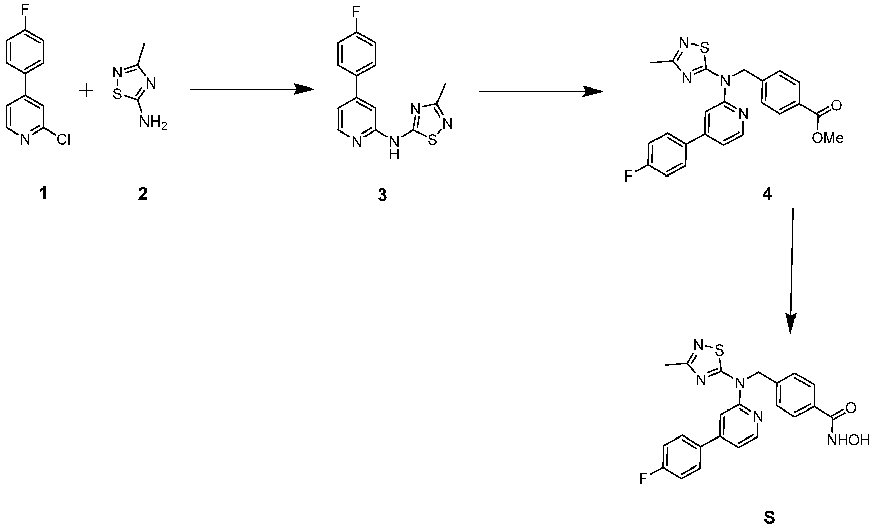

2-Chloro-4-(4-fluorophenyl)pyridine **(1)** (1.0g, 4.8mmol), 3-methyl-1, 2, 4-thiadiazol-5-amine **(2)** (0.56g, 4.8mmol), Xantphos (0.279g, 0.48mmol), and Cs₂CO₃ (2.35g, 7.24mmol) were combined in dry 1,4-dioxane (15mL). The reaction mixture was degassed with N₂(g) and placed under vacuum for 10min. Pd₂(dba)₃ (0.22g, 0.24mmol) was then added and the resulting reaction mixture was heated at 90°C for 30h. It was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide N-(4-(4-fluorophenyl)pyridin-2-yl)-3-methyl-1, 2, 4-thiadiazol-5-amine **(3)** as a yellow solid (1.1g, 80%).
LCMS (ES): Found 287.1 [M+H]⁺.

NaH (60%) (42mg, 1.05mmol) was added portion-wise to N-(4-(4-fluorophenyl)pyridin-2-yl)-3-methyl-1,2,4-thiadiazol-5-amine **(3)** (300mg,1.05mmol) in DMF (10mL) at 5°C under Ar(g). The reaction mixture was stirred for 20min, then methyl 4-(bromomethyl)benzoate (312mg, 1.36mmol) was added, and stirring was continued at 70°C under Ar(g) for 1h. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (3:7) to furnish methyl 4-(((4-(4-fluorophenyl)pyridin-2-yl)(3-methyl-1,2,4-thiadiazol-5-yl)amino)methyl)benzoate **(4)** as a yellow solid (325mg, 74%).
LCMS (ES): Found 421.1 [M+H]⁺.

A fresh solution of NH₂OH in MeOH was prepared: [KOH (1.96g, 35mmol) in MeOH (10mL) was added to NH₂OH.HCl (2.43g, 35mmol) in MeOH (10mL) at 0°C]. The reaction mixture was stirred for 20min at 0°C, then filtered to remove salts; it was then added to methyl 4-(((4-(4-fluorophenyl)pyridin-2-yl)(3-methyl-1,2,4-thiadiazol-5-yl)amino)methyl)benzoate **(4)** (319mg, 0.69mmol) followed by KOH (392mg, 7.0mmol) solubilized in MeOH (10mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to 4-(((4-(4-fluorophenyl)pyridin-2-yl)(3-methyl-1,2,4-thiadiazol-5-yl)amino)methyl)-N-hydroxybenzamide, **Example S,** as an off white solid (58mg, 19%).
¹H NMR (400 MHz, DMSO-*d*₆), δ_{H} ppm: 11.13 (br. s., 1H), 9.02 (br. s., 1H), 8.59 (d, *J*=5.3 Hz, 1H), 7.82 (dd, *J*=8.7, 5.3 Hz, 2H), 7.67 (d, *J*=8.2 Hz, 2H), 7.43-7.51 (m, 2H), 7.27-7.40 (m, 4H), 5.92 (s, 2H), 2.40 (s, 3H).
LCMS (ES): Found 436.4 [M+H]⁺.

### Example T

### 4-(((5-Fluoropyridin-2-yl)(3-(trifluoromethyl)-1,2,4-thiadiazol-5-yl)amino)methyl)-N-hydroxybenzamide

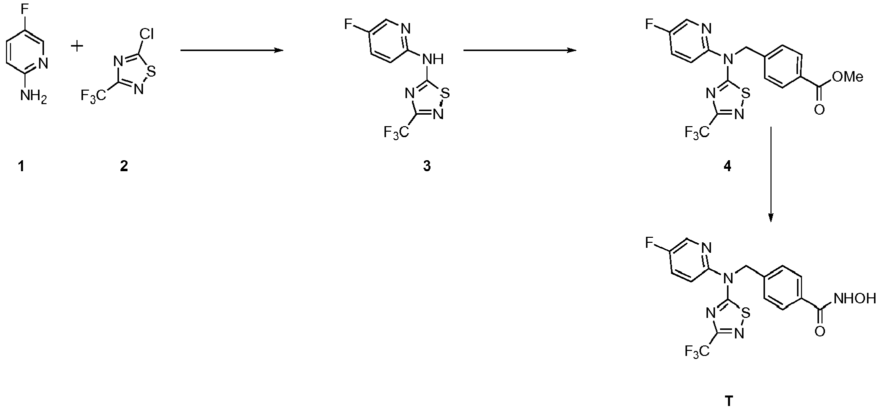

5-Fluoropyridin-2-amine **(1)** (1.0g, 8.9mmol), 5-chloro-3-(trifluoromethyl)-1, 2, 4-thiadiazole **(2)** (1.68g, 8.9mmol), Xantphos (0.52g, 0.89mmol), and Cs₂CO₃ (4.35g, 13.3mmol) were combined in dry 1,4-dioxane (15mL). The reaction mixture was degassed with N₂(g) and placed under vacuum for 10min. Pd₂(dba)₃ (0.41g, 0.44mmol) was then added and the resulting reaction mixture was heated at 90°C for 30h. It was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (3:7) to provide N-(5-fluoropyridin-2-yl)-3-(trifluoromethyl)-1, 2, 4-thiadiazol-5-amine **(3)** as a yellow solid (900mg, 38%).
LCMS (ES): Found 265.1 [M+H]⁺.

NaH (60%) (61mg, 1.51mmol) was added portion-wise to N-(5-fluoropyridin-2-yl)-3-(trifluoromethyl)-1,2,4-thiadiazol-5-amine **(3)** (400mg,1.51mmol) in DMF (10mL) at 5°C under Ar(g). The reaction mixture was stirred for 20min, then methyl 4-(bromomethyl) benzoate (451mg, 1.85mmol) was added, and stirring was continued at 70°C under Ar(g) for 1h in the dark. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (3:7) to furnish methyl 4-(((5-fluoropyridin-2-yl)(3-(trifluoromethyl)-1,2,4-thiadiazol-5-yl)amino)methyl)benzoate **(3)** as a yellow solid (535mg, 82%).
LCMS (ES): Found 413.3 [M+H]⁺.

A fresh solution of NH₂OH in MeOH was prepared: [KOH (3.63g, 64.0mmol) in MeOH (20mL) was added to NH₂OH.HCl (4.47g, 64.0mmol) in MeOH (20mL) at 0°C]. The reaction mixture was stirred for 20min at 0°C, then filtered to remove salts; it was then added to methyl 4-(((5-fluoropyridin-2-yl)(3-(trifluoromethyl)-1,2,4-thiadiazol-5-yl)amino)methyl)benzoate **(3)** (535mg, 1.2mmol) followed by KOH (720mg, 13.0mmol) solubilized in MeOH (10mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to provide 4-(((5-fluoropyridin-2-yl)(3-(trifluoromethyl)-1,2,4-thiadiazol-5-yl)amino)methyl)-N-hydroxybenzamide, **Example** T, as an orange solid (90mg, 17%).
¹H NMR (400 MHz, DMSO-*d*₆), δ_{H} ppm: 11.18 (br. s., 1H), 9.06 (br. s., 1H), 8.73 (d, *J*=2.7 Hz, 1H), 7.97 (td, *J*=8.6, 2.6 Hz, 1H), 7.69 (d, *J*=8.2 Hz, 2H), 7.46 (dd, *J*=9.0, 2.8 Hz, 1H), 7.31 (d, *J*=7.8 Hz, 2H), 5.80 (br. s., 2H), 5.72-5.87 (m, 1H).
LCMS (ES): Found 414.3 [M+H]⁺.

### Example U

### 4-(((4-(4-Fluorophenyl)pyridin-2-yl)(pyrazin-2-yl)amino)methyl)-N-hydroxybenzamide

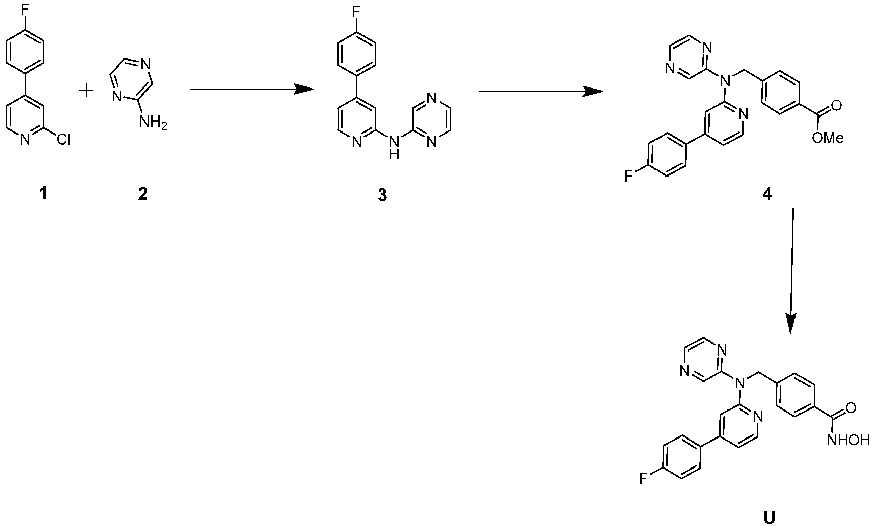

NaH (60%) (47mg, 1.19mmol) was added portion-wise to N-(4-(4-fluorophenyl)pyridin-2-yl)pyrazin-2-amine **(3)** (prepared using conditions as per Examples above) (300mg,1.13mmol) in DMF (10mL) at 5°C under Ar(g). The reaction mixture was stirred for 20min, then methyl 4-(bromomethyl)benzoate (337mg, 1.47mmol) was added, and stirring was continued at 70°C under Ar(g) for 1h. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (3:7) to furnish methyl 4-(((4-(4-fluorophenyl)pyridin-2-yl)(pyrazin-2-yl)amino)methyl)benzoate **(4)** as a yellow solid (220mg, 46%).
LCMS (ES): Found 414.4 [M+H]⁺.

A fresh solution of NH₂OH in MeOH was prepared: [KOH (1.49g, 26.9mmol) in MeOH (10mL) was added to NH₂OH.HCl (1.86g, 26.9mmol) in MeOH (10mL) at 0°C]. The reaction mixture was stirred for 20min at 0°C, then filtered to remove salts; it was then added to methyl 4-(((4-(4-fluorophenyl)pyridin-2-yl)(pyrazin-2-yl)amino)methyl)benzoate **(4)** (220mg,0.53mmol) followed by KOH (298mg, 5.3mmol) solubilized in MeOH (10mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to 4-(((4-(4-fluorophenyl)pyridin-2-yl)(pyrazin-2-yl)amino)methyl)-N-hydroxybenzamide,
**Example U,** as an off white solid (35mg, 16%).
¹H NMR (400 MHz, DMSO-*d*₆), δ_{H} ppm: 11.10 (br. s., 1H), 8.99 (br. s., 1H), 8.69 (d, *J*=1.4 Hz, 1H), 8.36 (d, *J*=5.3 Hz, 1H), 8.28 (dd, *J*=2.7, 1.5 Hz, 1H), 8.11 (d, *J*=2.7 Hz, 1H), 7.76-7.86 (m, 2H), 7.64 (d, *J*=8.4 Hz, 2H), 7.42 (d, *J*=8.2 Hz, 2H), 7.38 (dd, *J*=5.3, 1.4 Hz, 1H), 7.34 (t, *J*=8.9 Hz, 2H), 5.53 (s, 2H).
LCMS (ES): Found 416.1 [M+H]⁺.

### Example V

### 4-((Benzo[d]thiazol-2-yl(pyridin-2-yl)amino)methyl)-N-hydroxybenzamide

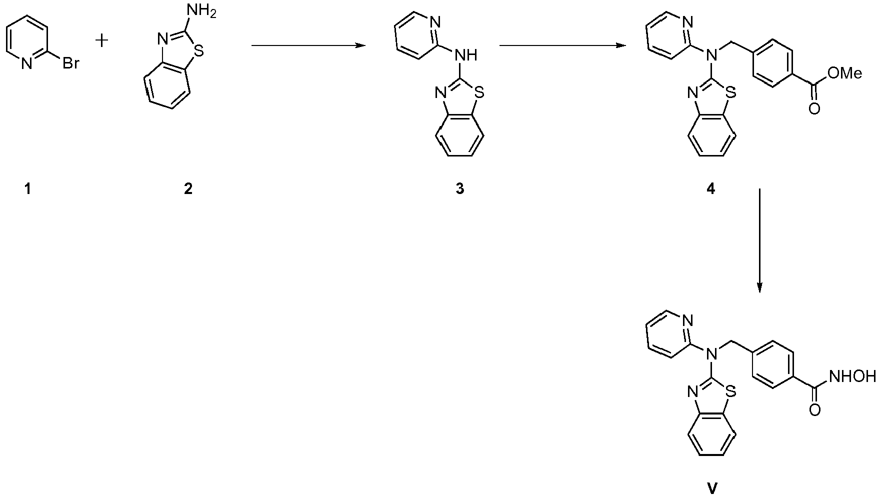

NaH (60%) (75mg, 1.8mmol) was added portion-wise to N-(pyridin-2-yl)benzo[d]thiazol-2-amine **(3)** (prepared using conditions as per Examples above) (430mg, 1.8mmol) in DMF (10mL) at 5°C under Ar(g). The reaction mixture was stirred for 20min, then methyl 4-(bromomethyl) benzoate (563mg, 2.4mmol) was added, and stirring was continued at 70°C under Ar(g) for 1h. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (3:7) to furnish methyl 4-((benzo[d]thiazol-2-yl(pyridin-2-yl)amino)methyl)benzoate **(4)** as a yellow solid (300mg, 42%).
LCMS (ES): Found 376.1 [M+H]⁺.

A fresh solution of NH₂OH in MeOH was prepared: [KOH (2.24g, 40.0mmol) in MeOH (15mL) was added to NH₂OH.HCl (2.78g, 40.0mmol) in MeOH (15mL) at 0°C]. The reaction mixture was stirred for 20min at 0°C, then filtered to remove salts; it was then added to methyl 4-((benzo[d]thiazol-2-yl(pyridin-2-yl)amino)methyl)benzoate **(4)** (300mg, 0.8mmol) followed by KOH (449mg, 8.0mmol) solubilized in MeOH (5mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to provide 4-((benzo[d]thiazol-2-yl(pyridin-2-yl)amino)methyl)-N-hydroxybenzamide, **Example V,** as a light orange solid (60mg, 20%).
¹H NMR (400 MHz, DMSO-*d*₆), δ_{H} ppm: 11.15 (br. s, 1H), 8.99 (br. s, 1H), 8.50 (dd, *J*=4.8, 1.4 Hz, 1H), 7.93 (d, *J*=7.6 Hz, 1H), 7.78-7.86 (m, 1H), 7.68 (d, *J*=8.2 Hz, 2H), 7.64 (d, *J*=7.9 Hz, 1H), 7.33-7.39 (m, 1H), 7.21-7.31 (m, 3H), 7.11-7.20 (m, 2H), 5.82 (s, 2H).
LCMS (ES): Found 377.1 [M+H]⁺.

### Example W

### N-Hydroxy-4-((pyridin-2-yl(3-(trifluoromethyl)-1,2,4-thiadiazol-5-yl)amino)methyl)benzamide

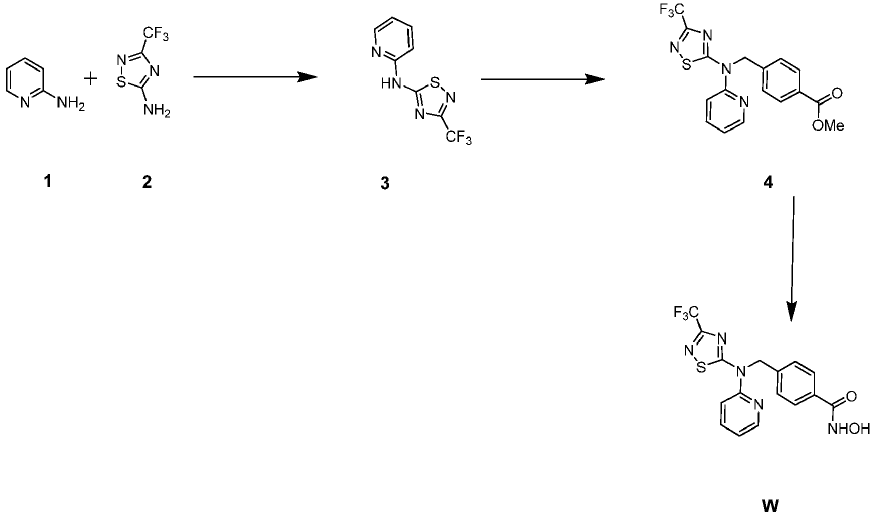

Pyridin-2-amine **(1)** (1.0g, 10.6mmol), 5-chloro-3-(trifluoromethyl)-1,2,4-thiadiazole **(2)** (1.82g, 10.6mmol), Xantphos (0.61g, 1.06mmol), and Cs₂CO₃ (5.18g, 15.9mmol) were combined in dry 1,4-dioxane (15mL). The reaction mixture was degassed with N₂(g) and placed under vacuum for 10min. Pd₂(dba)₃ (0.49g, 0.53mmol) was then added and the resulting reaction mixture was heated at 90°C for 30h. It was then poured onto demineralized water (200mL), and extracted with EtOAc (3 x 100mL). The organic phases were combined, dried over Na₂SO₄, filtered and subsequently concentrated *in vacuo.* The resulting residue was purified by flash chromatography with EtOAc/Hexane (1:1) to provide N-(pyridin-2-yl)-3-(trifluoromethyl)-1,2,4-thiadiazol-5-amine **(3)** as a yellow solid (1.4g, 57%).
LCMS (ES): Found 247.2 [M+H]⁺.

NaH (60%) (49mg, 1.21mmol) was added portion-wise to N-(pyridin-2-yl)-3-(trifluoromethyl)-1,2,4-thiadiazol-5-amine **(3)** (300mg,1.21mmol) in DMF (10mL) at 5°C under Ar(g). The reaction mixture was stirred for 20min, then methyl 4-(bromomethyl) benzoate (363mg, 1.58mmol) was added, and stirring was continued at 70°C under Ar(g) for 1h in the dark. The reaction mixture was then poured onto demineralized water (100mL), and extracted with EtOAc (3 x 50mL). The organic phases were combined, dried over Na₂SO₄, filtered and subsequently concentrated *in vacuo.* The resulting residue was purified by flash chromatography with EtOAc/Hexane (3:7) to furnish methyl 4-((pyridin-2-yl(3-(trifluoromethyl)-1,2,4-thiadiazol-5-yl)amino)methyl)benzoate **(4)** as a yellow solid (450mg, 90%).
LCMS (ES): Found 395.3 [M+H]⁺.

A fresh solution of NH₂OH in MeOH was prepared: [KOH (3.56g, 63.4mmol) in MeOH (20mL) was added to NH₂OH.HCl (4.41g, 63.4mmol) in MeOH (20mL) at 0°C]. The reaction mixture was stirred for 20min at 0°C, then filtered to remove salts; it was then added to methyl 4-((pyridin-2-yl(3-(trifluoromethyl)-1,2,4-thiadiazol-5-yl)amino)methyl)benzoate **(4)** (500mg, 1.2mmol) followed by KOH (712mg, 12.6mmol) solubilized in MeOH (10mL). The reaction mixture was stirred at rt for 21h, and then concentrated *in vacuo,* poured onto brine/H₂O (30mL/70mL), and extracted with CH₂Cl₂ (3 x 100mL). The organic phases were combined, dried over Na₂SO₄, filtered and subsequently concentrated *in vacuo.* The resulting residue was purified by flash chromatography with MeOH/CH₂Cl₂ (1:9) to provide N-hydroxy-4-((pyridin-2-yl(3-(trifluoromethyl)-1,2,4-thiadiazol-5-yl)amino)methyl)benzamide, **Example W,** as an off white solid (20mg, 4%).
¹H NMR (400 MHz, DMSO-*d*₆), δ_{H} ppm: 11.15 (br. s., 1H), 9.03 (br. s., 1H), 8.63-8.68 (m, *J*=5.0, 0.9 Hz, 1H), 7.97 (ddd, *J*=8.7, 7.2, 1.8 Hz, 1H), 7.69 (d, *J*=8.4 Hz, 2H), 7.41 (d, *J*=8.6 Hz, 1H), 7.32 (d, J=8.3 Hz, 2H), 7.28 (dd, *J*=7.0, 5.3 Hz, 1H), 5.80 (s, 2H).
LCMS (ES): Found 396.3 [M+H]⁺.

### Example X

### N-Hydroxy-4-(((3-methoxypyridin-2-yl)-(5-methylpyridin-2-yl)amino)methyl)benzamide

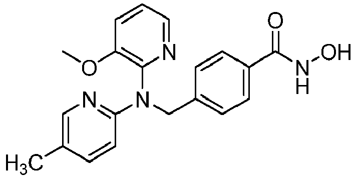

¹H NMR (400 MHz, Methanol-*d*₄), δ_{H} ppm: 7.97 (d, *J*=4.9 Hz, 1H), 7.89 (d, *J*=2.3 Hz, 1H), 7.61 (d, *J*=7.8 Hz, 2H), 7.46 (t, *J*=7.5 Hz, 3H), 7.33 (dd, *J*=8.5, 2.4 Hz, 1H), 7.22 (dd, *J*=8.2, 4.8 Hz, 1H), 6.41 (d, *J*=8.5 Hz, 1H), 5.31 (s, 2H), 3.73 (s, 3H), 2.20 (s, 3H).
LCMS (ES): Found 365.0 [M+H]⁺.

### Example Y

### N-Hydroxy-4-(((5-methoxypyridin-2-yl)(5-methylpyridin-2-yl)amino)methyl)benzamide

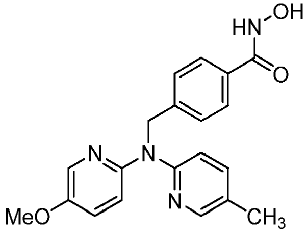

¹H NMR (400 MHz, Methanol-*d*₄), δ_{H} ppm: 7.99 (dd, *J*=4.8, 2.6 Hz, 2H), 7.62 (d, *J*=8.0 Hz, 2H), 7.41 (dd, *J*=8.2, 4.9 Hz, 3H), 7.31 (dd, *J*=9.1, 3.1 Hz, 1H), 7.14 (d, *J*=8.9 Hz, 1H), 6.84 (d, *J*=8.5 Hz, 1H), 5.36 (s, 2H), 3.83 (s, 3H), 2.22 (s, 3H). LCMS (ES): Found 365.0 [M+H]⁺.

### Example Z

### N-Hydroxy-4-(((3-methoxypyridin-2-yl)(5-morpholinopyridin-2-yl)amino)methyl)benzamide

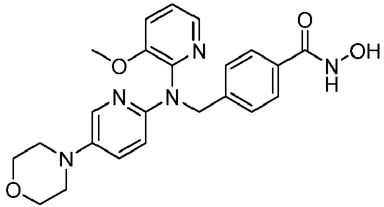

¹H NMR (400 MHz, Methanol-*d*₄), δ_{H} ppm: 7.94 (dd, *J*=4.8, 1.5 Hz, 1H), 7.78 (d, *J*=3.0 Hz, 1H), 7.61 (d, *J*=8.3 Hz, 2H), 7.38-7.51 (m, 3H), 7.27 (dd, *J*=9.0, 3.1 Hz, 1H), 7.17 (dd, *J*=8.1, 4.8 Hz, 1H), 6.51 (d, *J*=9.0 Hz, 1H), 5.31 (s, 2H), 3.77-3.89 (m, 4H), 3.72 (s, 3H), 2.97-3.08 (m, 4H).
LCMS (ES): Found 436.0 [M+H]⁺.

### Example AA

### N-Hydroxy-4-(((5-methoxypyridin-2-yl)(5-morpholinopyridin-2-yl)amino)methyl)benzamide

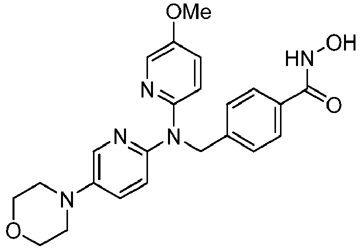

¹H NMR (400 MHz, Methanol-*d*₄), δ_{H} ppm: 7.88-7.95 (m, 2H), 7.58-7.66 (m, 2H), 7.42 (d, *J*=8.0 Hz, 2H), 7.33 (dd, *J*=9.0, 3.1 Hz, 1H), 7.26 (dd, *J*=9.1, 3.1 Hz, 1H), 6.99 (dd, *J*=9.0, 4.5 Hz, 2H), 5.34 (s, 2H), 3.71-3.94 (m, 7H), 3.04-3.15 (m, 4H).
LCMS (ES): Found 436.0 [M+H]⁺.

### Example BB

### N-Hydroxy-4-((pyridin-2-yl(thieno[3,2-c]pyridin-4-yl)amino)methyl)benzamide

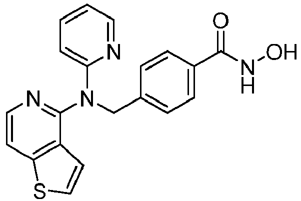

¹H NMR (400 MHz, Methanol-*d*₄), δ_{H} ppm: 7.97-8.10 (m, 1H), 7.76 (dd, *J* = 9.3, 7.1 Hz, 3H), 7.33-7.69 (m, 5H), 7.14 (d, *J*=5.4 Hz, 1H), 6.98 (d, *J*=9.1 Hz, 1H), 6.64 (t, *J*=6.8 Hz, 1H), 5.56 (s, 2H).
LCMS (ES): Found 377.0 [M+H]⁺.

### Example CC

### N-Hydroxy-4-(((6-methylpyridin-2-yl)(5-morpholinopyridin-2-yl)amino)methyl)benzamide

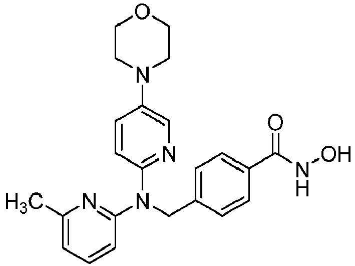

¹H NMR (400 MHz, Methanol-*d*₄), δ_{H} ppm: 7.99 (d, *J*=3.0 Hz, 1H), 7.62 (d, *J*=7.8 Hz, 2H), 7.42 (d, *J*=8.1 Hz, 2H), 7.34-7.39 (m, 2H), 7.14 (d, *J*=8.9 Hz, 1H), 6.64 (dd, *J*=8.1, 7.8 Hz, 2H), 5.39 (s, 2H), 3.79-3.86 (m, 4H), 3.14 (dd, *J*=6.1, 3.6 Hz, 4H), 2.37 (s, 3H).
LCMS (ES): Found 420.0 [M+H]⁺.

### Example DD

### N-Hydroxy-4-{[(pyrazin-2-yl)(pyrimidin-4-yl)amino]methyl}benzamide

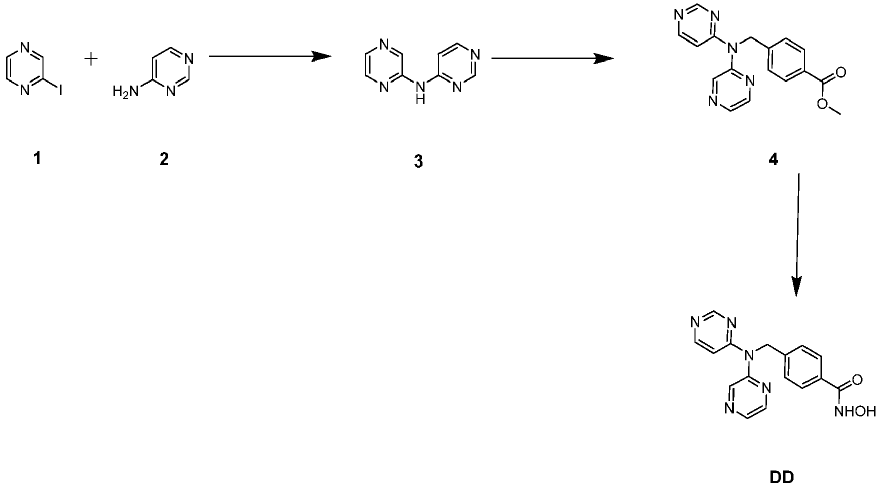

A solution of 2-iodopyrazine **(1)** (1.2g, 5.83mmol), pyrimidin-4-amine (2) (609mg, 6.41mmol), Cs₂CO₃ (3.80g, 11.65mmol) and Xantphos (148mg, 0.26mmol) in 1,4-Dioxane (15mL) was purged with N₂(g) for 10 min. Pd₂(dba)₃ (107mg, 0.12 mmol) was added and mixture was heated to 90°C for 3h. Reaction was cooled to rt and partitioned between water (300mL) and EtOAc (3 x 100mL). Combined organics were washed with water (50mL), dried over Na₂SO₄, filtered and concentrated *in vacuo.* The residue was purified by flash column chromatography with CH₂Cl₂/MeOH (1:0-9:1) to yield **(3)** (678mg, 66%).
¹H NMR (500 MHz, Methanol-*d*₄), δ_{H} ppm: 9.06 (d, *J*=1.3 Hz, 1H), 8.74 (s, 1H), 8.42 (d, *J*=6.0 Hz, 1H), 8.34 (dd, *J*=2.6, 1.5 Hz, 1H), 8.19 (d, *J*=2.7 Hz, 1H), 7.72 (dd, *J*=6.0, 1.0 Hz, 1H).
LCMS (ES): Found 174.0 [M+H]⁺.

NaH (60%, 48.5mg, 1.21mmol) was added to a solution of **(3)** (200mg, 1.15mmol) in DMF (7mL) at 5°C under N₂(g). The reaction mixture was stirred for 20min then methyl 4-(bromomethyl)benzoate (344mg, 1.5mmol) was added as a solution in DMF (3mL), the stirring was continued at 70°C for 1h. Reaction cooled to rt and poured onto water (100mL). Brine (25mL) was added and the aqueous was extracted with EtOAc (2 x 100mL). Combined organics were dried over Na₂SO₄, filtered and concentrated *in vacuo.* Purification by flash column chromatography with CH₂Cl₂/EtOAc (1:0-0:1) then EtOAc/MeOH (1:0-4:1) yielded **(4)** (187mg, 50%).
¹H NMR (500 MHz, Chloroform-*d*), δ_{H} ppm: 8.85 (d, *J*=1.4 Hz, 1H), 8.77-8.80 (m, 1H), 8.34-8.38 (m, 2H), 8.29 (d, *J*=2.6 Hz, 1H), 7.95 (d, *J*=8.4 Hz, 2H), 7.36 (d, *J*=8.4 Hz, 2H), 6.91 (dd, *J*=6.0, 1.2 Hz, 1H), 5.49 (s, 2H), 3.87 (s, 3H).
LCMS (ES): Found 322.0 [M+H]⁺.

A solution of **(4)** (0.09mL, 0.58mmol) in 0.85M hydroxylamine in MeOH (10 mL) was stirred at rt for 40h. Solvent was removed *in vacuo* and the residue purified by reverse phase HPLC to give **Example DD** (30mg,15%).
¹H NMR (500 MHz, Methanol-*d*₄), δ_{H} ppm: 8.89 (d, *J*=1.4 Hz, 1H), 8.69 (s, 1H), 8.47 (dd, *J*=2.5, 1.5 Hz, 1H), 8.25-8.37 (m, 2H), 7.68 (d, *J*=8.3 Hz, 2H), 7.38 (d, *J*=8.3 Hz, 2H), 7.08 (dd, *J*=6.2, 1.2 Hz, 1H), 5.51 (s, 2H).
LCMS (ES): Found 323.0 [M+H]⁺.

### Example EE

### N-Hydroxy-4-{[(pyrazin-2-yl)(pyrimidin-4-yl)amino]methyl}benzamide

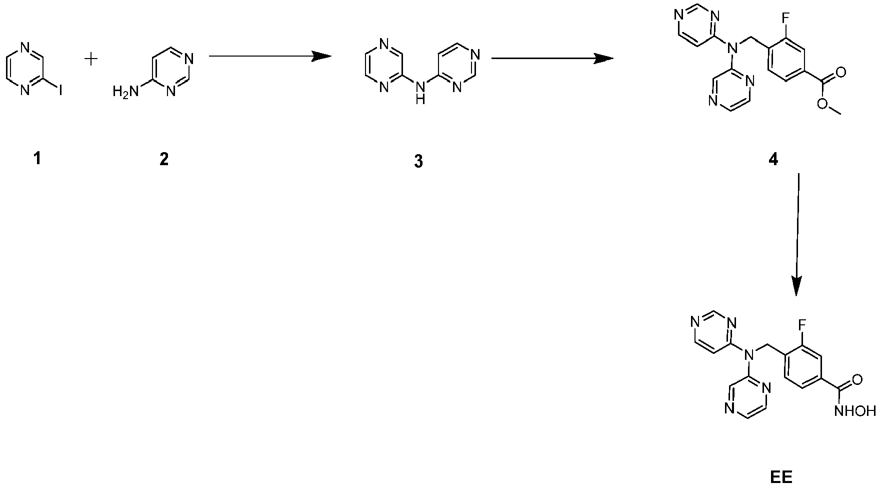

NaH (60%, 48.5mg, 1.21mmol) was added to a solution of **(3)** (200mg, 1.15mmol) in DMF (7mL) at 5°C under N₂(g). The reaction mixture was stirred for 20min then methyl 4-(bromomethyl)-3-fluorobenzoate (371mg, 1.5mmol) was added as a solution in DMF (3mL). The stirring was continued at 70°C for 1h. Reaction cooled to rt and poured onto water (100mL). Brine (25mL) was added and the aqueous was extracted with EtOAc (2 x 100mL). Combined organics were dried over Na₂SO₄, filtered and concentrated *in vacuo.* Purification by flash column chromatography with EtOAc/CH₂Cl₂ (0:1-1:0) then EtOAc/MeOH (1:0-4:1) yielded **(4)** (158mg, 40%).
¹H NMR (500 MHz, Chloroform-*d*), δ_{H} ppm: 8.87 (d, *J*=1.4 Hz, 1H), 8.76-8.78 (m, 1H), 8.36-8.40 (m, 2H), 8.31 (d, *J*=2.6 Hz, 1H), 7.69 (d, *J*=9.2 Hz, 2H), 7.30 (t, *J*=7.6 Hz, 1H), 6.92 (dd, *J*=6.1, 1.2 Hz, 1H), 5.50 (s, 2H), 3.87 (s, 3H).
LCMS (ES): Found 340.0 [M+H]⁺.

A solution of **(4)** (0.08 mL, 0.47 mmol) in 0.85M hydroxylamine in MeOH (10 mL) was stirred at rt for 18 h. Solvent was concentrated to dryness and the residue purified by neutral pH reverse phase HPLC to give **Example EE** (25mg, 15%).

¹H NMR (500 MHz, Methanol-*d*₄), δ_{H} ppm: 8.91 (d, *J*=1.4 Hz, 1H), 8.70 (s, 1H), 8.48 (dd, *J*=2.5, 1.5 Hz, 1H), 8.31-8.38 (m, 2H), 7.43-7.50 (m, 2H), 7.35 (t, *J*=7.9 Hz, 1H), 7.09 (dd, *J*=6.2, 1.2 Hz, 1H), 5.53 (s, 2H).
LCMS (ES): Found 341.0 [M+H]⁺.

### Example FF

### N-Hydroxy-6-{[(pyrazin-2-yl)(pyrimidin-4-yl)amino]methyl}pyridine-3-carboxamide

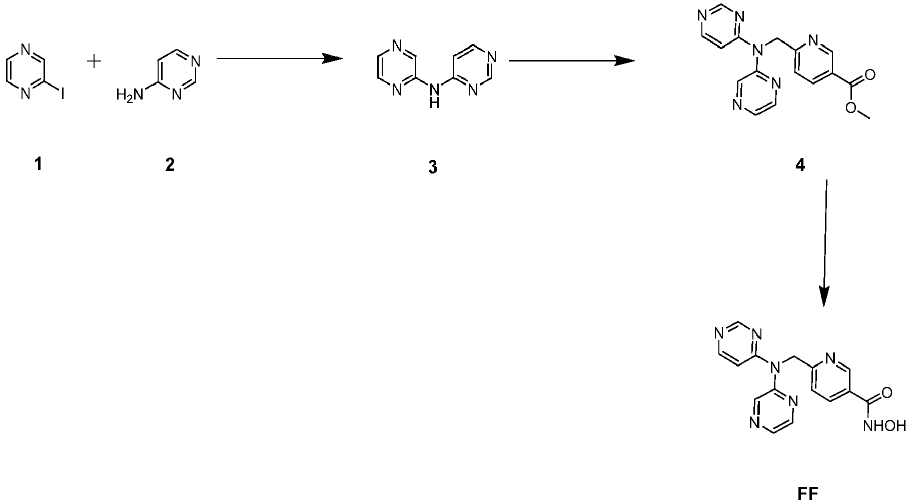

NaH (60%, 48.5 mg, 1.21mmol) was added to a solution of **(3)** (200 mg, 1.15mmol) in DMF (7mL) at 5°C under N₂(g). The reaction mixture was stirred for 20min then methyl 6-(bromomethyl)pyridine-3-carboxylate (345mg, 1.5mmol) was added as a solution in DMF (3mL). The stirring was continued at 70°C for 1h. Reaction cooled to rt and poured onto water (100mL). Brine (25mL) was added and the aqueous was extracted with EtOAc (2 x 100mL). Combined organics were dried over Na₂SO₄, filtered and concentrated *in vacuo.* The residue was purified by flash column chromatography with CH₂Cl₂/EtOAc (1:0-0:1) then CH₂Cl₂/MeOH (1:0-4:1) to yield **(4)** (116 mg, 27%).
¹H NMR (500 MHz, Chloroform-*d*), δ_{H} ppm: 9.11 (d, *J*=1.6 Hz, 1H), 8.97 (d, *J*=1.4 Hz, 1H), 8.70-8.77 (m, 1H), 8.34-8.40 (m, 2H), 8.31 (d, *J*=2.6 Hz, 1H), 8.18 (dd, *J*=8.2, 2.1 Hz, 1H), 7.36 (d, *J*=8.2 Hz, 1H), 7.01 (dd, *J*=6.1, 1.2 Hz, 1H), 5.56 (s, 2H), 3.90 (s, 3H).
LCMS (ES): Found 322.9 [M+H]⁺.

A solution of **(4)** (0.06 mL, 0.31 mmol) in 0.85M hydroxylamine in MeOH (10 mL) was stirred at rt for 18h. The reaction mixture was concentrated to dryness. The residue was purified by reverse phase HPLC to give **Example FF** (25.7 mg, 26%).
¹H NMR (500 MHz, DMSO-*d*₆), δ_{H} ppm: 8.99 (d, *J*=4.9 Hz, 1H), 8.64-8.76 (m, 2H), 8.32-8.51 (m, 3H), 7.82-7.93 (m, 1H), 7.03-7.30 (m, 2H), 5.45 (m, 2H). LCMS (ES): Found 324.1 [M+H]⁺.

### Example GG

### 4-([Bis(pyrazin-2-yl)amino]methyl)-N-hydroxybenzamide

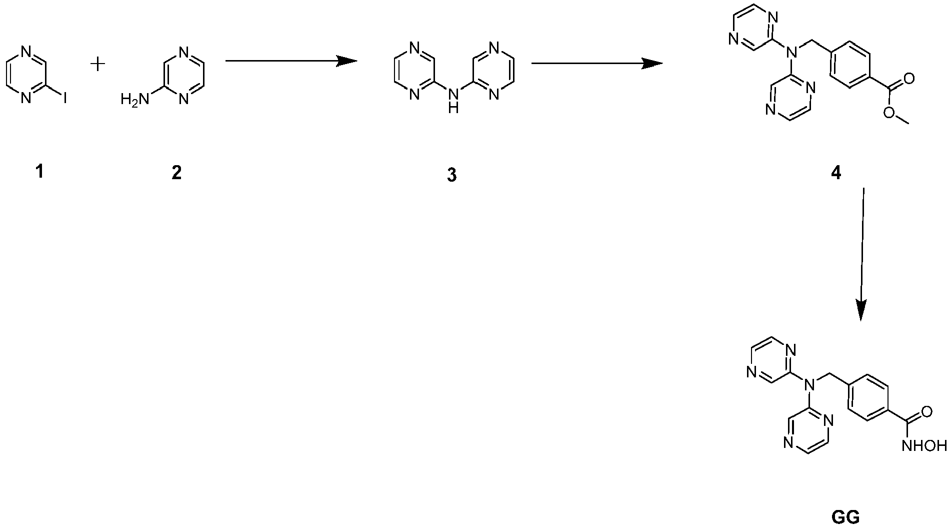

A solution of 2-iodopyrazine **(1)** (1.2g, 5.83mmol), pyrazin-2-amine **(2)** (609mg, 6.4mmol), Cs₂CO₃ (3.80g, 11.7mmol) and Xantphos (148mg, 0.26mmol) in dioxane (25mL) was purged with N₂(g) for 10min. Pd₂(dba)₃ (107mg, 0.12mmol) was added and mixture was heated to 90°C for 3h. Reaction cooled to rt and poured onto water (200mL), extracted with EtOAc (2 x 150mL) and CH₂Cl₂-IPA (150mL, 4:1). Combined organics were dried over Na₂SO₄, filtered and concentrated *in vacuo.* Flash column chromatography with heptane/EtOAc (4:1-0:1) then EtOAc/MeOH (1:0-3:1) yielded **(3)** as an off white solid (210 mg, 51%). ¹H NMR (500 MHz, Chloroform-*d*), δ_{H} ppm: 8.99 (d, *J*=1.4 Hz, 2H), 8.30 (dd, *J*=2.6, 1.5 Hz, 2H), 8.11 (d, *J*=2.7 Hz, 2H).
LCMS (ES): Found 174.1 [M+H]⁺.

NaH (60%, 48.5mg, 1.21mmol) was added to a solution of (3) (200mg, 1.15mmol) in DMF (7mL) at 5°C under N₂(g). The reaction mixture was stirred for 20min then methyl 4-(bromomethyl)benzoate (344mg, 1.5mmol) was added as a solution in DMF (3mL). The stirring was continued at 70°C for 1h. Reaction cooled to rt and poured onto water (100mL). Brine (25mL) was added and extracted with EtOAc (2 x 100mL). Combined organic was dried over Na₂SO₄, filtered and concentrated *in vacuo.* The residue was purified by flash column chromatography with CH₂Cl₂/EtOAc (1:0-0:1) then EtOAc/MeOH (1:0-4:1) to give **(4)** (196 mg, 53%).
¹H NMR (500 MHz, Chloroform-*d*), δ_{H} ppm: 8.59-8.65 (m, 2H), 8.23-8.26 (m, 2H), 8.16 (d, *J*=2.5 Hz, 2H), 7.94 (d, *J*=8.3 Hz, 2H), 7.38 (d, *J*=8.2 Hz, 2H), 5.50 (s, 2H), 3.86 (s, 3H).
LCMS (ES): Found 321.9 [M+H]⁺.

A solution of **(4)** (0.09mL, 0.61 mmol) in 0.85M hydroxylamine in MeOH (10 mL) was stirred at rt for 72h. Solvent concentrated to dryness and the residue purified by reverse phase HPLC to give **Example GG** (23 mg, 12%).
¹H NMR (500 MHz, Methanol-*d*₄), δ_{H} ppm: 8.66 (d, *J*=1.3 Hz, 2H), 8.28-8.36 (m, 2H), 8.16 (d, *J*=2.6 Hz, 2H), 7.67 (d, *J*=8.2 Hz, 2H), 7.45 (d, *J*=8.2 Hz, 2H), 5.56 (s, 2H).
LCMS (ES): Found 323.1 [M+H]⁺.

### Example HH

### 4-([Bis(pyrazin-2-yl)amino]methyl)-3-fluoro-N-hydroxybenzamide

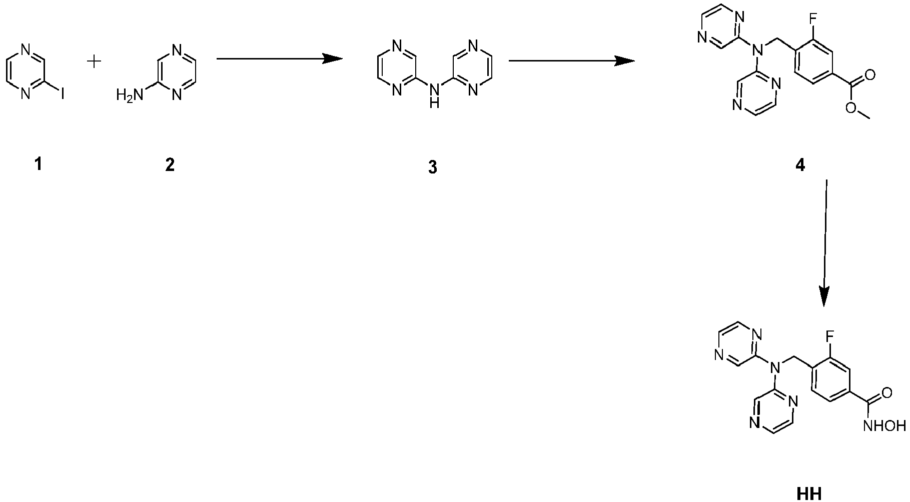

NaH (60%, 49mg, 1.21mmol) was added to a solution of **(3)** (200 mg, 1.15mmol) in DMF (7mL) at 5°C under N₂(g). The reaction mixture was stirred for 20min then methyl 4-(bromomethyl)-3-fluorobenzoate (371mg, 1.5mmol) was added as a solution in DMF (3mL). The stirring was continued at 70°C for 1h. Reaction cooled to rt and poured onto water (100mL). Brine (25mL) was added and the aqueous was extracted with EtOAc (2 x 100mL). Combined organics were dried over Na₂SO₄, filtered and concentrated *in vacuo.* Purification by flash column chromatography with CH₂Cl₂/EtOAc (1:0-0:1) then EtOAc/MeOH (1:0-4:1) yielded **(4)** (195mg, 50%).
¹H NMR (500 MHz, Chloroform-*d*), δ_{H} ppm: 8.65 (d, *J*=1.4 Hz, 2H), 8.25 (dd, *J*=2.5, 1.5 Hz, 2H), 8.18 (d, *J*=2.6 Hz, 2H), 7.65-7.72 (m, 2H), 7.31 (t, *J*=7.8 Hz, 1H), 5.53 (s, 2H), 3.87 (s, 3H).
LCMS (ES): Found 339.9 [M+H]⁺.

A solution of **(4)** (0.09mL, 0.57mmol) in 0.85M hydroxylamine in MeOH (10mL) was stirred at rt for 18h. Solvent was concentrated *in vacuo* and the residue purified by reverse phase HPLC to give **Example HH** (81 mg, 41%).
¹H NMR (500 MHz, DMSO-*d*₆), δ_{H} ppm: 8.76 (d, *J*=1.4 Hz, 2H), 8.34 (dd, *J*=2.5, 1.5 Hz, 2H), 8.25 (d, *J*=2.6 Hz, 2H), 7.51 (dd, *J*=11.1, 1.3 Hz, 1H), 7.45 (dd, *J*=8.0, 1.4 Hz, 1H), 7.34 (t, *J*=7.8 Hz, 1H), 5.50 (s, 2H).
LCMS (ES): Found 341.1 [M+H]⁺.

### Example II

### 6-([Bis(pyrazin-2-yl)amino]methyl)-N-hydroxypyridine-3-carboxamide

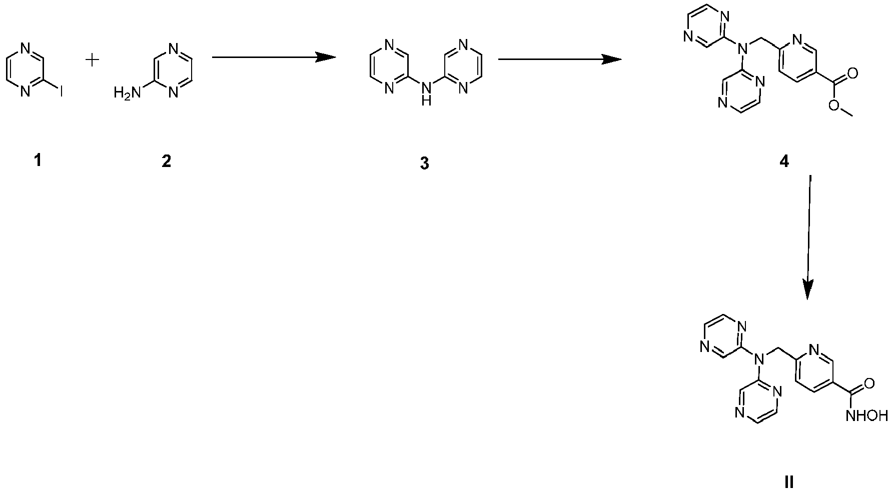

NaH (60%, 48.5mg, 1.21mmol) was added to a solution of **(3)** (200mg, 1.15mmol) in DMF (7mL) at 5°C under N₂(g). The reaction mixture was stirred for 20min then methyl 6-(bromomethyl)pyridine-3-carboxylate (345mg, 1.5mmol) was added as a solution in DMF (3mL). The stirring was continued at 70°C for 1h. Reaction cooled to rt and poured onto water (100mL). Brine (25mL) was added and the aqueous was extracted with EtOAc (2 x 100mL). Combined organics were dried over Na₂SO₄, filtered and concentrated *in vacuo.* The residue was purified by flash column chromatography with CH₂Cl₂/EtOAc (1:0-0:1) then EtOAc/MeOH (1:0-4:1) to give **(4)** (129mg, 35%).
¹H NMR (500 MHz, Chloroform-*d*), δ_{H} ppm: 9.04-9.13 (m, 1H), 8.70 (s, 2H), 8.19 (s, 2H), 8.13 (dd, *J*=5.6, 2.3 Hz, 3H), 7.32 (d, *J*=8.2 Hz, 1H), 5.55 (s, 2H), 3.86 (s, 3H).
LCMS (ES): Found 322.9 [M+H]⁺.

A solution of **(4)** (0.06mL, 0.4mmol) in 0.85M hydroxylamine in MeOH (10mL) was stirred at rt for 18h. The solvent was concentrated to dryness and the residue purified by reverse phase HPLC to give **Example II** (37mg, 28%).
¹H NMR (500 MHz, DMSO-*d*₆), δ_{H} ppm: 8.75 (d, *J*=1.3 Hz, 3H), 8.31 (dd, *J*=2.6, 1.5 Hz, 2H), 8.21 (d, *J*=2.6 Hz, 2H), 7.89 (dd, *J*=8.1, 2.0 Hz, 1H), 7.18 (d, *J*=8.1 Hz, 1H), 5.47 (s, 2H).
LCMS (ES): Found 324.1 [M+H]⁺.

### Example JJ

### N-Hydroxy-4-{[(3-methoxypyridin-2-yl)(pyrazin-2-yl)amino]methyl}benzamide

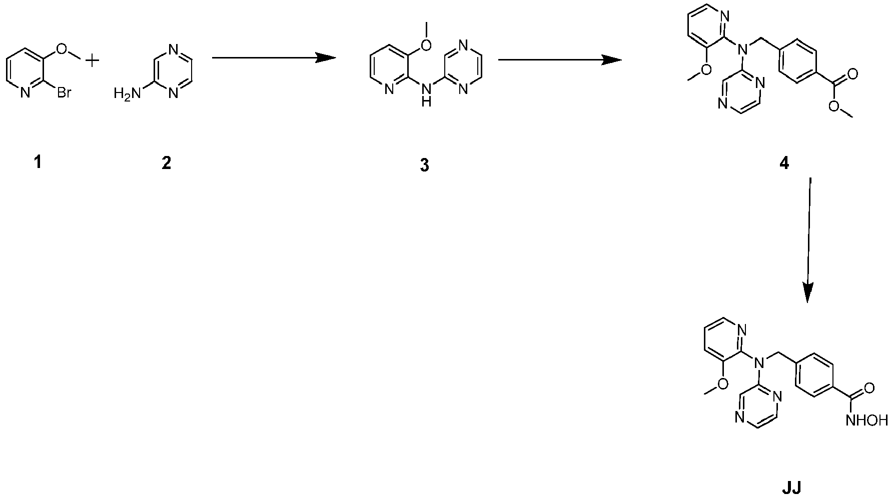

A solution of pyrazin-2-amine **(2)** (557mg, 5.85mmol), 2-bromo-3-methoxypyridine **(1)** (1.0g, 5.32mmol), Cs₂CO₃ (3.47g, 10.64mmol) and Xantphos (135mg, 0.23mmol) in dioxane (15mL) was purged with N₂(g) for 10min. Pd₂(dba)₃ (97.4mg, 0.11 mmol) was added and the mixture was heated to 90°C for 3h. The reaction was cooled to rt, partitioned between water (200mL) and EtOAc (200mL). Phases were separated and aqueous layer was washed with EtOAc (200+100+50mL). Combined organics were dried over Na₂SO₄, filtered and concentrated *in vacuo.* The residue was purified by flash column chromatography eluted with a gradient of CH₂Cl₂/EtOAc (1:0-0:1) to yield **(3)** (1.0g, 88%).
¹H NMR (500 MHz, Chloroform-*d*), δ_{H} ppm: 9.91 (d, *J*=1.2 Hz, 1H), 8.11-8.20 (m, 2H), 7.91 (dd, *J*=5.0, 1.4 Hz, 1H), 7.80 (s, 1H), 7.06 (dd, *J*=7.9, 1.3 Hz, 1H), 6.85 (dd, *J*=7.9, 5.0 Hz, 1H), 3.92 (s, 3H).
LCMS (ES): Found 203.2 [M+H]⁺.

NaH (60%, 41.5mg, 1.04mmol) was added to a solution of **(3)** (200mg, 0.99mmol) in DMF (10mL) at 5°C under N₂(g). The reaction mixture was stirred for 20 min then methyl 4-(bromomethyl)benzoate (294mg, 1.29mmol) was added. The stirring was continued at 70°C under N₂(g) for 1h. The reaction was cooled to rt and poured onto water (150mL) and brine (50mL), the aqueous was extracted with EtOAc (3 x 100mL). Combined organics were dried over Na₂SO₄, filtered and concentrated *in vacuo.* The residue was purified by flash column chromatography with CH₂Cl₂/EtOAc (1:0-0:1) then EtOAc/MeOH (1:0-4:1) to yield **(4)** (251mg, 73%).
¹H NMR (500 MHz, Chloroform-*d*), δ_{H} ppm: 8.06-8.10 (m, 2H), 7.87-7.92 (m, 3H), 7.78 (d, *J*=1.5 Hz, 1H), 7.44 (d, *J*=8.4 Hz, 2H), 7.23 (dd, *J*=8.2, 1.4 Hz, 1H), 7.15 (dd, *J*=8.1, 4.7 Hz, 1H), 5.42 (s, 2H), 3.85 (s, 3H), 3.73 (s, 3H).
LCMS (ES): Found 350.9 [M+H]⁺.

A solution of **(4)** (251mg, 0.72mmol) in 0.85M hydroxylamine in MeOH (10mL) was stirred at rt for 72h. The solvent concentrated to dryness and the residue purified by reverse HPLC to give **Example JJ** (101mg, 40%) as a beige solid.
¹H NMR (500 MHz, DMSO-*d*₆), δ_{H} ppm: 8.11 (dd, *J*=2.6, 1.6 Hz, 1H), 8.07 (dd, *J*=4.7, 1.3 Hz, 1H), 7.93 (d, *J*=2.7 Hz, 1H), 7.79 (d, *J*=1.4 Hz, 1H), 7.61 (d, *J*=8.2 Hz, 2H), 7.58 (dd, *J*=8.2, 1.2 Hz, 1H), 7.38 (d, *J*=8.2 Hz, 2H), 7.32 (dd, *J*=8.2, 4.7 Hz, 1H), 5.30 (s, 2H), 3.76 (s, 3H).
LCMS (ES): Found 352.1 [M+H]⁺.

### Example KK

### 3-Fluoro-N-hydroxy-4-([(3-methoxypyridin-2-yl)(pyrazin-2-yl)amino]methyl}benzamide

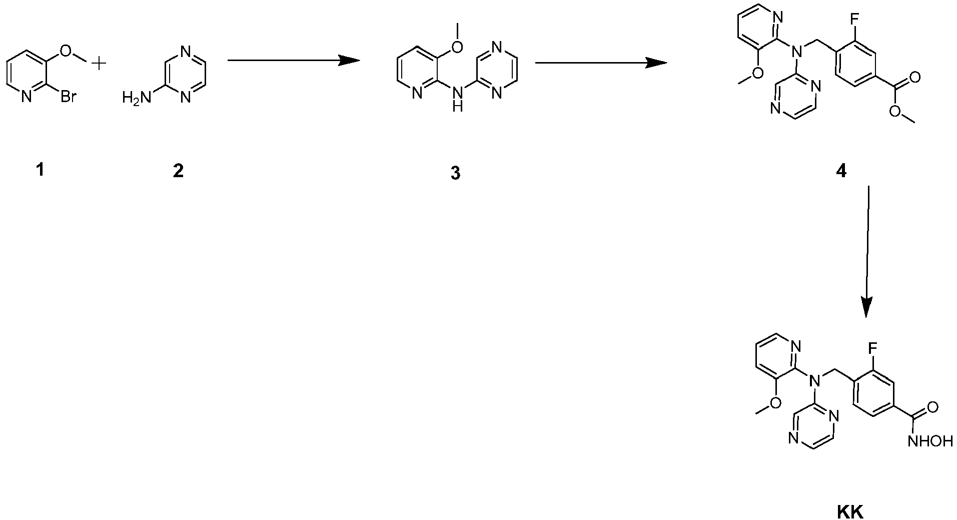

NaH (60%, 41.5mg, 1.04mmol) was added to a solution of (3) (200mg, 0.99mmol) in DMF (10mL) at 5°C under N₂(g). The reaction mixture was stirred for 20min then methyl 4-(bromomethyl)-3-fluorobenzoate (318mg, 1.29mmol) was added. The stirring was continued at 70°C under N₂(g) for 1h. The reaction cooled to rt and poured onto water (150mL) and brine (50mL), the aqueous extracted with EtOAc (3 x 100mL). Combined organics were dried over Na₂SO₄, filtered and concentrated *in vacuo.* The residue was purified by flash column chromatography with CH₂Cl₂/EtOAc (1:0-0:1) then EtOAc/MeOH (1:0-4:1) to give **(4)** (269mg, 74%).
¹H NMR (500 MHz, Chloroform-*d*), δ_{H} ppm: 8.09 (dd, *J*=4.7, 1.4 Hz, 1H), 8.06 (dd, *J*=2.6, 1.6 Hz, 1H), 7.90 (d, *J*=2.7 Hz, 1H), 7.80 (d, *J*=1.3 Hz, 1H), 7.68 (dd, *J*=8.0, 1.4 Hz, 1H), 7.62 (dd, *J*=10.5, 1.4 Hz, 1H), 7.56 (t, *J*=7.7 Hz, 1H), 7.27 (dd, *J*=8.3, 1.5 Hz, 1H), 7.18 (dd, *J*=8.2, 4.7 Hz, 1H), 5.43 (s, 2H), 3.86 (s, 3H), 3.77 (s, 3H).
LCMS (ES): Found 368.9 [M+H]⁺.

A solution of **(4)** (269mg, 0.73mmol) in 0.85M hydroxylamine in MeOH (10mL) was stirred at rt for 72h. The solvent was concentrated to dryness and the residue purified by reverse phase HPLC to give **Example KK** (93mg, 35%).
¹H NMR (500 MHz, DMSO-*d*₆), δ_{H} ppm: 8.13 (dd, *J*=2.6, 1.6 Hz, 1H), 8.08 (dd, *J*=4.7, 1.3 Hz, 1H), 7.95 (d, *J*=2.7 Hz, 1H), 7.80 (d, *J*=1.3 Hz, 1H), 7.61 (dd, *J*=8.3, 1.2 Hz, 1H), 7.48-7.43 (m, 3H), 7.35 (dd, *J*=8.2, 4.7 Hz, 1H), 5.32 (s, 2H), 3.78 (s, 3H).
LCMS (ES): Found 370.1 [M+H]⁺.

### Example LL

### N-Hydroxy-6-([(3-methoxypyridin-2-yl)(pyrazin-2-yl)amino]methyl}pyridine-3-carboxamide

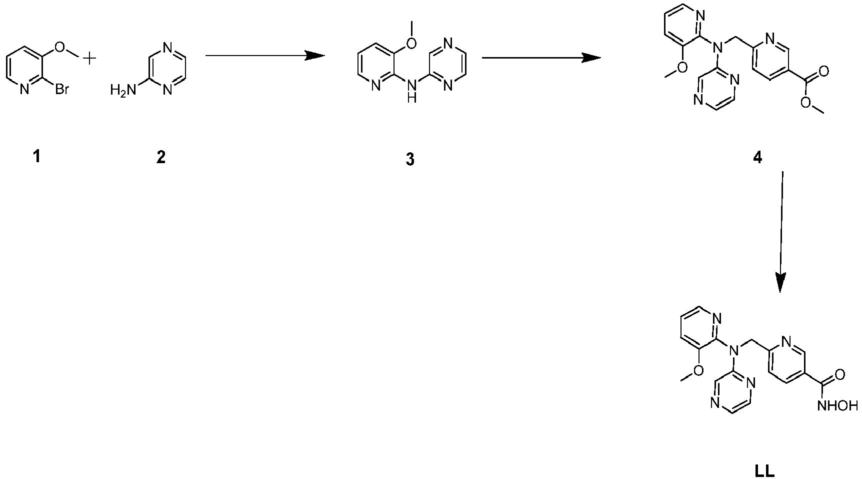

NaH (60%, 41.5mg, 1.04mmol) was added to a solution of (3) (200mg, 0.99mmol) in DMF (10mL) at 5°C under N₂(g). The reaction mixture was stirred for 20 min then methyl 6-(bromomethyl)pyridine-3-carboxylate (296mg, 1.29mmol) was added. The stirring was continued at 70°C under N₂(g) for 1h. The reaction was cooled to rt and poured onto water (150mL) and brine (50mL) and the aqueous extracted with EtOAc (3 x 100mL). Combined organics were dried over Na₂SO₄, filtered and concentrated *in vacuo.* The residue was purified by flash column chromatography with CH₂Cl₂/EtOAc (1:0-0:1) then EtOAc/MeOH (1:0-4:1) to give **(4)** (191mg, 55%).
¹H NMR (500 MHz, Chloroform-*d*), δ_{H} ppm: 9.07 (d, *J*=1.9 Hz, 1H), 8.12 (dd, *J*=8.2, 2.1 Hz, 1H), 8.06 (dd, *J*=4.7, 1.4 Hz, 1H), 8.01 (dd, *J*=2.6, 1.6 Hz, 1H), 7.88 (d, *J*=2.7 Hz, 1H), 7.84 (d, *J*=1.4 Hz, 1H), 7.54 (d, *J*=8.2 Hz, 1H), 7.27 (dd, *J*=8.2, 1.4 Hz, 1H), 7.17 (dd, *J*=8.2, 4.7 Hz, 1H), 5.46 (s, 2H), 3.86 (s, 3H), 3.76 (s, 3H).
LCMS (ES): Found 352.0 [M+H]⁺.

A solution of **(4)** (191mg, 0.54mmol) in 0.85M hydroxylamine in MeOH (10mL) was stirred at rt for 72h. After this time the solvent was concentrated to dryness and the residue purified by reverse phase HPLC to give **Example LL** (35mg, 19%).
¹H NMR (500 MHz, DMSO-*d*₆), δ_{H} ppm: 8.72 (d, *J*=1.8 Hz, 1H), 8.12-8.08 (m, 1H), 8.06 (dd, *J*=4.7, 1.3 Hz, 1H), 7.93 (d, *J*=2.7 Hz, 1H), 7.81-7.87 (m, 2H), 7.56-7.61 (m, 1H), 7.32 (dd, *J*=8.2, 4.7 Hz, 1H), 7.25 (d, *J*=8.1 Hz, 1H), 5.29 (s, 2H), 3.77 (s, 3H).
LCMS (ES): Found 353.1 [M+H]⁺.

### Example MM

### N-Hydroxy-4-([(pyrazin-2-yl)(pyridazin-3-yl)amino]methyl}benzamide

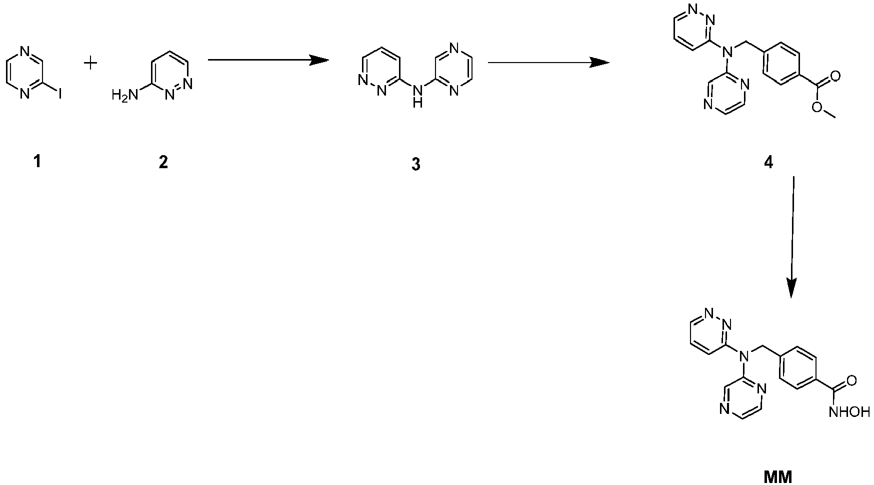

A solution of 2-iodopyrazine **(1)** (2.40g, 11.65mmol), pyridazin-3-amine **(2)** (1.2g, 12.82mmol), CS₂CO₃ (7.6g, 23.3mmol) and Xantphos (297mg, 0.51 mmol) in dioxane (45mL) was purged with N₂(g) for 10min. Pd₂(dba)₃ (214mg, 0.23mmol) in dioxane (5mL) was added and mixture was heated to 90°C for 3h. The reaction was cooled to rt and partitioned between water (200mL) and EtOAc
(200mL). The insoluble solid was filtered and put a-side. The phases were separated and aqueous was extracted with EtOAc (200mL), then CH₂Cl₂-IPA (200mL, 4:1). Combined organics were dried over Na₂SO₄, filtered and concentrated *in vacuo.* The residue was purified by flash column chromatography with CH₂Cl₂/EtOAc (1:0-0:1) then EtOAc/MeOH (1:0-4:1) to yield **(3).** The solid [from filtration] was washed with water (100mL) and triturated with hot MeOH (3x100mL) and filtered. The filtrates were concentrated to yield a second batch of **(3).** The solid was further washed with water (100mL) and was sucked dry to yield a third batch of **(3).** All three batches were combined to give **(3)** (1.63g, 80%).
¹H NMR (500 MHz, DMSO-*d*₆), δ_{H} ppm: 10.49 (s, 1H), 9.00 (d, *J*=1.2 Hz, 1H), 8.83 (dd, *J*=4.6, 1.2 Hz, 1H), 8.27 (dd, *J*=2.5, 1.5 Hz, 1H), 8.16 (d, *J*=2.7 Hz, 1H), 8.06 (dd, *J*=9.1, 1.2 Hz, 1H), 7.60 (dd, *J*=9.1, 4.6 Hz, 1H).
LCMS (ES): Found 174.2 [M+H]⁺.

NaH (60%, 49mg, 1.21mmol) was added to a solution of **(3)** (200mg, 1.15mmol) in DMF (8mL) at 5°C under N₂(g). The reaction mixture was stirred for 20min then methyl 4-(bromomethyl)benzoate (344mg, 1.5mmol) in DMF (2mL) was added. The stirring was continued at 70°C under N₂(g) for 1h. The reaction was cooled to rt, and poured onto water (200mL) and brine (50mL) and the aqueous extracted with EtOAc (2 x 150mL). Combined organics were dried over Na₂SO₄, filtered and concentrated *in vacuo.* The residue was purified by flash column chromatography with heptane/EtOAc (1:0-0:1) then EtOAc/MeOH (1:0-4:1) yielded **(4)** (119mg, 32%) as a brown oil.
¹H NMR (250 MHz, Chloroform-*d*), δ_{H} ppm: 8.85 (dd, *J*=4.6, 1.4 Hz, 1H), 8.56 (d, *J*=1.4 Hz, 1H), 8.25 (dd, *J*=2.6, 1.5 Hz, 1H), 8.17 (d, *J*=2.6 Hz, 1H), 7.89-7.97 (m, 2H), 7.48 (dd, *J*=9.1, 1.4 Hz, 1H), 7.42 (d, *J*=8.5 Hz, 2H), 7.33 (dd, *J*=9.1, 4.6 Hz, 1H), 5.64 (s, 2H), 3.86 (s, 3H).
LCMS (ES): Found 321.0 [M+H]⁺.

A solution of **(4)** (119mg, 0.37mmol) in 0.85M hydroxylamine in MeOH (10mL) was stirred at rt for 72 h. After this time the solvent was concentrated to dryness and the residue purified by reverse phase HPLC to give **Example MM** (24mg, 20%) as a beige solid.
¹H NMR (500 MHz, Methanol-*d*₄), δ_{H} ppm: 8.81 (dd, *J*=4.6, 1.2 Hz, 1H), 8.65 (d, *J*=1.4 Hz, 1H), 8.33 (dd, *J*=2.6, 1.5 Hz, 1H), 8.16 (d, *J*=2.6 Hz, 1H), 7.68 (d, *J*=8.6 Hz, 3H), 7.56 (dd, *J*=9.1, 4.6 Hz, 1H), 7.35 (d, *J*=8.2 Hz, 2H), 5.57 (s, 2H).
LCMS (ES): Found 322.2 [M+H]⁺.

### Example NN

### 3-Fluoro-N-hydroxy-4-([(pyrazin-2-yl)(pyridazin-3-yl)amino]methyl}benzamide

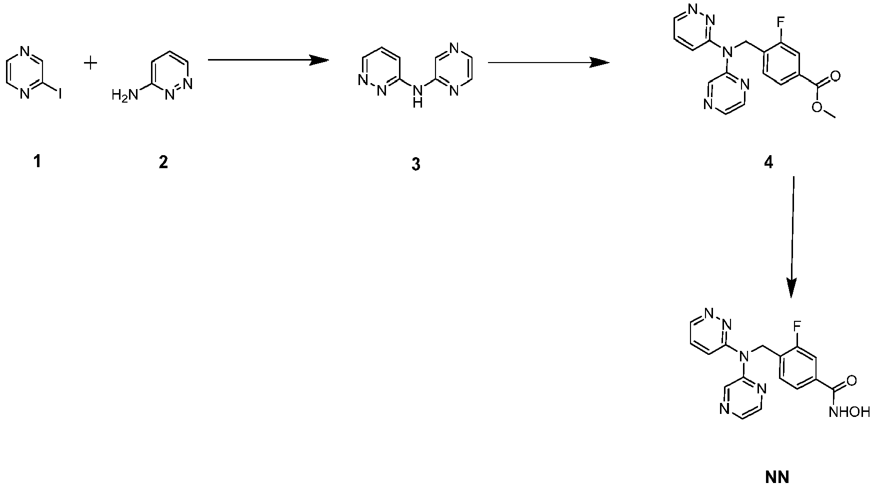

NaH (60%, 73mg, 1.82mmol) was added to a solution of **(3)** (300mg, 1.73mmol) in DMF (11mL) at 5°C under N₂(g). The reaction mixture was stirred for 20min then methyl 4-(bromomethyl)-3-fluorobenzoate (556mg, 2.25mmol) in DMF (4mL) was added. The stirring was continued at 70°C under N₂(g) for 1h. The reaction was cooled to rt and poured onto water (150mL) and brine (25mL) and the aqueous extracted with EtOAc (150+100mL). Combined organic were dried over Na₂SO₄, filtered and concentrated. The residue was purified by flash column chromatography with CH₂Cl₂/EtOAc (1:0-0:1) then EtOAc/MeOH (1:0-4:1) to yield **(4)** (141 mg, 24%) as a brown oil.
¹H NMR (500 MHz, Chloroform-*d*), δ_{H} ppm: 8.85 (dd, *J*=4.6, 1.3 Hz, 1H), 8.59 (d, *J*=1.4 Hz, 1H), 8.23 (dd, *J*=2.6, 1.5 Hz, 1H), 8.18 (d, *J*=2.6 Hz, 1H), 7.61-7.71 (m, 2H), 7.50 (dd, *J*=9.1, 1.3 Hz, 1H), 7.32-7.42 (m, 2H), 5.64 (s, 2H), 3.86 (s, 3H).
LCMS (ES): Found 339.9 [M+H]⁺.

A solution of **(4)** (141 mg, 0.42 mmol) in 0.85M hydroxylamine in MeOH (10 mL) was stirred at rt for 18h. The solvent was concentrated to dryness and the residue purified by reverse phase HPLC to give **Example NN** (51mg, 36%) as a beige solid.
¹H NMR (500 MHz, Methanol-*d*₄), δ_{H} ppm: 8.83 (dd, *J*=4.6, 1.1 Hz, 1H), 8.67 (d, *J*=1.3 Hz, 1H), 8.34 (dd, *J*=2.5, 1.5 Hz, 1H), 8.18 (d, *J*=2.6 Hz, 1H), 7.70 (dd, *J*=9.1, 1.2 Hz, 1H), 7.59 (dd, *J*=9.1, 4.6 Hz, 1H), 7.47 (d, *J*=11.7 Hz, 2H), 7.32 (t, *J*=8.0 Hz, 1H), 5.60 (s, 2H).
LCMS (ES): Found 341.0 [M+H]⁺.

### Example OO

### N-Hydroxy-4-([(3-methyl-1,2,4-thiadiazol-5-yl)(pyrazin-2-yl)amino] methyl}benzamide

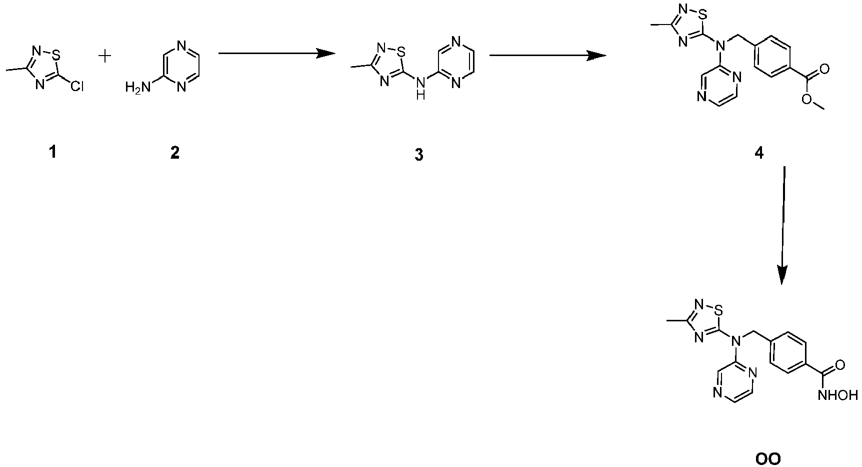

NaH (60%, 120mg, 3.3mmol) was added to a solution of (2) (140mg, 1.47mmol) in THF (10mL) under N₂(g). The reaction mixture was stirred for 10min then 5-chloro-3-methyl-1,2,4-thiadiazole **(1)** (190mg, 1.41mmol) was added. The mixture was heated up at 50°C under N₂(g) for 24h.
LCMS (ES): Found 194.0 [M+H]⁺.

To this mixture was added MeCN (10mL), methyl 4-(bromomethyl)benzoate (400mg, 1.74mmol) and potassium carbonate (350mg, 1.65mmol). Heating was then continued at 50°C for 2h. Once cooled, the mixture was partitioned between H₂O (10mL) and EtOAc (3 x 20mL). Combined organics were dried over Na₂SO₄, filtered and concentrated *in vacuo.* The residue was purified by flash column chromatography with Petrol/EtOAc (1:0-1:1) to yield **(4)** (300mg, 60% over 2 steps) as a white solid.
¹H NMR (400 MHz, DMSO-*d*₆), δ_{H} ppm: 8.55-8.77 (m, 2H), 8.41 (s, 1H), 7.92 (d, *J*=7.9 Hz, 2H), 7.39 (d, *J=*7.9 Hz, 2H), 5.92 (s, 2H), 3.82 (s, 3H), 2.42 (s, 3H).
LCMS (ES): Found 342.0 [M+H]⁺.

A solution of **(4)** (174 mg, 0.51mmol) in 0.85M hydroxylamine in MeOH (10 mL) was stirred at 70°C for 8h. The solvent was concentrated to dryness and the residue purified by reverse phase HPLC to give **Example OO** (44mg, 25%).
¹H NMR (400 MHz, DMSO-*d*₆), δ_{H} ppm:
11.45-10.94 (m, 1H), 9.43-8.80 (m, 1H), 8.70 (d, *J*=1.3 Hz, 1H), 8.61 (dd, *J*=2.6, 1.5 Hz, 1H), 8.40 (d, *J*=2.6 Hz, 1H), 7.70 (d, *J*=8.5 Hz, 2H), 7.31 (d, *J*=8.3 Hz, 2H), 5.88 (s, 2H), 2.43 (s, 3H).
LCMS (ES): Found 343.0 [M+H]⁺.

### Example PP

### N-Hydroxy-4-([(4-methoxypyridin-2-yl)(pyrazin-2-yl)amino]methyl}benzamide

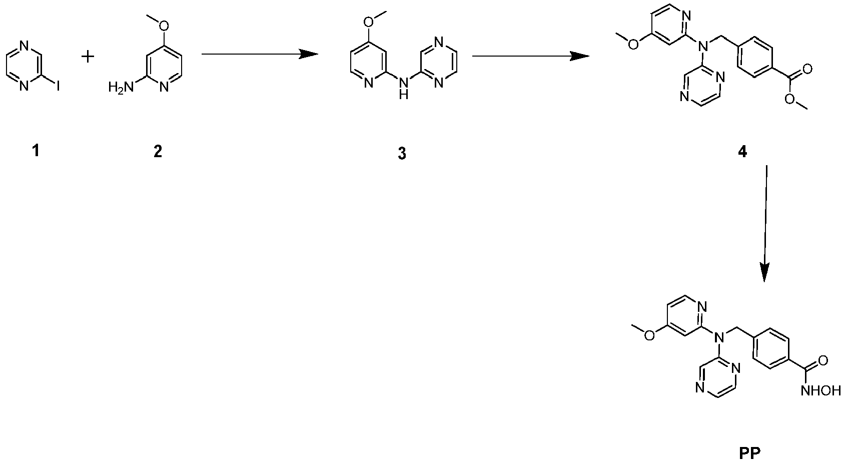

A solution of 2-iodopyrazine (1) (1.34g, 6.51mmol), 4-methoxypyridin-2-amine (2) (0.85g, 6.83mmol), Cs₂CO₃ (4.24g, 13.01mmol) and Xantphos (0.17g, 0.29mmol) in dioxane (22mL) was purged with N₂(g) for 10min then Pd₂(dba)₃ (0.12g, 0.13mmol) was added, re-purged for ∼5min and reaction was heated to 90°C for 4h. Once cooled down to rt, the mixture was partitioned between H₂O (150mL) and EtOAc (3 x 120mL). Combined organics were dried over Na₂SO₄, filtered and concentrated *in vacuo.* The residue was purified by flash column chromatography with CH₂Cl₂/EtOAc (9:1-0:1) to yield **(3)** (809mg, 61%) as a yellow solid.
¹H NMR (500 MHz, Chloroform-*d*), δ_{H} ppm: 8.70 (d, *J*=1.3 Hz, 1H), 8.11-8.22 (m, 3H), 8.08 (d, *J*=2.7 Hz, 1H), 7.43 (d, *J*=2.2 Hz, 1H), 6.52 (dd, *J*=5.8, 2.3 Hz, 1H), 3.88 (s, 3H).
LCMS (ES): Found 203.2 [M+H]⁺.

NaH (60%, 42mg, 1.04mmol) was added to a solution of (3) (200mg, 0.99mmol) in DMF (7mL) at rt under N₂(g). The reaction mixture was stirred for 30min then methyl 4-(bromomethyl)-3-fluorobenzoate (249mg, 1.09mmol) in DMF (2mL) was added. The reaction was heated up to 70°C under N₂(g) for 2h, then at rt overnight. The reaction was cooled to rt and partitioned between H₂O (150mL) and EtOAc (2 x 100mL). Combined organics were dried over Na₂SO₄, filtered and concentrated *in vacuo.* The residue was purified by flash column chromatography with CH₂Cl₂/EtOAc (1:0-0:1) to yield **(4)** (173mg, 50%) as a viscous oil.
¹H NMR (300 MHz, Chloroform-*d*), δ_{H} ppm: 8.63 (dd, *J*=1.4 Hz, 1H), 8.14-8.22 (m, 2H), 8.01 (d, *J*=2.6 Hz, 1H), 7.92 (d, *J*=8.2 Hz, 2H), 7.39 (d, *J*=8.2 Hz, 2H), 6.61 (d, *J*=2.1 Hz, 1H), 6.54 (dd, *J*=5.8, 2.2 Hz, 1H), 5.46 (s, 2H), 3.85 (s, 3H), 3.75 (s, 3H).
LCMS (ES): Found 350.9 [M+H]⁺.

A solution of **(4)** (173mg, 0.49mmol) in 0.85M hydroxylamine in MeOH (10mL) was stirred at rt for 72h. The solvent was concentrated to dryness and the residue purified by reverse phase HPLC to give **Example PP** (15mg, 9%).
¹H NMR (500 MHz, Methanol-*d*₄), δ_{H} ppm: 8.46 (d, *J*=1.4 Hz, 1H), 8.24 (dd, *J*=2.6, 1.5 Hz, 1H), 8.14 (d, *J*=5.9 Hz, 1H), 8.00 (d, *J*=2.7 Hz, 1H), 7.65 (d, *J*=8.3 Hz, 2H), 7.42 (d, *J*=8.3 Hz, 2H), 6.79 (d, *J*=2.2 Hz, 1H), 6.73 (dd, *J*=5.9, 2.2 Hz, 1H), 5.45 (s, 2H), 3.82 (s, 3H).
LCMS (ES): Found 352.0 [M+H]⁺.

### Example QQ

### N-Hydroxy-4-{[(pyrazin-2-yl)[6-(trifluoromethyl)pyrazin-2-yl]amino]methyl}benzamide

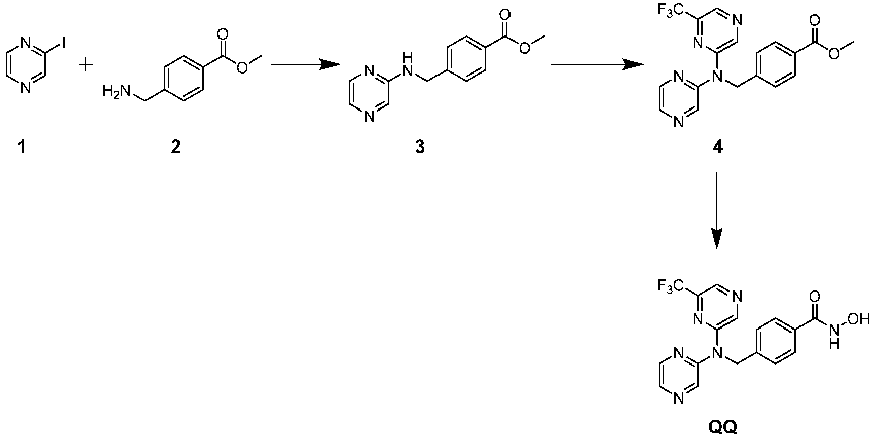

To a solution of methyl 4-(aminomethyl)benzoate hydrochloride (1.47g, 7.3mmol) in DMSO (14mL) was added 2-iodopyrazine (1g, 4.9mmol) followed by K₂CO₃ (1.7g, 12.1mmol) under Ar(g). After 2 min vigorous stirring, CuI (46mg, 0.2mmol) was added and the mixture was left to stir at rt overnight. It was partitioned between EtOAc (150mL) and 50% brine (50mL) and the organic layer separated, the aqueous extracted with EtOAc (2 x 15mL), before the combined organic phase was washed with 50% brine (15mL), dried (MgSO₄), and concentrated *in vacuo.* The residue was purified by flash column chromatography with Hexane/EtOAc (7:3-0:1) to yield **(3)** (670mg, 57%) as a white solid.
¹H NMR (300MHz, CHLOROFORM-*d*) δ_{H} ppm: 7.76-8.11 (m, 5H), 7.43 (d, *J*=8.5 Hz, 2H), 5.01-5.16 (m, 1H), 4.66 (d, *J*=5.8 Hz, 2H), 3.92 (s, 3H).
LCMS (ES): Found 352.0 [M+H]⁺.

To compound **(2)** (60mg, 0.25mmol), Pd₂(dba)₃ (11mg, 0.01mmol), (±)-BINAP (15mg, 0.025mmol) and Cs₂CO₃ (241mg, 0.74mmol) was added a solution of 2-chloro-6-(trifluoromethyl)pyrazine (90mg, 0.49mmol) in dioxane (2mL) under Ar(g). The reaction mixture was heated at 90°C for 4h then allowed to cool to rt overnight. EtOAc (15mL), water (4mL) and brine (2mL) were then added and the organic phase separated, extracting the aqueous with EtOAc (10mL). The combined organic phases were dried (MgSO₄) and concentrated *in vacuo* to give a crude residue (153mg). The residue was scavenged by dissolving in CH₂Cl₂/MeOH (1:1, 10mL) followed by the addition of MP-TMT (370mg, 0.68mmol/g). The mixture was agitated for 24h before filtering off the resin, washing with CH₂Cl₂/MeOH (1:1, 2 x 5mL). The filtrate was then concentrated *in vacuo* to give crude **(3)** (132mg), as a brown solid which was used directly in the next step.

To a solution of crude **(3)** (132mg total, containing maximum 0.25mmol) in THF/MeOH (1:1, 4mL) was added NH₂OH solution (50% wt. H₂O, 306□L, 5mmol) followed by NaOH (6M, 83□L, 0.5mmol). After 50 min stirring at rt, KHSO₄ (1M, 2mL), water (5mL) and CH₂Cl₂ (6mL) were added. The organic phase was separated and the aqueous extracted with CH₂Cl₂ (2 x 5mL). The combined organic phase was dried (MgSO₄) and concentrated *in vacuo* to give a yellow solid. Purification by reverse phase C-18 chromatography with MeCN/H₂O (19:1-1:1) gave **Example QQ** (81mg, 83% over 2 steps) as a light brown solid.
¹H NMR (DMSO-*d*₆) δ_{H} ppm: 8.93 (s, 1H), 8.88 (d, *J*=1.7 Hz, 1H), 8.62 (s, 1H), 8.42 (dd, *J*=2.6, 1.5 Hz, 1H), 8.34 (d, *J*=2.6 Hz, 1H), 7.62 (d, *J*=8.3 Hz, 2H), 7.27 (d, J=8.3 Hz, 2H), 5.46 (s, 2H).
LCMS (ES): Found 391.1 [M+H]⁺.

### Example RR

### 4-({[5-(6-Aminopyridin-3-yl)pyridin-2-yl](pyrazin-2-yl)amino}methyl)-N-hydroxybenzamide

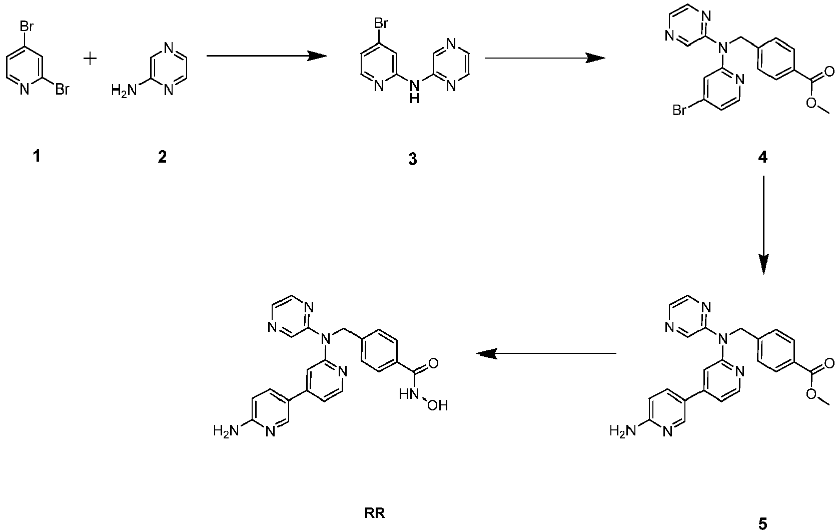

A mixture of 2,4-dibromopyridine **(1)** (5.0g, 21.1mmol), pyrazin-2-amine **(2)** (2.21g, 23.22mmol), CS₂CO₃ (15.1g, 46.4mmol) and Xantphos (611mg, 1.05mmol) was suspended in dioxane (50mL). The mixture was flushed with N₂(g) for 1min before Pd₂(dba)₃ (386mg, 0.422mmol) was added. Mixture was flushed again with N₂(g) and it was heated up to 90°C overnight. Once cooled, the mixture was partitioned between H₂O (150mL) and EtOAc (3 x 150mL). The combined organic extracts were washed with brine, dried with MgSO₄, filtered and concentrated *in vacuo.* Purification by flash column chromatography with heptane/EtOAc (9:1-2:3) to yield **(3)** (2.6g, 49%) as pale yellow solid.
¹H NMR (500 MHz, Chloroform-*d*), δ_{H} ppm: 8.74 (d, *J*=1.3 Hz, 1H), 8.22 (dd, *J*=2.6, 1.5 Hz, 1H), 8.15 (d, *J*=2.7 Hz, 1H), 8.11 (d, *J*=5.4 Hz, 1H), 8.07 (d, *J*=1.5 Hz, 1H), 7.63 (s, 1H), 7.10 (dd, *J*=5.4, 1.6 Hz, 1H).
LCMS (ES): Found 251.0-253.0 [M+H]⁺.

To a solution of **(3)** (1.08g, 4.3mmol) in DMF (15mL) cooled to 0°C under N₂(g) was added NaH (60%, 206mg, 5.16mmol). The mixture was stirred for 30min. Then, a solution of methyl 4-(bromomethyl)benzoate (1.08g, 4.73mmol) in DMF (5mL) was added and the mixture was heated up to 50°C for 1.5h. Once cooled down, the reaction was partitioned between H₂O (150mL) and EtOAc (3 x 150mL). The combined organic extracts were washed with brine, dried with MgSO₄, filtered and concentrated *in vacuo.* Purification by flash column chromatography with heptane/EtOAc (9:1-2:3) to yield **(4)** (915mg, 53%) as white solid.
¹H NMR (500 MHz, Chloroform-*d*), δ_{H} ppm: 8.66 (d, *J*=1.4 Hz, 1H), 8.25 (dd, *J*=2.5, 1.6 Hz, 1H), 8.15 (d, *J*=5.3 Hz, 1H), 8.13 (d, *J*=2.6 Hz, 1H), 7.95 (d, *J*=8.3 Hz, 2H), 7.39 (d, *J*=8.3 Hz, 2H), 7.33 (d, *J*=1.4 Hz, 1H), 7.10 (dd, *J*=5.3, 1.5 Hz, 1H), 5.49 (s, 2H), 3.88 (s, 3H).
LCMS (ES): Found 399.0-401.0 [M+H]⁺.

To a suspension of **(4)** (200mg, 0.50mmol), 5-(4,4,5,5-tetramethyl-1,3,2-dioxaborolan-2-yl)pyridin-2-amine (132.3mg, 0.6mmol) and CS₂CO₃ (326mg, 1.0mmol) in DMF (4mL) and H₂O (1mL) was added Pd(PPh₃)₄ (58mg, 0.05mmol). The mixture was flushed with N₂(g) then it was heated up to 90°C for 2h. Once cooled down, H₂O (20mL) was added and a precipitate was left to settle at rt for 72h.

After filtration, washings with H₂O (2mL) and drying, **(5)** was obtained as a brown solid (219mg, quant.).
¹H NMR (500 MHz, Methanol-*d*₄), δ_{H} ppm: 8.54 (s, 1H), 8.31 (d, *J*=5.3 Hz, 1H), 8.25-8.28 (m, 1H), 8.23 (d, *J*=2.3 Hz, 1H), 8.02 (d, *J*=2.6 Hz, 1H), 7.92 (d, *J*=8.2 Hz, 2H), 7.77 (dd, *J*=8.8, 2.4 Hz, 1H), 7.50 (s, 1H), 7.48 (d, *J*=5.5 Hz, 2H), 7.32 (d, *J*=5.4 Hz, 1H), 6.65 (d, *J*=8.8 Hz, 1H), 5.55 (s, 2H), 3.86 (s, 3H).
LCMS (ES): Found 413.0 [M+H]⁺.

A solution of **(5)** (219mg, 0.53mmol) in 0.85M NH₂OH in MeOH (5mL) was stirred at rt overnight. The volatiles were then removed *in vacuo* and the residue was purified by reverse prep HPLC to give **Example RR** (19mg, 8%) as pale yellow solid.
1H NMR (500 MHz, DMSO-*d*₆), δ_{H} ppm: 8.63 (d, *J*=1.4 Hz, 1H), 8.35 (d, *J*=2.3 Hz, 1H), 8.27-8.28 (m, 1H), 8.26-8.27 (m, 1H), 8.07 (d, *J*=2.6 Hz, 1H), 7.76 (d, *J*=2.6 Hz, 1H), 7.61 (d, *J*=8.3 Hz, 2H), 7.51 (s, 1H), 7.30 (dd, *J*=5.3, 1.5 Hz, 1H), 7.26 (d, *J*=8.2 Hz, 2H), 6.52 (d, *J*=8.7 Hz, 1H), 6.36 (s, 2H), 5.45 (s, 2H).
LCMS (ES): Found 414.0 [M+H]⁺.

### Example SS

### 4-({[5-(2-Aminopyridin-4-yl)pyridin-2-yl](pyrazin-2-yl)amino}methyl)-N-hydroxybenzamide

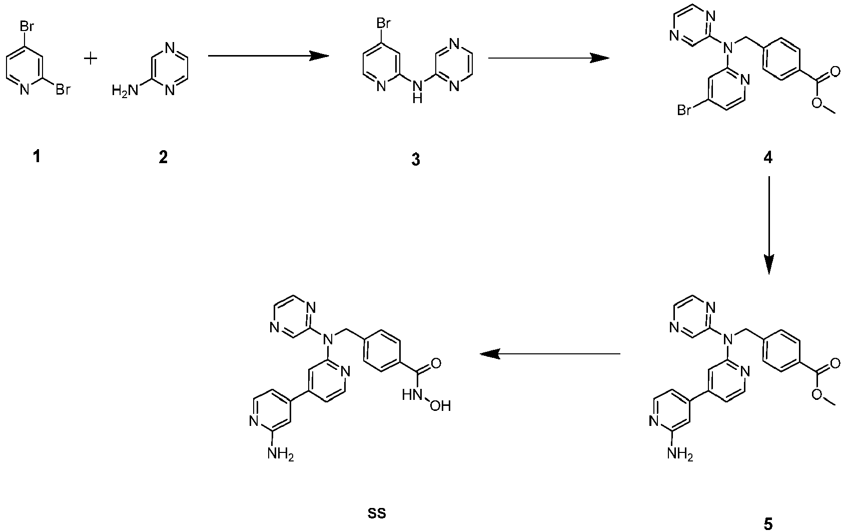

To a suspension of **(4)** (200mg, 0.50mmol), 4-(4,4,5,5-tetramethyl-1,3,2-dioxaborolan-2-yl)pyridin-2-amine (132.3mg, 0.6mmol) and Cs₂CO₃ (326mg, 1.0mmol) in DMF (4mL) and H₂O (1mL) was added Pd(PPh₃)₄ (58mg, 0.05mmol). The mixture was flushed with N₂(g) then it was heated up to 90°C for 2h. Once cooled down, H₂O (20mL) was added and a precipitate was left to settle at rt for 3h.

After filtration, washings with H₂O (2mL) and drying, a pale orange solid was obtained, which was purified by flash column chromatography with heptane/EtOAc (4:1-0:1) then EtOAc/MeOH (1:0-7:3) to give **(5)** (82mg, 40%) as a yellow solid.
1H NMR (500 MHz, Methanol-*d*₄), δ_{H} ppm: 8.60 (s, 1H), 8.41 (d, *J*=5.2 Hz, 1H), 8.29 (d, *J*=1.3 Hz, 1H), 8.06 (d, *J*=2.5 Hz, 1H), 7.97 (d, *J*=5.4 Hz, 1H), 7.93 (d, *J*=8.3 Hz, 2H), 7.53 (s, 1H), 7.49 (d, *J*=8.1 Hz, 2H), 7.34 (d, *J*=5.2 Hz, 1H), 6.81-6.84 (m, 1H), 6.81 (s, 1H), 5.58 (s, 2H), 3.86 (s, 3H).
LCMS (ES): Found 413.0 [M+H]⁺.

A solution of **(5)** (82mg, 0.20mmol) in 0.85M NH₂OH in MeOH (5mL) was stirred at rt overnight. The volatiles were then removed *in vacuo* and the residue was purified by reverse prep HPLC to give **Example SS** (19mg, 8%) as white solid.
1H NMR (500 MHz, Methanol-*d*₄), δ_{H} ppm: 8.59 (d, *J*=1.4 Hz, 1H), 8.39 (d, *J*=5.2 Hz, 1H), 8.29 (dd, *J*=2.7, 1.5 Hz, 1H), 8.05 (d, *J*=2.7 Hz, 1H), 7.97 (d, *J*=5.5 Hz, 1H), 7.66 (d, *J*=8.3 Hz, 2H), 7.49 (s, 1H), 7.45 (d, *J*=8.2 Hz, 2H), 7.32 (dd, *J*=5.2, 1.2 Hz, 1H), 6.82 (dd, *J*=5.5, 1.3 Hz, 1H), 6.78 (s, 1H), 5.55 (s, 2H).
LCMS (ES): Found 414.0 [M+H]⁺.

### Example TT

### N-hydroxy-4-[({5-[2-(methylamino)pyridin-4-yl]pyridin-2-yl}(pyrazin-2-yl)amino)methyl]benzamide

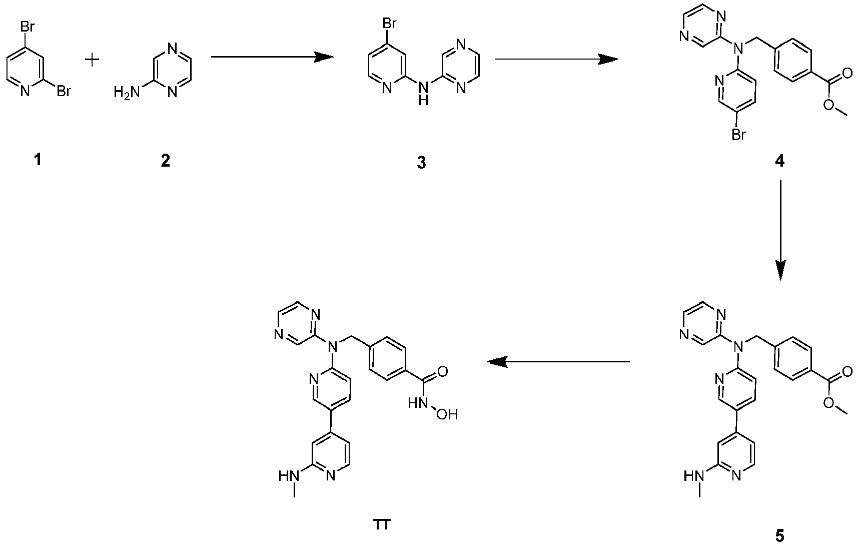

To a suspension of **(4)** (120mg, 0.3mmol), N-methyl-4-(tetramethyl-1,3,2-dioxaborolan-2-yl)pyridin-2-amine (84mg, 0.36mmol) and CS₂CO₃ (196mg, 0.6mmol) in DMF (2mL) and H₂O (0.5mL) was added Pd(PPh₃)₄ (58mg, 0.05mmol). The mixture was flushed with N₂(g) then it was heated up to 90°C for 4h. Once cooled down, H₂O (10mL) was added and the reaction was stirred for 20 min.

After filtration, washings with MeCN (2mL) and drying, a black solid was obtained, which was purified by preparative HPLC to give **(5)** (80mg, 59%) as a white solid.
1H NMR (500 MHz, DMSO-*d*₆), δ_{H} ppm: 8.70 (d, *J*=1.4 Hz, 1H), 8.39 (d, *J*=5.2 Hz, 1H), 8.29 (dd, *J*=2.6, 1.5 Hz, 1H), 8.14 (d, *J*=2.6 Hz, 1H), 8.07 (d, *J*=5.3 Hz, 1H), 7.87 (d, *J*=8.4 Hz, 2H), 7.54-7.56 (m, 1H), 7.50 (d, *J*=8.3 Hz, 2H), 7.32 (dd, *J*=5.2, 1.4 Hz, 1H), 6.77 (dd, *J*=5.3, 1.5 Hz, 1H), 6.65-6.67 (m, 1H), 6.61 (d, *J*=5.2 Hz, 1H), 5.56 (s, 2H), 3.80 (s, 3H), 2.80 (d, *J*=4.9 Hz, 3H).
LCMS (ES): Found 427.5 [M+H]⁺.

To a solution of **(5)** (80mg, 0.20mmol) in MeOH/THF (1:1, 2mL) was added hydroxylamine (50% w/w in H₂O; 0.11mL, 3.75mmol) followed by 6N NaOH (63□L, 0.38mmol). The mixture was stirred at rt for 3h. Then, 1M KHSO₄ (2mL) was added followed by H₂O (6mL). It was extracted with IPA/Chloroform (1:2, 3 x 20mL).

The combined organic extracts were washed with brine, dried with MgSO₄, filtered and concentrated *in vacuo.* Purification by preparative HPLC yielded **Example TT** (21 mg, 25%) as a pale orange solid.
1H NMR (500 MHz, Methanol-*d*₄), δ_{H} ppm: 11.08 (br.s., 1H), 8.69 (dd, *J*=6.3, 1.4 Hz, 1H), 8.39 (dd, *J*=5.0, 1.4 Hz), 8.28-8.32 (m, 1H), 8.13 (dd, *J*=6.0, 2.6 Hz, 1H), 8.07 (dd, *J*=5.2, 3.3 Hz, 1H), 7.63-7.67 (m, 1H), 7.58 (d, *J*=8.4 Hz, 1H), 7.53 (m, 1H), 7.42 (d, *J*=8.4 Hz, 1H), 7.36 (d, *J*=8.4 Hz, 1H), 7.31 (ddd, *J*=8.5, 5.3, 1.4, 1H), 6.65 (ddd, *J*=8.5, 5.4, 1.5 Hz), 6.66 (d, *J*=9.1 Hz, 1H), 6.58-6.63 (m, 1H), 5.51 (m, 1H), 2.80 (dd, *J*=4.8, 2.9 Hz, 3H).
LCMS (ES): Found 428.2 [M+H]⁺.

### Example UU

### N-hydroxy-4-{[(pyrazin-2-yl)[5-(pyridin-4-yl)pyridin-2-yl]amino]methyl}benzamide

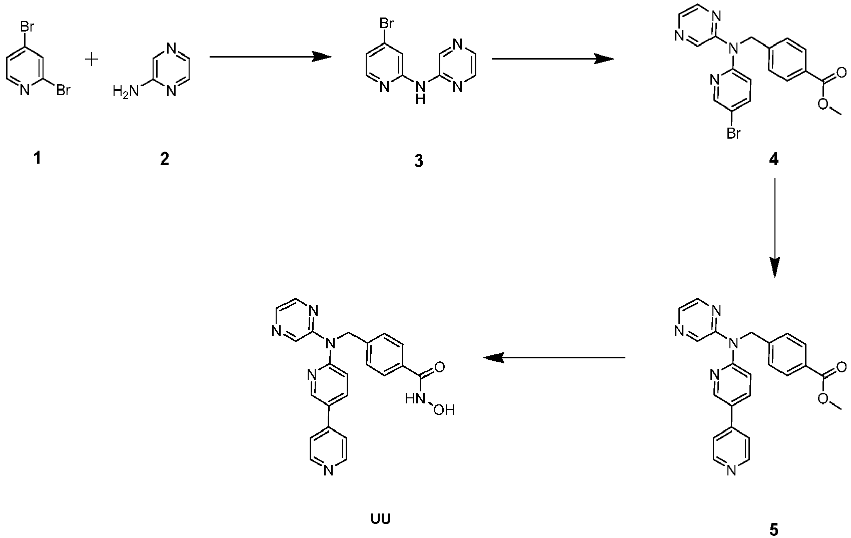

To a suspension of **(4)** (120mg, 0.3mmol), (pyridin-4-yl)boronic acid (49mg, 0.36 mmol) and Cs₂CO₃ (196mg, 0.6mmol) in DMF (2mL) and H₂O (0.5mL) was added Pd(PPh₃)₄ (35mg, 0.03mmol). The mixture was flushed with N₂(g) then it was heated up to 90°C for 4h. Once cooled down, H₂O (10mL) was added and the reaction was stirred for 20 min.

After filtration, a gum was obtained, which was purified by preparative HPLC then SCX column to give **(5)** (82mg, 65%) as a colourless oil.
LCMS (ES): Found 398.5 [M+H]⁺.

To a solution of **(5)** (82mg, 0.21mmol) in MeOH/THF (1:1, 2mL) was added hydroxylamine (50% w/w in H₂O; 0.15mL, 0.42mmol) followed by 6N NaOH (80□L, 0.42mmol). The mixture was stirred at rt for 2h.

The volatiles were then removed *in vacuo* and the residue was purified by reverse prep HPLC to give **Example UU** (39mg, 48%) as white solid.
1H NMR (500 MHz, DMSO-*d*₆), δ_{H} ppm: 11.05 (br. s., 1H), 8.95 (br. s., 1H), 8.68-8.71 (m, 3H), 8.44 (d, *J*=5.2 Hz, 1H), 8.28-8.31 (m, 1H), 8.14 (d, *J*=2.6 Hz, 1H), 7.72-7.78 (m, 3H), 7.64 (d, *J*=8.2 Hz, 2H), 7.47 (dd, *J*=5.2, 1.4 Hz, 1H), 7.42 (d, *J*=8.0 Hz, 2H), 5.55 (s, 2H).
LCMS (ES): Found 399.4 [M+H]⁺.

### Biochemical Assay and Data - Compounds of Formula I

Fold form selectivity inhibition data against class I PI3K isoforms, as determined using a HTRF biochemical assay conducted at Reaction Biology Corp., is listed below.

| **Example** | Fold IC₅₀ | | | |
|---|---|---|---|---|
| | p110β/p110α | p110β/p110γ | p110δ/p110α | p110δ/p110γ |
| **A** | * | ** | * | ** |
| **B** | ** | ** | ** | ** |
| **C** | * | ** | ** | ** |
| **D** | ** | ** | ** | ** |
| **E** | ** | ** | ** | ** |
| **F** | * | * | ** | ** |
| **G** | * | ** | ** | ** |

| | | | | |
|---|---|---|---|---|
| Key : * = ≥ 10x ≥ 50x; ** = > 50x | | | | |

### Rodent Pharmacokinetic Comparative Data

Disclosed compounds have increased bioavailability and reduced clearance (data below for mice).

### Example A

The following protocol was used to determine oral bioavailability and clearance, and the results are shown below:
- Species = male mouse;
- Strain = CD1;
- n = 3 male mice per time point per route;
- Terminal blood sampling at 8 time points (5min, 10min, 0.5hr, 1hr, 3hr, 6hr, 8hr and, 24hr);
- Collection of plasma, bio-analysis and report of pharmacokinetic parameters.

Formulation: 10% DMSO, 90% Saline
Dosing: 10mg/kg P.O. and 5mg/kg I.V.

Plasma PK Summary:

| **Parameters - IV, 5mg/kg** | **Value - Mesylate Salt** |
|---|---|
| t_{1/2} (hr) | 1.3 |
| Tₘₐₓ (hr) | 0.08 |
| Cₘₐₓ (ng/mL) | 2640 |
| AUCₗₐₛₜ (hr*ng.mL) | 3905 |
| AUCₐₗₗ (hr*ng/mL) | 3905 |
| AUC_{inf} (hr*ng/mL) | 3946 |
| Clearance (mL/hr/Kg) | 1267 |
| Vd (mL/Kg) | 2441 |

| **Parameters** - **PO, 10mg/kg** | **Value - Mesylate Salt** |
|---|---|
| t_{1/2} (hr) | 1.3 |
| Tₘₐₓ (hr) | 1.00 |
| Cₘₐₓ (ng/mL) | 1973 |
| AUCₗₐₛₜ (hr*ng/mL) | 5625 |
| AUCₐₗₗ (hr*ng/mL) | 5625 |
| AUC_{inf} (hr* ng/mL) | 5822 |
| F | 73.77% |

### Example A

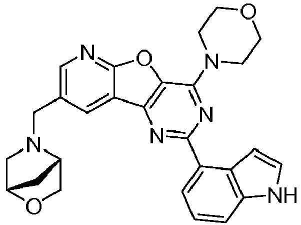

Oral bioavailability (F) = 74%
Clearance = 21mL/min/kg

### Example B

The following protocol was used to determine oral bioavailability and clearance, and the results are shown below:
- Species = male mouse;
- Strain = Balb/c;
- 18 male mice were divided into two groups Group 1 (3 mg/kg; I.V.), Group 2 (10 mg/kg; P.O.) with each group comprising of nine mice;
- Blood samples (approximately 60 µL) were collected from retro orbital plexus under light isoflurane anesthesia such that the samples were obtained at pre-dose, 0.08, 0.25, 0.5, 1, 2, 4, 8 and 24 hr (I.V.) and pre-dose, 0.25, 0.5, 1, 2, 4, 6, 8 and 24 hr (P.O.);
- The blood samples were collected from a set of three mice at each time point in labeled micro centrifuge tube containing K2EDTA as anticoagulant;
- Plasma samples were separated by centrifugation of whole blood and stored below -70°C until bioanalysis;
- All samples were processed for analysis by protein precipitation using acetonitrile (ACN) and analyzed with fit for purpose LC/MS/MS method (LLOQ: 2.02 ng/mL);
- Pharmacokinetic parameters were calculated using the non-compartmental analysis tool of Phoenix WinNonlin (Version 6.3).

### Formulation:

Animals in Group 1 were administered intravenously with Example B solution formulation in 20% Propylene Glycol, 50% of PEG 400 and 30% of (20% HPβCD in water) via tail vein at a dose of 3 mg/kg.

Animals in Group 2 were administered with oral solution formulation of Example B in 20% Propylene Glycol, 50% of PEG 400 and 30% of (20% HPβCD in water) at a dose of 10 mg/kg;

Dosing: 10mg/kg P.O. and 3mg/kg I.V.

Plasma PK Summary:

| **Parameters** - **IV, 3mg/kg** | **Value** - **Mesylate Salt** |
|---|---|
| t_{1/2} (hr) | 1.23 |
| Cₘₐₓ (ng/mL) | 621.42 |
| AUCₗₐₛₜ (hr*ng.mL) | 1512.20 |
| AUC_{inf} (hr*ng/mL) | 1512.20 |
| Clearance (mL/hr/Kg) | 1983.6 |
| Vss (L/Kg) | 5.51 |

| **Parameters** - **PO, 10mg/kg** | **Value** - **Mesylate Salt** |
|---|---|
| Tₘₐₓ(hr) | 1.00 |
| Cₘₐₓ (ng/mL) | 779.58 |
| AUCₗₐₛₜ (hr*ng/mL) | 3725.56 |
| AUC_{inf}(hr*ng/mL) | 4103.86 |
| F | 74% |

### Example B

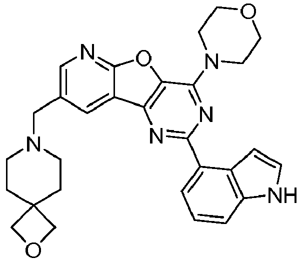

Oral bioavailability (F) = 74%
Clearance = 33mL/min/kg

### Example G

The following protocol was used to determine oral bioavailability and clearance, and the results are shown below:
- Species = male mouse;
- Strain = Balb/c;
- 18 male mice were divided into two groups Group 1 (3 mg/kg; I.V.), Group 2 (10 mg/kg; P.O.) with each group comprising of nine mice;
- Blood samples (approximately 60 µL) were collected from retro orbital plexus under light isoflurane anesthesia such that the samples were obtained at pre-dose, 0.08, 0.25, 0.5, 1, 2, 4, 8 and 24 hr (I.V.) and pre-dose, 0.25, 0.5, 1, 2, 4, 6, 8 and 24 hr (P.O.);
- The blood samples were collected from set of three mice at each time point in labeled micro centrifuge tube containing K2EDTA as anticoagulant;
- Plasma samples were separated by centrifugation of whole blood and stored below -70°C until bioanalysis;
- All samples were processed for analysis by protein precipitation using acetonitrile (ACN) and analyzed with fit for purpose LC/MS/MS method (LLOQ: 2.47 ng/mL);
- Pharmacokinetic parameters were calculated using the non-compartmental analysis tool of Phoenix WinNonlin (Version 6.3).

### Formulation:

Animals in Group 1 were administered intravenously with Example G solution formulation in 5% NMP, 5% solutol HS-15 in 90% HPβCD solution (20% HPβCD in RO water) at 3 mg/kg dose.

Animals in Group 2 were administered orally with 10 mg/kg solution formulation of Example G in 5% NMP, 5% solutol HS-15 in 90% HPβCD solution (20% HPβCD in RO water)

Dosing: 10mg/kg P.O. and 3mg/kg I.V.

Plasma PK Summary:

| **Parameters** - **IV, 3mg/kg** | **Value** - **Mesylate Salt** |
|---|---|
| t_{1/2} (hr) | 0.59 |
| Cₘₐₓ(ng/mL) | 2205.80 |
| AUCₗₐₛₜ(hr*ng.mL) | 1918.37 |
| AUC_{inf}(hr*ng/mL) | 1935.24 |
| Clearance (mL/hr/Kg) | 1550.4 |
| Vss (L/Kg) | 1.25 |

| **Parameters** - **PO, 10mg/kg** | **Value** - **Mesylate Salt** |
|---|---|
| Tₘₐₓ(hr) | 0.25 |
| Cₘₐₓ(ng/mL) | 833.35 |
| AUCₗₐₛₜ(hr*ng/mL) | 1892.53 |
| AUC_{inf}(hr*ng/mL) | 2144.97 |
| F | 30% |

### Example G

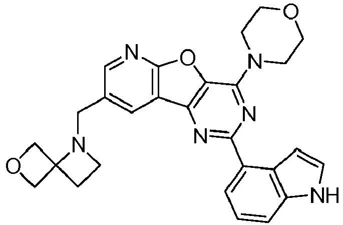

Oral bioavailability (F) = 30%
Clearance = 26 mL/min/kg

### Comparative Example (Example I in WO2011/021038)

The following protocol was used to determine oral bioavailability and clearance, and the results are shown below:
- Species = male mouse;
- Strain = CD1;
- n=3 male mice per time point per route;
- Terminal blood sampling at 8 time points (5min, 10min, 0.5hr, 1hr, 3hr, 6hr, 8hr and, 24hr);
- Collection of plasma, bio-analysis and report of pharmacokinetic parameters.

Formulation: 10% DMSO, 90% Saline
Dosing: 10mg/kg P.O. and 5mg/kg I.V.

Plasma PK Summary:

| **Parameters** - **IV, 5mg/kg** | **Value** - **Mesylate Salt** | **Value** - **HCl Salt** |
|---|---|---|
| t_{1/2} (hr) | 1.6 | 7.6 |
| Tₘₐₓ(hr) | 0.08 | 0.08 |
| Cₘₐₓ(ng/mL) | 1618 | 1712 |
| AUCₗₐₛₜ(hr*ng.mL) | 1245 | 1479 |
| AUCₐₗₗ (hr*ng/mL) | 1245 | 1479 |
| AUC_{inf}(hr*ng/mL) | 1261 | 1515 |
| Clearance (mL/hr/Kg) | 3966 | 3300 |
| Vd (mL/Kg) | 4601 | 10063 |

| **Parameters** - **PO, 10mg/kg** | **Value** - **Mesylate Salt** | **Value** - **Hcl Salt** |
|---|---|---|
| t_{1/2} (hr) | 1.9 | 1.8 |
| Tₘₐₓ(hr) | 1.0 | 1.0 |
| Cₘₐₓ(ng/mL) | 212 | 322 |
| AUCₗₐₛₜ (hr*ng/mL) | 657 | 849 |
| AUCₐₗₗ (hr*ng/mL) | 657 | 849 |
| AUC_{inf}(hr* ng/mL) | 700 | 896 |
| F | 27.8% | 29.6% |

Example I in WO2011/021038 (Comparative) - mesylate salt form

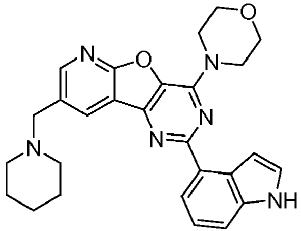

Oral bioavailability (F) = 28%
Clearance = 66mL/min/kg

### Summary

| **Compound** | **Oral Bioavailability (F)** | **Clearance (mL/min/kg)** |
|---|---|---|
| Example A | 74 | 21 |
| Example B | 74 | 33 |
| Example G | 30 | 26 |
| Example I from WO2011 /021038 (comparative) | 28 | 66 |

### Biochemical Assay and Data - Compounds of Formula II

### 1) Assay

### i. Biochemical Assay Description

Activity against all zinc-dependent HDACs 1 to 11 was assessed by using an acetylated AMC-labeled peptide substrate. The substrate RHKKAc was used for all class I and IIb HDACs; for HDAC8, the substrate used was RHKAcKAc. Activity against the class IIa HDACs (HDAC4, 5, 7, 9) was determined using a class IIa-specific substrate, Acetyl-Lys(trifluoroacetyl)-AMC (Lahm et al, 2007, PNAS, 104, 17335-17340). All assays were based on the AMC-labeled substrate and developer combination.

The protocol involved a two-step reaction: first, the substrate with the acetylated lysine side chain is incubated with a sample containing HDAC activity, to produce the deacetylated products, which are then digested in the second step by the addition of developer to produce the fluorescent signal proportional to the amount of deacetylated substrates.

### ii. Enzymes

Human HDAC1 (GenBank Accession No. NM_004964), full length with C-terminal His-tag and C-terminal FLAG-tag, MW= 56 kDa, expressed in baculovirus expression system.

Human HDAC2 (GenBank Accession No. NM_001527), full length with C-terminal His-tag, MW= 56 kDa, expressed inbaculovirus expression system. Complex of human HDAC3 (GenBank Accession No. NM_003883), full length with C-terminal His tag, MW= 49.7 kDa, and human NCOR2 (amino acid 395-489) (GenBank Accession No.NM_006312), N-terminal GST tag, MW=37.6 kDa, co-expressed in baculovirus expression system.

Human HDAC4 (GenBank Accession No. NM_006037), amino acids627- 1085 with N-terminal GST tag, MW=75.2 kDa, expressed in baculovirus expression system.

Human HDAC5 (GenBank Accession No. NM_005474), full length with N-terminal GST tag, MW= 150 kDa, expressed in baculovirus expression system. Recombinant human HDAC6 (GenBank Accession No. BC069243), full length, MW=180 kDa, was expressed by baculovirus in Sf9 insect cells using an N-terminal GST tag.

Human HDAC7 (GenBank Accession No. AY302468), (a.a. 518-end) with N-terminal GST tag, MW= 78 kDa, expressed in baculovirus expression system. Human HDAC8 (GenBankAccession No. NM_018486), full length with C-terminal His tag, MW= 46.4 kDa, expressed in a baculovirus expression system. Human HDAC9 (GenBank Accession No. NM_178423), amino acids 604-1066 with C-terminal His tag, MW=50.7 kDa, expressed in baculovirus expression system.

Human HDAC10 (a.a. 1-481), GenBank Accession No. NM_032019 with N-terminal GST tag and C-terminal His tag, MW= 78 kDa, expressed in baculovirus expression system.

Human HDAC11 (full length) (GenBank Accession No.NM_024827) with N-terminal GST tag, MW= 66 kDa, expressed in baculovirus expression system.

### iii. Reaction Conditions

Assay Buffer: 50mM Tris-HCl, pH8.0, 137 mM NaCl, 2.7 mM KCI, 1 mM MgCl₂. Before use, 1mg/mL BSA and DMSO are added.
HDAC1: 2.68 nM HDAC1 and 50m M HDAC substrate are in the reaction buffer with 1% DMSO final. Incubate for 2 hours at 30°C.
HDAC2: 3.33 nM HDAC2 and 50mM HDAC substrate are in the reaction buffer with 1% DMSO final. Incubate for 2 hours at 30°C.
HDAC3: 1.13 nM HDAC3 and 50mM HDAC substrate are in the reaction buffer with 1% DMSO final. Incubate for 2 hours at 30°C.
HDAC6: 0.56 nM HDAC6 and 50mM HDAC substrate are in the reaction buffer with 1% DMSO final. Incubate for 2 hours at 30°C.
HDAC8: 46.4 nM HDAC8 and 50mM HDAC8 substrate are in the reaction buffer with 1% DMSO final. Incubate for 2 hours at 30°C.
HDAC10: 96.15 nM HDAC10 and 50mM HDAC substrate are in the reaction buffer with 1% DMSO final. Incubate for 2 hours at 30°C.
HDAC11: 227.27 nM HDAC11 and 50mMHDAC substrate are in the reaction buffer with 1% DMSO final. Incubate for 2 hours at 30°C.

For class IIa HDACs, assay buffer is the same.

Other reaction conditions are as follows:
HDAC4: 0.03 nM HDAC4 and 50mM Class IIa HDAC substrate are in the reaction buffer with 1% DMSO final. Incubate for 30 minutes at room temperature.
HDAC5: 0.67 nM HDAC5 and 50mM Class IIa HDAC substrate are in the reaction buffer with 1% DMSO final. Incubate for 30 minutes at room temperature.
HDAC7: 0.26 nM HDAC7 and 50mM Class IIa HDAC substrate are in the reaction buffer with 1% DMSO final. Incubate for 30 minutes at room temperature.
HDAC9: 2.37 nM HDAC9 and 50mM Class IIa HDAC substrate are in the reaction buffer with 1% DMSO final. Incubate for 30 minutes at room temperature.

Control Inhibitor: Trichostatin A (TSA)
Fluorescent Deacetylated Standard: Biomol, Cat#KI-142;
For Standard Control, compound is added at assay concentration to 2.5 uM Fluorescent Deacetylated Standard; 10 doses in 6 uL
For Fluorescence Background Control, compound is added at assay concentrations to 50 mM HDAC substrate; 10 doses in 6 uL.

Fluorescence background signal is then subtracted from compound data signal. % Conversion must be between 5% and 15% to obtain optimum result.

### iv. Assay Procedure

Stage 1: Deacetylation of substrate by incubation of HDAC enzymes with compounds
Stage 2: Development by addition of Developer to digest the deacetylated substrate, and generate the fluorescent color; Detection: 360/460 Ex/Em

### 2) Inhibition of HDAC enzymes

| **Example** | **IC₅₀ (nM) HDAC** | |
|---|---|---|
| | **1** | **6** |
| **A** | **** | * |
| **B** | **** | * |
| **C** | *** | * |
| **D** | *** | * |
| **E** | *** | * |
| **F** | **** | * |
| **G** | **** | * |
| **H** | **** | * |
| **I** | *** | * |
| **J** | **** | * |
| **K** | **** | * |
| **L** | **** | * |
| **M** | **** | * |
| **N** | **** | * |
| **O** | **** | * |
| **P** | **** | * |
| **Q** | *** | * |
| **R** | **** | * |
| **S** | **** | *** |
| **T** | **** | *** |
| **U** | *** | * |
| **V** | **** | * |
| **W** | **** | * |
| **X** | **** | * |
| **Y** | **** | * |
| **Z** | **** | * |
| **AA** | *** | * |
| **BB** | *** | * |
| **CC** | **** | ** |
| **DD** | *** | * |
| **EE** | *** | * |
| **FF** | **** | * |
| **GG** | *** | * |
| **HH** | *** | * |
| **II** | *** | * |
| **JJ** | *** | * |
| **KK** | *** | * |
| **LL** | **** | * |
| **MM** | **** | * |
| **NN** | **** | * |
| **OO** | *** | * |
| **PP** | *** | * |
| **RR** | *** | * |
| **SS** | *** | * |

| | | |
|---|---|---|
| Key: **** ≥ 10uM *** ≤ 10uM ≥ 1uM ** ≤ 1uM ≥ 500nM * ≤ 500nM | | |

### Combination Data

### Combination Study 1

### Introduction

Data for two *in vitro* combination studies are provided below.

The effects on the growth of cancer cells of an HDAC6 selective inhibitor (Example GG of a Compound of Formula II (referred to in this experimental section simply as "Compound GG"), which is 4-{[Bis(pyrazin-2-yl)amino]methyl}-N-hydroxybenzamide) alone or in combination with a PI3K-p110β/δ inhibitor (Example A of a Compound of Formula I (referred to in this experimental section simply as "Compound A"), which is *4-(1H-lndol-4-yl)-6-(morpholin-4-yl)-12-[(1S,4S)-2-oxa-5-azabicyclo[2.2.1]heptan-5-ylmethyl]-8-oxa-3,5,10-triazatricyclo[7.4.0.0^{2,7}]trideca-1(13),2(7),3,5,9, 11-hexaene)* were tested:
i. in a panel of 94 cancer cell lines with in the presence of a fixed combination of Compound A (potentiation study #2015030003) or
ii. in a panel of 21 cancer cell lines with a 7x7 matrix of different compound concentrations of either Compound GG or Compound A (concentration matrix study #2015030004).

### Materials and Methods

### Proliferation assay

In study 2015030003, 94 cell lines were tested in parallel (22RV1^{‡}, 5637, 786O^{‡}, A204, A2780, A375^{‡}, A431, A549, A673, ACHN, ASPC1, BT20, BXPC3, C33A, CACO2, CAKI1, CALU6, CASKI, CLS439, COLO205, COLO678, DLD1^{‡}, DU145^{‡}, EFO21, EJ28^{‡}, GRANTA-519^{‡}, HCT116, HCT15, HEK293, HELA, HEPG2, HL-60, HS578T, HS729, HT1080, HT29, IGROV1, IMR90, J82, JAR, JEG3, JIMT1, KASUMI-1^{‡}, K-562, L-363^{‡}, LOVO, MCF7, MDAMB231^{‡}, M14, MDAMB436, MDAMB468^{‡}, MG63, MHHES1, MIAPACA2, MINO^{‡}, MT3, MV4-11, NCIH292, NCIH358M, NCIH460, NCIH82, OVCAR3, OVCAR4, PANC1^{‡}, PANC1005, PC3^{‡}, PLC-PRF-5, RD, RAMOS, RDES, SAOS2, SF268^{‡}, SF295, SKBR3, SKHEP1, SKLMS1, SKMEL28^{‡}, SKMEL5, SKNAS, SKNSH, SKOV3, SNB75, SU-DHL-6^{‡}, SU-DHL-10, SW620, T24, TE671, THP-1, U2OS, U87MG^{‡}, UMUC3^{‡}, UO31^{‡}, WSU-NHL^{‡} and human Peripheral Blood Mononuclear Cell, PBMC).

Cell lines marked with ‡ were also tested in study #2015030004.

Cell growth and treatment were performed in CELLSTAR® 96-well microtitre plates (Greiner Bio-One, Germany). Cells were harvested from exponential phase cultures by trypsinization and plated in 190 µL of media at optimal seeding densities. 48 hours later, cells were treated with 10 µL media containing compound at 20X final concentration (resulting in a final DMSO concentration of 0.1%). The cells were allowed to grow at 37°C for 72 hours. In addition, control plates with cells not exposed to compound were analyzed after 48 hours (time zero, *T_{z}).* Cell viability was determined using a sulforhodamine B (SRB) total protein staining assay. Briefly - after compound treatment, media was aspirated and cells were fixed by addition of 10% TCA. After an hour of incubation at 4°C plates were washed two times with 400µL of deionized water and dried. Cells were then stained with 100µL of 0.04% wt/v SRB. The plates were incubated at room temperature for at least 30 min and washed six times with 1% acetic acid to remove unbound stain. The plates were left to dry at room temperature and bound SRB was solubilized with 100µL of 10mM Tris base. Measurement of optical density was performed at 492, 520, and 560nm by using a Victor-2 plate reader (Perkin Elmer).

### Data analysis

Average background optical density (derived from plates and wells containing medium without cells) was subtracted from the optical density values from all controls and treated cells. Non-linear curve fitting calculations were performed using algorithms and visualization tools developed at Oncolead. The calculations included the dose response curves with the best approximation line, a 95% confidence interval for the 50% effect (IC₅₀) and the concentration of test agents giving a % T/C value of 50%, or 50% growth inhibition (IC₅₀), and a % T/C value of 10%, or 90% growth inhibition (IC₉₀). The IC₅₀, IC₉₀, GI₅₀, GI₉₀ and TGI values were computed automatically. All values were log10-transformed for z-score analysis performed using proprietary software developed at Oncolead integrated as a database analysis tool. The screening was designed to identify potential synergistic combinations using Cl, Bliss and highest single agent (HSA) indexation. Data are plotted as Loewe additivity isobolograms or Bliss independence or HSA calculations.

### Results

### Potentiation study #2015030003

The effects on the growth of cancer cells of the HDAC6 selective inhibitor Compound GG alone or in the presence of 100nM of the PI3K-p110β/δ inhibitor Compound A was tested in a panel of 94 cancer cell lines representing diverse lineages and cancer mutational status. Compound GG inhibited cell viability in these cell lines at GI₅₀ values ranging from 4.7µM to 33µM for the individual cell lines with a mean (± s.e.m) GI₅₀ value across the whole panel of 17.3µM) ±0.67. In the presence of 100nM Compound A, Compound GG inhibited cell viability in these cell lines at GI₅₀ values ranging from 1.7µM to 35µM for the individual cell lines with a mean (± s.e.m) GI₅₀ value across the whole panel of 14.1 µM ±0.7. The presence of Compound A appeared to potentiate the pharmacological activity of Compound GG in a cell-line specific manner, notably (but not exclusively) in cell lines derived from patients with mantle cell lymphoma (MINO), colorectal adenocarcinoma (LoVo) and prostate adenocarcinoma (PC-3). The potentiation effect manifested as either a shift in the Compound GG GI₅₀ in the presence of Compound A, shift in sensitivity relative to the mean sensitivity in z-score analysis and/or in HSA analysis.

### Concentration matrix study #2015030004

The effects on the growth of cancer cells of the HDAC6 selective inhibitor Compound GG alone or in combination with the PI3K-p110β/δ inhibitor Compound A was further tested in a panel of 21 cancer cell lines in a matrix dose response study. The averaged Bliss independence (across all concentrations tested) suggested a synergistic effect on the growth inhibition of UO31, MINO, PANC1, SU-DHL-6, DLD1, DU145 and PC-3 cells when combining Compound GG & Compound A. No synergy or potential antagonism was observed in the other cell lines tested.

### Combination Study 2

### Combination Therapy In Vivo: Compound A and Compound GG

### Tumor growth inhibition following daily oral dosing

An *in vivo* combination study involving daily oral co-administration of Compound A and Compound GG, alongside a daily dose of Compound A, revealed tumour growth inhibition of the combination.

In a 4T1 syngeneic mouse model of breast cancer, Compound A was dosed at 50mg/kg, QD, PO. In additional treatment groups, Compound A (50mg/kg, QD, PO) was combined with Compound GG (50mg/kg, QD, PO in one group, and 100mg/kg, QD, PO in a separate group). Daily dosing occurred for 18 consecutive days, after which anti-tumour activity was determined.

Tumor growth in vehicle-treated controls occurred in line with expectations, with all tumors growing steadily throughout the treatment period (Figure 1, below). Animals from drug treatment groups exhibited significant control of tumor growth after 10 days of treatment; this was maintained throughout the study. Animals treated with Compound A and Compound GG combinations exhibited the smallest tumors at the end of the study.

### Embodiments

Embodiment 1. A pharmaceutical composition comprising:
   a) at least one PI3K inhibitor of Formula I or a pharmaceutically acceptable salt thereof and at least one HDAC inhibitor such as a compound of Formula II or a pharmaceutically acceptable salt thereof; or
   b) at least one PI3K inhibitor such as a compound of Formula I or a pharmaceutically acceptable salt thereof and at least one HDAC inhibitor of Formula II or a pharmaceutically acceptable salt thereof: 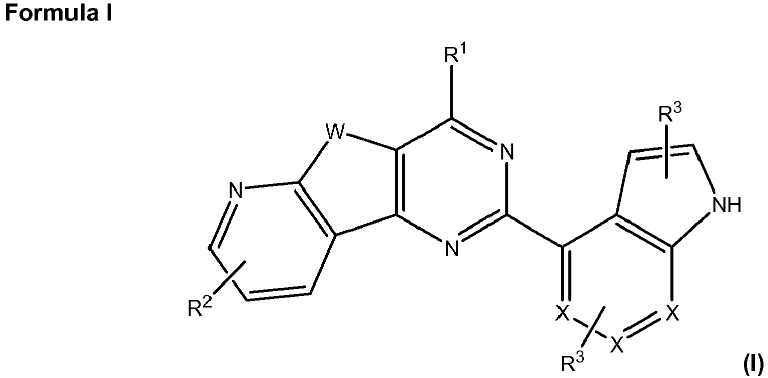 wherein:
      W is O, N-H, N-(C₁-C₁₀ alkyl) or S;
      each X is independently CH or N;
      R¹ is a 5 to 7-membered saturated or unsaturated, optionally substituted heterocycle containing at least 1 heteroatom selected from N or O;
      R² is LY;
      each L is a direct bond, C₁-C₁₀ alkylene, C₂-C₁₀ alkenylene or C₂-C₁₀ alkynylene;
      Y is an optionally substituted fused, bridged or spirocyclic non-aromatic 5-12 membered heterocycle containing up to 4 heteroatoms selected from N or O; and
      each R³ is independently H, C₁-C₁₀ alkyl, halogen, fluoro C₁-C₁₀ alkyl, O-C₁-C₁₀ alkyl, NH-C₁-C₁₀ alkyl, S-C₁-C₁₀ alkyl, O-fluoro C₁-C₁₀ alkyl, NH-acyl, NH-C(O)-NH-C₁₋C₁₀ alkyl, C(O)-NH-C₁-C₁₀ alkyl, aryl or heteroaryl;
      and 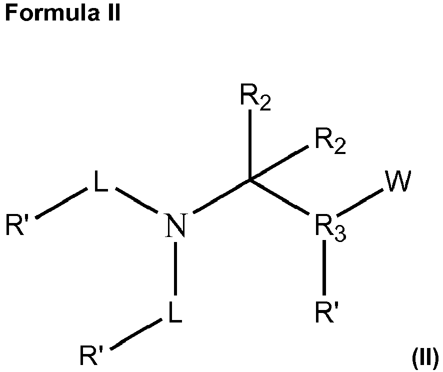
      or a pharmaceutically acceptable salt thereof, wherein:
      each R' is independently selected from H and QR₁;
      each Q is independently selected from a bond, CO, CO₂, NH, S, SO, SO₂ or O;
      each R₁ is independently selected from H, C₁-C₁₀ alkyl, C₂-C₁₀ alkenyl, C₂-C₁₀ alkynyl, aryl, heteroaryl, C₁-C₁₀ cycloalkyl, halogen, C₁-C₁₀ alkylaryl, C₁-C₁₀ alkyl heteroaryl or C₁-C₁₀ heterocycloalkyl;
      each L is independently selected from a 5 to 10-membered nitrogen-containing heteroaryl;
      W is a zinc-binding group;
      each R₂ is independently hydrogen or C₁ to C₆ alkyl; and
      R₃ is an aryl or heteroaryl;
      each aryl or heteroaryl may be substituted by up to three substituents selected from C₁-C₆ alkyl, hydroxy, C₁-C₃ hydroxyalkyl, C₁-C₃ alkoxy, C₁-C₃ haloalkoxy, amino, C₁-C₃ mono alkylamino, C₁-C₃ bis alkylamino, C₁-C₃ acylamino, C₁-C₃ aminoalkyl, mono (C₁-C₃ alkyl) amino C₁-C₃ alkyl, bis(C₁-C₃ alkyl) amino C₁-C₃ alkyl, C₁-C₃-acylamino, C₁-C₃ alkyl sulfonylamino, halo, nitro, cyano, trifluoromethyl, carboxy, C₁-C₃ alkoxycarbonyl, aminocarbonyl, mono C₁-C₃ alkyl aminocarbonyl, bis C₁-C₃ alkyl aminocarbonyl, -SO₃H, C₁-C₃ alkylsulfonyl, aminosulfonyl, mono C₁-C₃ alkyl aminosulfonyl and bis C₁-C₃-alkyl aminosulfonyl; and
      each alkyl, alkenyl or alkynyl may be substituted with halogen, NH₂, NO₂ or hydroxyl.
Embodiment 2. A kit comprising:
   a) at least one PI3K inhibitor of Formula I or a pharmaceutically acceptable salt thereof and at least one HDAC inhibitor such as a compound of Formula II or a pharmaceutically acceptable salt thereof; or
   b) at least one PI3K inhibitor such as a compound of Formula I or a pharmaceutically acceptable salt thereof and at least one compound of Formula II or a pharmaceutically acceptable salt thereof,
   as a combined preparation for simultaneous, sequential or separate use in therapy.
Embodiment 3. A method of treating or preventing a condition in a patient comprising administering to the patient a therapeutically effective amount of:
   a) at least one compound of Formula I or a pharmaceutically acceptable salt thereof and a HDAC inhibitor such as a compound of Formula II or a pharmaceutically acceptable salt thereof; or
   b) a PI3K inhibitor such as a compound of Formula I or a pharmaceutically acceptable salt and at least one compound of Formula II of a pharmaceutically acceptable salt thereof.
Embodiment 4. A composition according to Embodiment 1 wherein the PI3K inhibitor is selected from a compound of Formula I or a pharmaceutically acceptable salt thereof or Pictilisib, Dactolisib, Alpelisib, Voxtalisib, Gedatolisib, Copanlisib, Wortmannin, Apitolisib, Idelalisib, Buparlisib, Duvelisib, Pilaralisib, LY294002, GSK-2636771, AZD6482, PF-4989216, GS-9820, AMG319, SAR260301, MLN1117, PX-866, CH5132799, AZD8186, RP6530, GNE-317, PI-103, NU7441, HS-173, VS-5584, CZC24832, TG100-115, ZSTK474, AS-252424, AS-604850, NVP-BGT226, XL765, GDC-0032, A66, CAY10505, PF04691502, PIK-75, PIK-93, AS-605240, BGT226, CUDC-907, IC-87114, CH5132799, PKI-420, TGX-221, PIK-90; and/or wherein the HDAC inhibitor is selected from a compound of Formula II or a pharmaceutically acceptable salt thereof or Vorinostat, Entinostat, Panobinostat, Mocetinostat, Belinostat, Ricolinostat, Romidepsin, Givinostat, Dacinostat, Quisinostat, Pracinostat, Resminostat, Droxinostat, Abexinostat, RGFP966, AR-42, PCI34051, Trichostatin A, SB939, CI994, CUDC-907, Tubacin, Chidamide, RG2833, M344, MC1568, Tubastatin A, Scriptaid, Valproic Acid, Sodium Phenylbutyrate, Tasquinimod, Kevetrin, HPOB, 4SC-202, TMP269, CAY10603, BRD73954, BG45, LMK-235, Nexturastat A, CG200745, CHR2845, CHR3996. Embodiment 5. A composition according to any preceding Embodiment wherein the PI3K inhibitor is a compound of formula I or a pharmaceutically acceptable salt thereof and the HDAC inhibitor is a compound of formula II or a pharmaceutically acceptable salt thereof.
Embodiment 6. A method according to Embodiment 3, wherein the administration is separate, sequential or simultaneous.
Embodiment 7. The composition, method or kit according to any preceding Embodiment, wherein R¹ in Formula I is represented by any of the following structures: 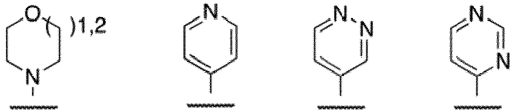
Embodiment 8. The composition, method or kit according to any preceding Embodiment, wherein R¹ in Formula I is morpholine.
Embodiment 9. The composition, method or kit according to any one of the preceding Embodiments, wherein W in Formula I is O or S.
Embodiment 10. The composition, method or kit according to any one of the preceding Embodiments, wherein W in Formula I is O.
Embodiment 11. The composition, method or kit according any one of the preceding Embodiments, wherein X in Formula I is CH.
Embodiment 12. The composition, method or kit according to any one of the preceding Embodiments, wherein R³ in Formula I is H.
Embodiment 13. The composition, method or kit according to any one of the preceding Embodiments, wherein L in Formula I is C₁-C₁₀ alkylene, preferably methylene.
Embodiment 14. The composition, method or kit according to any one of the preceding Embodiments, wherein Y in Formula I contains one or two heteroatoms, preferably two heteroatoms.
Embodiment 15. The composition, method or kit according to any one of the preceding Embodiments, wherein Y in Formula I is selected from: 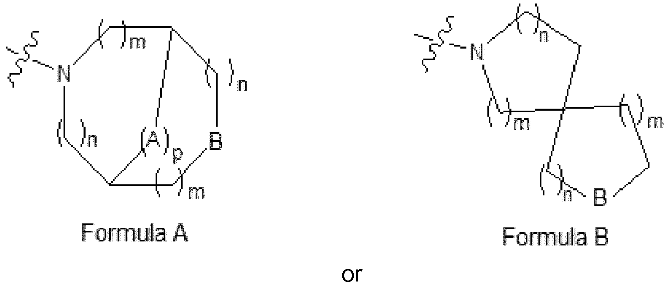 wherein:
   A is selected from O, S, NR⁴ or optionally substituted C₁-C₃ alkylene, C₂-C₃ alkenylene or C₂-C₃ alkynylene;
   B is NR⁴, O or CH₂;
   wherein R⁴ is H or optionally substituted C₁-C₁₀ alkyl, C₂-C₁₀ alkenyl or C₂-C₁₀ alkynyl;
   p is selected from 0 or 1;
   each m is independently selected from 0, 1 or 2; and
   each n is independently selected from 1, 2 or 3.
Embodiment 16. The composition, method or kit according to any preceding Embodiment, wherein A in Formula I is O or C₁-C₃ alkylene, preferably methylene.
Embodiment 17. The composition, method or kit according to any preceding Embodiment, wherein B in Formula I is O or CH₂, preferably O.
Embodiment 18. A composition, method or kit according to any preceding Embodiment, wherein a compound of Formula I is illustrated by any one of the following structures: 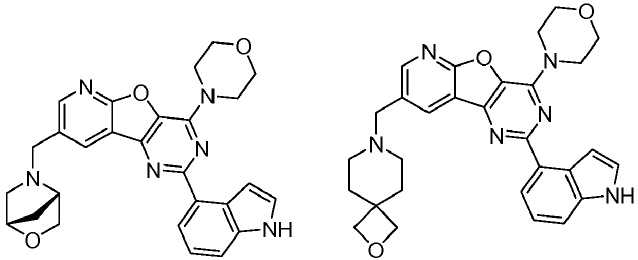 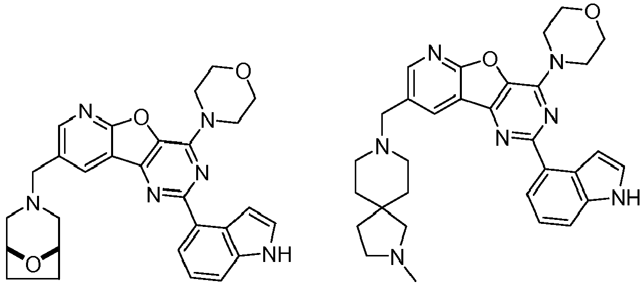 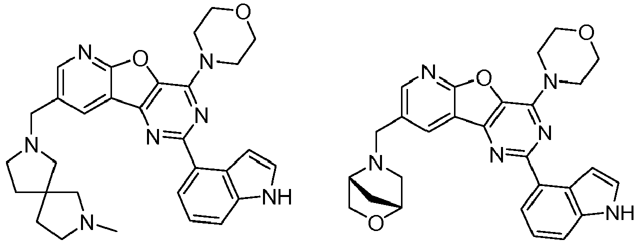 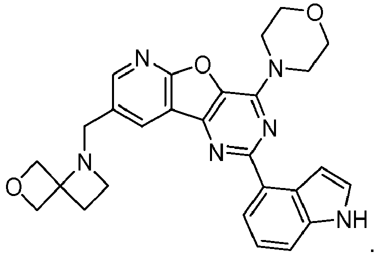
Embodiment 19. A composition, kit or method according to any preceding Embodiment, wherein W in formula II is selected from: 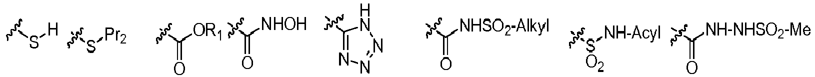 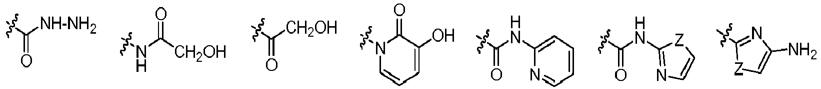 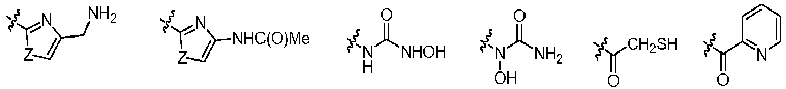 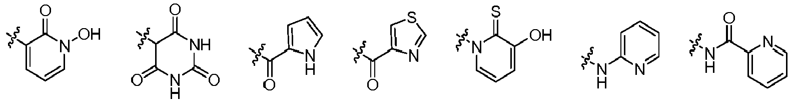 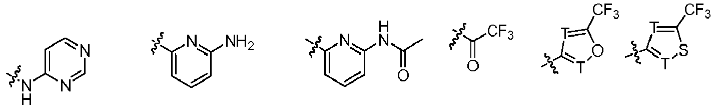 wherein R₁ is as defined in claim 1, Pr² is H or a thiol protecting group, Z is selected from O, S or NH and T is N or CH.
Embodiment 20. A composition, kit or method according to any preceding Embodiment, wherein W in formula II is -CONHOH.
Embodiment 21. A composition, kit or method according to any preceding Embodiment, wherein each L in formula II is independently selected from a 5 or 6-membered nitrogen-containing heteroaryl, which is optionally fused to a benzene.
Embodiment 22. A composition, kit or method according to any preceding Embodiment, wherein in at least one, preferably both L groups in formula II, the atom that is directly bonded to the N is a carbon, and at least one nitrogen atom is directly bonded to said carbon.
Embodiment 23. A composition, kit or method according to any preceding Embodiment, wherein L in formula II is independently selected from pyridinyl, pyrimidinyl, pyridazinyl, oxadiazolyl, pyrazolyl, thiadiazolyl, pyrazinyl, benzofused thiazolyl, benzofused oxazolyl or benzofused imidazolyl, preferably, L is independently selected from pyridyl and pyrazinyl.
Embodiment 24. A composition, kit or method according to any preceding Embodiment, wherein at least one L group in formula II is pyridinyl, oxadiazolyl, pyrazolyl, thiadiazolyl, pyrazinyl, benzofused thiazolyl, benzofused oxazolyl or benzofused imidazolyl, preferably at least one L group is pyridyl or pyrazinyl.
Embodiment 25. A composition, kit or method according to any preceding Embodiment, wherein R₃ in formula II is phenylene or phenylene substituted with a halogen.
Embodiment 26. A composition, kit or method according to any preceding Embodiment, wherein at least one, preferably both, R₂ in formula II is/are H.
Embodiment 27. A composition, kit or method according to any preceding Embodiment, wherein R' that is attached to L in formula II is independently selected from H, C₁-C₁₀ alkyl or O-(C₁-C₁₀ alkyl), halogen, C₁-C₁₀ heterocycloalkyl, aryl, trifluoromethyl or heteroaryl.
Embodiment 28. A composition, kit or method according to any preceding Embodiment, wherein at least one R' in formula II is H, halogen, CF₃, C₁-C₆ alkyl, aryl optionally substituted with halogen, heteroaryl optionally substituted with halogen or heterocycloalkyl.
Embodiment 29. A composition, kit or method according to any preceding Embodiment, wherein at least one of the R' that is attached to L in formula II is heterocycloalkyl.
Embodiment 30. A composition, kit or method according to any preceding Embodiment, wherein R' attached to R₃ in formula II is hydrogen or halogen.
Embodiment 31. A composition, kit or method according to any preceding Embodiment, wherein at least one R' in formula II is C₁-C₆ alkyl optionally substituted with halogen, NH₂, NO₂ or hydroxyl.
Embodiment 32. A composition, kit or method according to any preceding Embodiment, wherein at least one R' in formula II is C₁-C₆ alkyl optionally substituted with halogen.
Embodiment 33. A composition, kit or method according to any preceding Embodiment, wherein Formula II is as exemplified herein.
Embodiment 34. A composition, kit or method according to any preceding Embodiment, wherein the compound of Formula I is 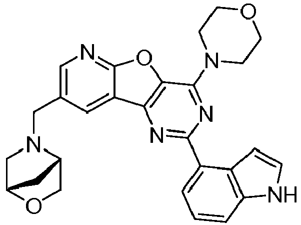
   or a pharmaceutically acceptable salt thereof,
   and/or the compound of Formula II is 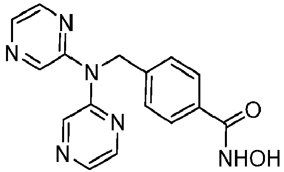
   or a pharmaceutically acceptable salt thereof.
Embodiment 35. A pharmaceutical composition comprising a composition as defined in any preceding Embodiment, and a pharmaceutically acceptable excipient.
Embodiment 36. A composition or kit according to any preceding Embodiment, for use in therapy.
Embodiment 37. A composition, kit or method according to any preceding Embodiment, wherein the therapy is of cancer, an immune disorder or an inflammatory disorder.
Embodiment 38. A composition, kit or method according to Embodiment 37, wherein the cancer is a leukaemia or a PTEN-negative solid tumour. Embodiment 39. A composition, kit or method according to Embodiment 36 or Embodiment 37, wherein the therapy is of rheumatoid arthritis.
Embodiment 40. A composition, kit or method according to Embodiment 36 or Embodiment 37, for use in anti-rejection therapy following an organ transplant.

## Claims

1. A pharmaceutical composition comprising:
at least one PI3K inhibitor such as a compound of Formula I or a pharmaceutically acceptable salt thereof and at least one HDAC inhibitor of Formula II or a pharmaceutically acceptable salt thereof: 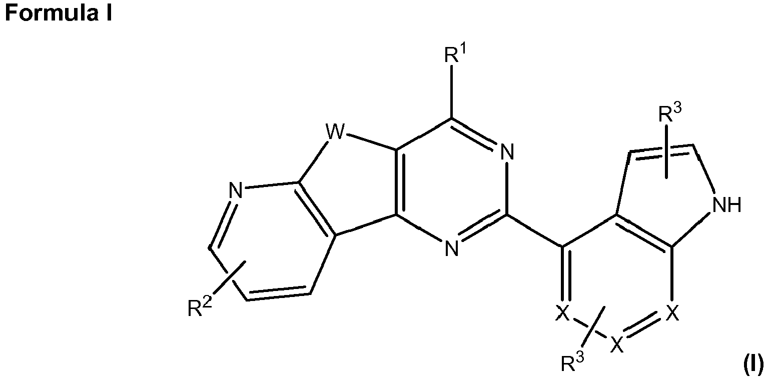 wherein:
W is O, N-H, N-(C₁-C₁₀ alkyl) or S;
each X is independently CH or N;
R¹ is a 5 to 7-membered saturated or unsaturated, optionally substituted heterocycle containing at least 1 heteroatom selected from N or O;
R² is LY;
each L is a direct bond, C₁-C₁₀ alkylene, C₂-C₁₀ alkenylene or C₂-C₁₀ alkynylene;
Y is an optionally substituted fused, bridged or spirocyclic non-aromatic 5-12 membered heterocycle containing up to 4 heteroatoms selected from N or O; and
each R³ is independently H, C₁-C₁₀ alkyl, halogen, fluoro C₁-C₁₀ alkyl, O-C₁-C₁₀ alkyl, NH-C₁-C₁₀ alkyl, S-C₁-C₁₀ alkyl, O-fluoro C₁-C₁₀ alkyl, NH-acyl, NH-C(O)-NH-C₁-C₁₀ alkyl, C(O)-NH-C₁-C₁₀ alkyl, aryl or heteroaryl;
**and** 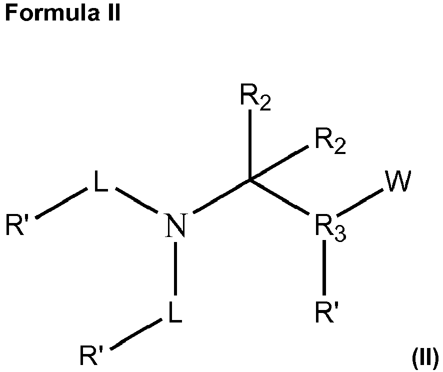
or a pharmaceutically acceptable salt thereof, wherein:
each R' is independently selected from H and QR₁;
each Q is independently selected from a bond, CO, CO₂, NH, S, SO, SO₂ or O;
each R₁ is independently selected from H, C₁-C₁₀ alkyl, C₂-C₁₀ alkenyl, C₂-C₁₀ alkynyl, aryl, heteroaryl, C₁-C₁₀ cycloalkyl, halogen, C₁-C₁₀ alkylaryl, C₁-C₁₀ alkyl heteroaryl or C₁-C₁₀ heterocycloalkyl;
each L is independently selected from a 5 to 10-membered nitrogen-containing heteroaryl;
W is a zinc-binding group;
each R₂ is independently hydrogen or C₁ to C₆ alkyl; and
R₃ is an aryl or heteroaryl;
each aryl or heteroaryl may be substituted by up to three substituents selected from C₁-C₆ alkyl, hydroxy, C₁-C₃ hydroxyalkyl, C₁-C₃ alkoxy, C₁-C₃ haloalkoxy, amino, C₁-C₃ mono alkylamino, C₁-C₃ bis alkylamino, C₁-C₃ acylamino, C₁-C₃ aminoalkyl, mono (C₁-C₃ alkyl) amino C₁-C₃ alkyl, bis(C₁-C₃ alkyl) amino C₁-C₃ alkyl, C₁-C₃-acylamino, C₁-C₃ alkyl sulfonylamino, halo, nitro, cyano, trifluoromethyl, carboxy, C₁-C₃ alkoxycarbonyl, aminocarbonyl, mono C₁-C₃ alkyl aminocarbonyl, bis C₁-C₃ alkyl aminocarbonyl, -SO₃H, C₁-C₃ alkylsulfonyl, aminosulfonyl, mono C₁-C₃ alkyl aminosulfonyl and bis C₁-C₃-alkyl aminosulfonyl; and
each alkyl, alkenyl or alkynyl may be substituted with halogen, NH₂, NO₂ or hydroxyl.

2. The composition according to claim 1 wherein the PI3K inhibitor is selected from a compound of Formula I or a pharmaceutically acceptable salt thereof or Pictilisib, Dactolisib, Alpelisib, Voxtalisib, Gedatolisib, Copanlisib, Wortmannin, Apitolisib, Idelalisib, Buparlisib, Duvelisib, Pilaralisib, LY294002, GSK-2636771, AZD6482, PF-4989216, GS-9820, AMG319, SAR260301, MLN1117, PX-866, CH5132799, AZD8186, RP6530, GNE-317, PI-103, NU7441, HS-173, VS-5584, CZC24832, TG100-115, ZSTK474, AS-252424, AS-604850, NVP-BGT226, XL765, GDC-0032, A66, CAY10505, PF04691502, PIK-75, PIK-93, AS-605240, BGT226, CUDC-907, IC-87114, CH5132799, PKI-420, TGX-221, PIK-90; and/or wherein the HDAC inhibitor is selected from a compound of Formula II or a pharmaceutically acceptable salt thereof or Vorinostat, Entinostat, Panobinostat, Mocetinostat, Belinostat, Ricolinostat, Romidepsin, Givinostat, Dacinostat, Quisinostat, Pracinostat, Resminostat, Droxinostat, Abexinostat, RGFP966, AR-42, PCI34051, Trichostatin A, SB939, CI994, CUDC-907, Tubacin, Chidamide, RG2833, M344, MC1568, Tubastatin A, Scriptaid, Valproic Acid, Sodium Phenylbutyrate, Tasquinimod, Kevetrin, HPOB, 4SC-202, TMP269, CAY10603, BRD73954, BG45, LMK-235, Nexturastat A, CG200745, CHR2845, CHR3996.

3. The composition according to any preceding claim wherein the PI3K inhibitor is a compound of formula I or a pharmaceutically acceptable salt thereof and the HDAC inhibitor is a compound of formula II or a pharmaceutically acceptable salt thereof.

4. The composition according to any preceding claim, wherein R¹ in Formula I is represented by any of the following structures: 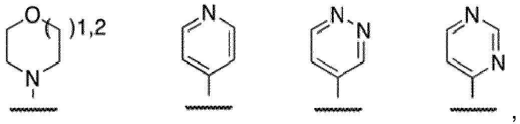 preferably wherein R¹ in Formula I is morpholine.

5. The composition according to any one of the preceding claims, wherein W in Formula I is O or S, preferably O.

6. The composition according any one of the preceding claims, wherein X in Formula I is CH and/or wherein R³ in Formula I is H and/or wherein L in Formula I is C₁-C₁₀ alkylene, preferably methylene.

7. The composition according to any one of the preceding claims, wherein Y in Formula I contains one or two heteroatoms, preferably two heteroatoms, further preferably wherein Y in Formula I is selected from: 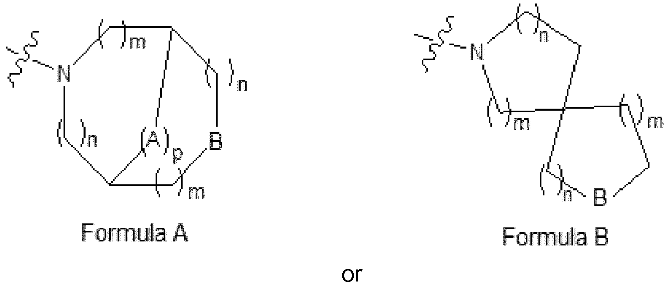 wherein:
A is selected from O, S, NR⁴ or optionally substituted C₁-C₃ alkylene, C₂-C₃ alkenylene or C₂-C₃ alkynylene;
B is NR⁴, O or CH₂;
wherein R⁴ is H or optionally substituted C₁-C₁₀ alkyl, C₂-C₁₀ alkenyl or C₂-C₁₀ alkynyl;
p is selected from 0 or 1;
each m is independently selected from 0, 1 or 2; and
each n is independently selected from 1, 2 or 3.

8. The composition according to any preceding claim, wherein A in Formula I is O or C₁-C₃ alkylene, preferably methylene and/or wherein B in Formula I is O or CH₂, preferably O.

9. The composition according to any preceding claim, wherein a compound of Formula I is illustrated by any one of the following structures:

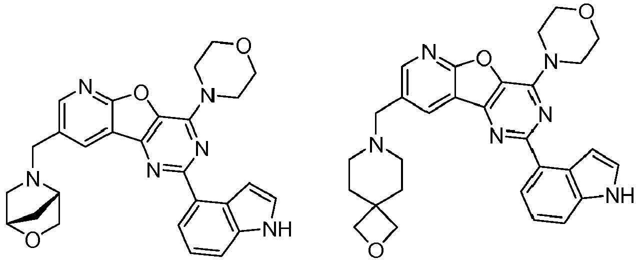

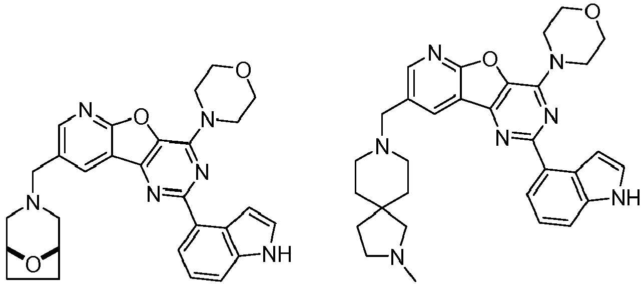

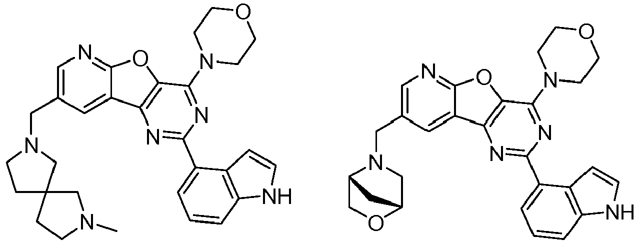

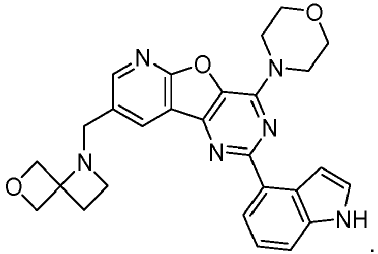

10. The composition according to any preceding claim, wherein W in formula II is selected from: 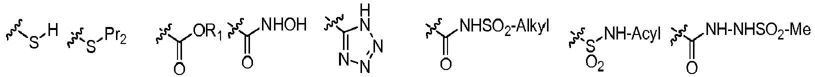 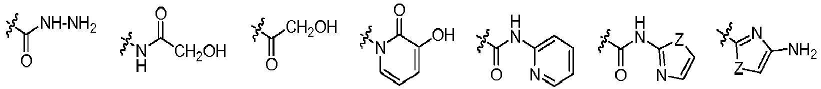 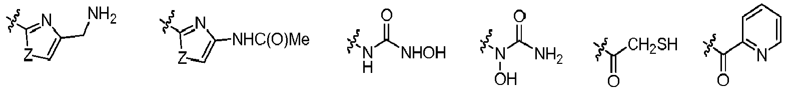 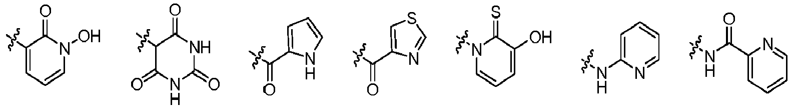 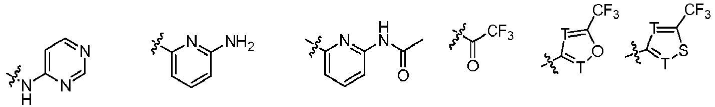 wherein R₁ is as defined in claim 1, Pr² is H or a thiol protecting group, Z is selected from O, S or NH and T is N or CH, preferably wherein W in formula II is -CONHOH.

11. The composition according to any preceding claim, wherein each L in formula II is independently selected from a 5 or 6-membered nitrogen-containing heteroaryl, which is optionally fused to a benzeneand/or wherein in at least one, preferably both L groups in formula II, the atom that is directly bonded to the N is a carbon, and at least one nitrogen atom is directly bonded to said carbon.

12. The composition according to any preceding claim, wherein L in formula II is independently selected from pyridinyl, pyrimidinyl, pyridazinyl, oxadiazolyl, pyrazolyl, thiadiazolyl, pyrazinyl, benzofused thiazolyl, benzofused oxazolyl or benzofused imidazolyl, preferably, L is independently selected from pyridyl and pyrazinyl and/or wherein at least one L group in formula II is pyridinyl, oxadiazolyl, pyrazolyl, thiadiazolyl, pyrazinyl, benzofused thiazolyl, benzofused oxazolyl or benzofused imidazolyl, preferably at least one L group is pyridyl or pyrazinyl, and/or wherein R₃ in formula II is phenylene or phenylene substituted with a halogen and/or wherein at least one, preferably both, R₂ in formula II is/are H, and/or wherein R' that is attached to L in formula II is independently selected from H, C₁-C₁₀ alkyl or O-(C₁-C₁₀ alkyl), halogen, C₁-C₁₀ heterocycloalkyl, aryl, trifluoromethyl or heteroaryl and/or wherein at least one R' in formula II is H, halogen, CF₃, C₁-C₆ alkyl optionally substituted with halogen, NH₂, NO₂ or hydroxyl, aryl optionally substituted with halogen, heteroaryl optionally substituted with halogen or heterocycloalkyl, preferably wherein at least one of the R' that is attached to L in formula II is heterocycloalkyl and/or wherein R' attached to R₃ in formula II is hydrogen or halogen, further preferably wherein at least one R' in formula II is C₁-C₆ alkyl optionally substituted with halogen.

13. The composition according to any preceding claim, wherein the compound of Formula I is 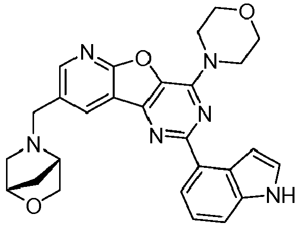 or a pharmaceutically acceptable salt thereof, and/or
the compound of Formula II is 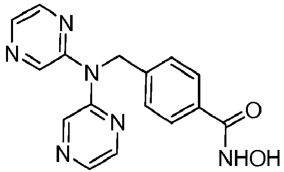 or a pharmaceutically acceptable salt thereof.

14. A kit comprising the composition according to any preceding claim such that the at least one PI3K inhibitor and the at least one HDAC inhibitor are a combined preparation for simultaneous, sequential, or separate use.

15. A pharmaceutical composition comprising the composition as defined in any preceding claim, and a pharmaceutically acceptable excipient.

16. The composition or kit according to any preceding claim, for use in the treatment of cancer, an immune disorder or an inflammatory disorder, or for use in anti-rejection therapy following an organ transplant, preferably wherein the cancer is a leukaemia or a PTEN-negative solid tumour and/or the inflammatory disorder is rheumatoid arthritis, preferably wherein the administration of the at least one PI3K inhibitor and the at least one HDAC inhibitor is separate, sequential or simultaneous.
